# EUROPÄISCHE PATENTANMELDUNG

(11) **EP 2 340 822 A1**
(43) Veröffentlichungstag der Anmeldung: **06.07.2011**
(21) Anmeldenummer: 10013065.7
(22) Anmeldetag: 15.01.2004
(51) Int. Cl.: A61K 31/198, A61K 31/401, A61K 31/435, A61P 7/02, A61P 9/00, A61P 9/02, A61P 9/10, A61P 37/00, A61P 37/08, A61P 43/00, C07K 5/00

(54) **Acylierte 4-Amidino- und 4-Guanidinobenzylaminen zur Inhibierung von Plasmakallikrein**

(30) Priorität: 15.01.2003 DE 10301300
(62) Teilanmeldung aus: 04702332.0
(71) Anmelder: The Medicines Company (Leipzig) GmbH, 04103 Leipzig (DE)
(72) Erfinder: Stürzebecher, Jörg, 99094 Erfurt (DE); Steinmetzer, Torsten, 07743 Jena (DE); Schweinitz, Andrea, 07749 Jena (DE)
(74) Vertreter: Bösl, Raphael Konrad

(57) **Zusammenfassung**

Die Erfindung betrifft unter anderem die Verwendung von acyliertem 4-Amidino- oder 4-Guanidinobenzylamin gemäss der allgemeinen Formel (I): P4-P3-P2-P1, wobei P4 eine einfach oder mehrfach substituierte oder unsubstituierte Benzylsulfonylgruppe ist, P3 eine einfach oder mehrfach substituierte oder unsubstituierte, natürliche oder unnatürliche α-Amino- oder α-Iminosäure in der D-Konfiguration ist, P2 eine einfach oder mehrfach substituierte oder unsubstituierte, natürliche oder unnatürliche α-Amino-oder α-Iminosäure in der L-Konfiguration ist, und P1 eine einfach oder mehrfach substituierte oder unsubstituierte 4-Amidino- oder 4-Guanidino-benzylamingruppe ist, zur Inhibierung von Plasmakallikrein (PK), Faktor XIa und/oder Faktor XIIa.

## Beschreibung

Die Erfindung betrifft die Verwendung von acyliertem 4-Amidino- oder 4-Guanidinobenzylamin gemäß der allgemeinen Formel P4-P3-P2-P1 (I), wobei P4 eine einfach oder mehrfach substituierte oder unsubstituierte Benzylsulfonylgruppe ist, P3 eine einfach oder mehrfach substituierte oder unsubstituierte, natürliche oder unnatürliche α-Amino- oder α-Iminosäure in der D-Konfiguration ist, P2 eine einfach oder mehrfach substituierte oder unsubstituierte, natürliche oder unnatürliche α-Amino- oder α-Iminosäure in der L-Konfiguration ist, und P1 eine einfach oder mehrfach substituierte oder unsubstituierte 4-Amidino- oder 4-Guanidinobenzylamingruppe ist, zur Inhibierung von Plasmakallikrein (PK). Dabei werden die neuen PK-Inihibitoren zur Verhinderung der Gerinnungsaktivierung an künstlichen Oberflächen und zur systemischen Gabe als Antikoagulanzien/Antithrombotika, vor allem zur Verhinderung der Gerinnungsaktivierung an künstlichen Oberflächen eingesetzt, um thromboembolischen Ereignissen vorzubeugen.

Weiterhin betrifft die vorliegende Erfindung die neuen acylierten 4-Amidino- oder 4-Guanidinobenzylamine an sich, wobei insbesondere solche bevorzugt sind, die eine Linkergruppe an P2 oder P4 aufweisen, wobei insbesondere bevorzugt diese Linkergruppen Oligo- oder Polyalkylenglycole sind.

Ebenso betrifft die vorliegende Erfindung auch die Verwendung der oben genannten acylierten 4-Amidino- oder 4-Guanidinobenzylamine zur Inhibierung von Faktor XIa und/oder Faktor XIIa. Im Rahmen der vorliegenden Erfindung ist auch die Verwendung der oben genannten Verbindung zur Inhibierung von Thrombin und Prothrombin beschrieben.

PK ist eine multifunktionelle, trypsinartige Serinprotease für die mehrere physiologische Substrate bekannt sind. So kann PK durch proteolytische Spaltung das vasoaktive Peptid Bradykinin aus hochmolekularem Kininogen freisetzen und die Proteasen Gerinnungsfaktor XII, Pro-Urokinase, Plasminogen und Pro-MMP 3 aktivieren. Deshalb wird angenommen, dass das PK/Kinin-System eine wichtige Rolle bei verschiedenen Krankheitsbildern besitzt, so z.B. bei thromboembolischen Situationen, der disseminierten intravasalen Gerinnung, septischem Schock, Allergien, dem Postgastrektomiesyndrom, Arthritis und ARDS (adult respiratory distress syndrome) (Tada et al., Biol. Pharm. Bull 24, 520-524, 2001).

Durch Aktivierung des Gerinnungsfaktors XII zu Faktor XIIa spielt PK vor allem bei der Aktivierung der intrinsischen Gerinnungskaskade eine Rolle. Eine Aktivierung der intrinsischen Gerinnungskaskade kann stattfinden, wenn Blut in extrakorporealen Blutkreisläufen in Kontakt mit künstlichen Oberflächen kommt, so z.B. bei der Hämodialyse oder bei der Verwendung von Oxygenatoren. Durch die Bindung von Faktor XII an insbesondere negativ geladene und/oder künstliche Oberflächen wird über Autoaktivierung bzw. durch Spuren von PK die intrinsische Gerinnungskaskade angestoßen (Kaplan, Prog. Hemostasis Thromb. 4, 127-175, 1978). Der aktivierte Faktor XII (F XIIa) katalysiert die Umwandlung von Plasmapräkallikrein zu PK, das - im Sinne eines positiven Feedback - die weitere Bildung von Faktor XIIa bewirkt (Griffin, Proc. natl. Acad. Sci. USA 75, 1998-2002, 1978). Entsprechend der Bedeutung von Faktor XIIa und PK in der Frühphase der intrinsischen Gerinnungskaskade sollten auch Inhibitoren dieser Enzyme gerinnungshemmend wirken. Im Rahmen dieser Frühphase der intrinsischen Gerinnungsaktivierung aktiviert Faktor XI-Ia den Faktor XI zu Faktor XIa.

Als Inhibitoren sowohl der intrinsischen als auch der extrinsischen Gerinnungskaskade und damit zur Prophylaxe und Therapie der oben genannten Krankheitsbilder, wie z.B. thromboembolischer Situationen, disseminierter intravasaler Gerinnung, septischem Schock, Allergien, dem Postgastrektomiesyndrom, Arthritis und ARDS, werden Antikoagulantien vom Heparin-Typ, Vitamin-K-Antagonisten oder Hirudin eingesetzt. Da die gängigen Antikoagulantien aber nicht allen Anforderungen an ein "ideales" Antithrombotikum gerecht werden, beispielsweise aufgrund ihrer geringen Spezifität, auftretenden Blutungskomplikationen, geringer Halbwertszeit oder mangelnder oraler Verfügbarkeit, wird versucht, mit kleinmolekularen Inhibitoren der Gerinnungsproteasen Thrombin und Faktor Xa Alternativen zu entwickeln. Auch Faktor VIIa, das initiale Enzym des extrinsischen Gerinnungsweges, ist ein vielfältig untersuchtes Zielenzym für die Inhibitorentwicklung (Robinson und Saiah, Ann. Rep. Med. Chem. 37, 85-94, 2002). Ein Thrombin- und F Xa-Hemmstoff bzw. ein F VIIa-Hemmstoff als ein spezifischer Hemmstoff der extrinsischen Gerinnungskaskade besitzt jedoch keine Hemmwirkung auf die z.B. durch Kontakt des Blutes mit künstlichen Oberflächen ausgelöste Aktivierung der intrinsischen Gerinnungskaskade.

Bei der Suche nach Inhibitoren für die beiden Enzyme Faktor XIIa und PK, die die intrinsische Gerinnung nach Aktivierung an einer geladenen Oberfläche einleiten, gibt es nur wenige Ansätze. Für das Guanidinoalkylcarbonsäure-Derivat FOY (Isobe, Blood & Vessel 12, 135-138, 1981), Leupeptin, den Thrombinhemmstoff Na-Dansyl-L-arginin-4-ethylpiperidid (Ratnoff, Blood 57, 55-58, 1981) und verschiedene Tripeptide (Ester, Amide) (Fareed et al. Ann. N. York Acad. Sci. 370, 765-784, 1981; Silverberg und Kaplan, Blood 60, 64-70, 1982) wurde eine gewisse Hemmwirkung gegenüber Faktor XIIa mitgeteilt. Wirksamere Inhibitoren wurden mit Amiden der Nα-substituierten 4-Amidinophenyl-α-aminobuttersäure beschrieben (Stürzebecher et al., Zentralbl. Pharm. Pharmakother. Lab. Diagn. 122, 240-241, 1983).

Als PK-Inhibitoren erwiesen sich verschiedene Bis-benzamidine wie Pentamidin und verwandte Verbindungen mit Kᵢ-Werten um 50 µM als wirksam (Ashgar et al., Biochim. Biophys. Acta 438, 250-264, 1976). Auch Ester von ω-Amino- und ω-Guanidinoalkylcarbonsäuren wurden als PK-Inhibitoren mit mikromolaren Kᵢ-Werten beschrieben (Muramatu und Fuji, Biochim. Biophys. Acta 242, 203-208, 1971; Muramatu und Fuji, Biochim. Biophys. Acta 268, 221-224, 1972; Ohno et al. Thromb. Res. 19, 579-588, 1980; Muramatu et al. Hoppe-Seyler's Z. Physiol. Chem. 363, 203-211, 1982; Satoh et al. Chem. Pharm. Bull. 33, 647-654, 1985; Teno et al. Chem. Pharm. Bull. 39, 2930-2936, 1991). Die ersten hochselektiven kompetitiven Inhibitoren, die sich vom Arginin bzw. Phenylalanin ableiten, wurde von Okamoto et al. (Thromb. Res., Suppl. VIII, 131-141, 1988) entwickelt und hemmen PK mit Kᵢ-Werten um 1 µM. Von der Gruppe um Okada wurden mehrere Arbeiten zur Entwicklung kompetitiver PK-Inhibitoren veröffentlicht, wobei die wirksamsten Verbindungen, die sich vom trans-4-Amino-methylcyclohexancarbonyl-Phe-4-Carboxymethylanilid ableiten, Hemmkonstanten um 0,5 µM besitzen (Okada et al., Biopolymers 51, 41-50, 1999; Okada et al., Bioorg. Med. Chem. Lett. 10, 2217-2221; 2000, Tsuda et al., Chem. Pharm. Bull. 49, 1457-1463, 2001). Gemeinsam ist den genannten PK-Inhibitoren ihr relativ hoher Kᵢ-Wert. In WO 00/41531 wurden potente PK-Inhibitoren mit Hemmkonstanten um 1 nM beschrieben, die ein 4-Amidinoanilin als P1 Rest besitzen. Diese in WO 00/41531 beschriebenen Inhibitoren sind jedoch nicht geeignet, um kovalent an künstliche Oberflächen gekoppelt zu werden. PK-Hemmstoffe wurden auch in WO 94/29336 beschrieben. der wesentliche Unterschied zu den Verbindungen gemäß der vorliegenden Erfindung besteht darin, dass die in WO 94/29336 enthaltenen Verbindungen nicht den entscheidenden Benzylsulfonylrest (P4) enthalten. Zudem wurde in WO 94/29336 keine Kopplung der Verbindungen z.B. an künstliche Oberflächen beschrieben.

Inzwischen sind auch einige Übergangszustands-analoge PK-Inhibitoren beschrieben, die ein Arginal (z.B. Adamantyloxycarbonyl-D-Phe-Phe-Arginal, Kᵢ 12 nM, Garrett et al., J. Pept. Res. 52, 60-71, 1998) oder Arginyltrifluormethylketon (z.B. Adamantyloxycarbonyl-D-tert.Butylglycin-Phe-Arg-CF₃, Kᵢ 2 nM, Garrett et al., Bioorg. Med. Chem. Lett. 9, 301-306, 1999) als P1-Rest besitzen. Auch für das ursprünglich als Thrombinhemmstoff entwickelte Boroarginin-Derivat DuP 714 (Ac-D-Phe-Pro-Boroarginin) wurde eine starke PK-Hemmung (Kᵢ 1,6 nM) gefunden (Kettner et al., J. Biol. Chem. 265, 18289-18297). Solche Übergangszustands-analogen Proteaseinhibitoren haben jedoch den Nachteil, dass sie nur durch aufwendige Synthesen zugänglich sind, zur Racemisierung neigen und sehr unspezifische Inhibitoren sind.

Eine irreversible Hemmung von PK wird auch durch verschiedene Chlormethylketone erreicht. H-Ala-Phe-ArgCH₂Cl und H-Pro-Phe-ArgCH₂Cl wurden als die reaktivsten Verbindungen beschrieben (Kettner und Shaw, Biochemistry 17, 4778-4784, 1978). Allerdings sind Peptidylchlormethylketone ausschließlich für Forschungszwecke geeignet, da sie in vivo nur eine Stabilität von wenigen Minuten besitzen (Lawson et al., Folia Haematol. (Leipzig) 109, 52-60, 1982; Collen et al., J. Lab. Clin. Med. 99,76-83, 1982).

Der Erfindung liegt daher die Aufgabe zu Grunde, für therapeutische Anwendungen geeignete Wirkstoffe bereitzustellen, die Plasmakallikrein mit hoher Aktivität und Spezifität inhibieren und die nach Kopplung an eine künstlichen Oberfläche bzw. nach parenteraler, enteraler oder topischer Gabe, insbesondere intravenöser oder subkutaner Gabe gerinnungshemmend wirksam sind.

Überraschend wurde gefunden, dass acyliertes 4-Amidino- oder 4-Guanidinobenzylamin gemäß der allgemeinen Formel P4-P3-P2-P1 (I), wobei P4 (in Anlehnung an die Definition nach Schechter und Berger, Biochem. Biophys. Res. Comm. 27, 157-162) eine einfach oder mehrfach substituierte oder unsubstituierte Benzylsulfonylgruppe ist, P3 eine einfach oder mehrfach substituierte oder unsubstituierte, natürliche oder unnatürliche α-Amino- oder α-Iminosäure in der D-Konfiguration, P2 eine einfach oder mehrfach substituierte oder unsubstituierte, natürliche oder unnatürliche α-Amino- oder α-Iminosäure in der L-Konfiguration, und P1 eine einfach oder mehrfach substituierte oder unsubstituierte 4-Amidino- oder 4-Guanidinobenzylamingruppe, Plasmakallikrein sehr wirksam inaktivieren, auch nach Kopplung an eine künstliche Oberfläche gerinnungshemmend wirksam sind und sowohl parenteral, enteral oder topisch, insbesondere intravenös oder subkutan eingesetzt werden können.

Ein besonderer Vorteil der erfindungsgemäßen Derivate des acylierten 4-Amidino- bzw. 4-Guanidinobenzylamins ist somit ihre Fähigkeit, PK auch nach der Bindung an eine künstliche Oberfläche mit hoher Aktivität zu inaktivieren. Daher stellen die erfindungsgemäßen Verbindungen eine neue Gruppe von hochaktiven und insbesondere koppelbaren Plasmakallikrein-Inhibitoren dar.

Eine künstliche Oberfläche im Sinne der vorliegenden Erfindung ist eine Oberfläche, die beispielsweise aus Cellulose-Diacetat, Cellulose-Triacetat, Poly(ethersulfon), Poly(arylethersulfon), regenerierter Cellulose, Cuprophan, Hemophan, Poly(sulfon), Poly(acrylnitril), Poly(vinylalkohol), Poly(carbonat), Poly(amid), Poly(methylmethacrylat), Poly(ethylen-co-vinylalkohol) oder einem anderen in Geräten wie Dialysatoren, Oxygenatoren, Kathetern, Membranen und/oder den zu den Geräten gehörenden Schlauchsystemen und Luftfallen, die insbesondere in extrakorporalen Kreisläufen mit Blut in Kontakt kommen, verwendeten Material besteht, wobei die Oberflächenmaterialien gegebenenfalls mit funktionellen Gruppen, z.B. Aminogruppen, Aminoalkylgruppen, Carboxylgruppen, Carboxyalkylgruppen, Mercaptogruppen, Mercaptoalkylgruppen, Hydroxylgruppen, Hydroxyalkylgruppen modifiziert sind, um eine kovalente Kopplung der Inhibitoren zu ermöglichen.

Gemäß einer bevorzugten Ausführungsform ist der Substituent am substituierten P4, P3, P2 und/oder P1 Wasserstoff und/oder ein Halogen, vorzugsweise Fluor, Chlor und/oder Brom und/oder ein substituierter oder nicht-substituierter, verzweigter oder linearer Alkylrest mit 1-6 C-Atomen, vorzugsweise 1-3 C-Atomen, insbesondere Methyl, oder ein substituierter oder nicht-substituierter, verzweigter oder linearer Aralkylrest mit 1-10 C-Atomen ist, wobei der Substituent des substituierten, verzweigten oder linearen Alkylrests oder Aralkylrestes vorzugsweise eine Halogen-, Hydroxy-, Amino-, Cyano-, Amidino-, Guanidino- und/oder eine Carboxylgruppe, gegebenenfalls verestert mit einem Niederalkylrest, insbesondere mit Methyl oder Ethyl ist, und/oder eine Hydroxy-, Amino-, Cyano-, Amidino-, Guanidino-, Methyloxycarbonyl-, Benzyl-, Benzyloxycarbonyl-, Aminomethyl- oder Glutaryl- oder Succinyl-Amidomethylgruppe ist und/oder eine Oxyalkylcarbonyl, Carboxyl-, Carboxymethyl- oder Carboxyethylgruppe ist gegebenenfalls verestert mit einem Niederalkylrest, insbesondere mit Methyl oder Ethyl oder eine Oxyalkylcarbonyl, Carboxyl-, Carboxymethyl- oder Carboxyethylgruppe, die als unsubstituiertes oder mit einer Alkyl- oder Arylgruppe substituiertes Amid vorliegt.

Falls nicht anders angegeben ist unter einem Alkylrest im Sinne der vorliegenden erfmdung immer ein Alkylrest mit 1-12 C-Atomen zu verstehen, unter einem Arylrest ein Arylrest mit 6 bis 10 C-Atomen und unter einem Aralkylrest ein Aralkylrest mit 6 bis 12 C-Atomen.

Unter einem Niederalkylrest im Sinne der vorliegenden Erfindung versteht man einen Alkylrest mit 1 bis 6 C-Atomen, vorzugsweise 1-3 C-Atomen.

An P4 oder P2 kann zusätzlich eine Linkergruppe gekoppelt sein, wobei die Linkergruppe über einen oben beschriebenen Substituenten an P4 oder direkt an eine funktionelle Gruppe von P2, insbesondere über eine -NH- oder eine -CO-Gruppe gekoppelt ist.

Eine Linkergruppe im Sinne der vorliegenden Erfindung ist definiert als eine chemische Struktur, die zumindest eine funktionelle Gruppe zur kovalenten Kopplung an ein acyliertes 4-Amidino- bzw. 4-Guanidinobenzylamin über P4 oder P2 aufweist und darüber hinaus entweder zumindest eine zweite funktionelle Gruppe zur gleichzeitigen kovalenten Kopplung an eine künstliche Oberfläche oder gleichzeitigen Kopplung eines zweiten Moleküls des acylierten 4-Amidino- bzw. 4-Guanidinobenzylamins aufweist und/oder eine Oligo-oder Polyalkylenglykolgruppe aufweist, die in der Lage ist, durch Interaktion mit der künstlichen Oberfläche nicht-kovalent an diese zu koppeln.

Eine Linkergruppe gemäß der vorliegenden Erfindung ist daher vorzugsweise eine Dicarbonsäure, eine Aminocarbonsäure, ein Diamin, eine Disulfonsäure, oder eine Aminosulfonsäure mit einem Alkyl-, Aryl- oder Aralkylgrundgerüst, wobei das Alkylgrundgerüst 1 bis 12 C-Atome, insbesondere 2-6 C-Atome aufweist, das Arylgrundgerüst 6-10 C-Atome, insbesondere Phenyl aufweist, das Aralkylgrundgerüst 6-12 C-Atome, insbesondere Benzyl aufweist, oder eine Aminoalkyl- oder Carboxyalkylgruppe mit 2-12 C-Atomen, insbesondere 2-6 C-Atomen; oder wobei die Linkergruppe an P4 oder P2 eine Oligo- oder Polyalkylenglycolkette ist, insbesondere eine Poly- oder Oligoethylen- oder Poly- oder Oligopropylenglycolkette ist, wobei das Oligo- oder Polyalkylenglycol zumindest an beiden Enden eine funktionelle Gruppe, insbesondere eine substituierte oder unsubstituierte Amino-, Carboxyl- und/oder Mercaptogruppe aufweist oder wobei das Oligo- oder Polyalkylenglycol zumindest an einem Ende eine funktionelle Gruppe, insbesondere eine substituierte oder unsubstituierte Amino-, Carboxyl- und/oder Mercaptogruppe aufweist und am anderen Ende mit einer CH₃-Gruppe modifiziert ist.

Bei Kopplung der Linkergruppe an P4 ist die Linkergruppe vorzugsweise über eine -NH-Gruppe, -NH-Alkyl-Gruppe mit 1 bis 6 C-Atomen, insbesondere Methyl, eine -CO-Gruppe, eine -CO-Alkyl-Gruppe mit 2-6 C-Atomen, insbesondere -CO-Methyl, eine -CO-O-Alkyl-Gruppe mit 1-6 C-Atomen, insbesondere Methyl, eine -S-Gruppe, eine -S-Alkyl-Gruppe mit 1 bis 6 C-Atomen, insbesondere Methyl, eine -0-Alkyl-Gruppe mit 1-6 C-Atomen, insbesondere Methyl, eine -SO₂-Gruppe oder eine -SO₂-Alkyl-Gruppe mit 1-6 C-Atomen, insbesondere Methyl an P4 gekoppelt.

Die Linkergruppe kann statt an P4 auch an P2 gekoppelt sein, wobei P2 vorzugsweise Lysin oder dessen Homologe mit 1-5 C-Atomen in der Seitenkette, insbesondere Ornithin, Homolysin, α-γ-Diaminobuttersäure, α-β-Diaminopropionsäure, α-Diaminoglycin oder Glutaminsäure oder dessen Homologe mit 1-5 C-Atomen in der Seitenkette, insbesondere Asparaginsäure, Glutaminsäure oder Homoglutaminsäure oder Cystein oder Homocystein oder Serin oder Threonin ist.

Gemäß einer bevorzugten Ausführungsform der vorliegenden Erfindung weist die an P4 gekoppelte Linkergruppe mit dem Substituenten zur Kopplung an P4 die allgemeine Formel U-Z-Y-X- (II) auf, wobei U gleich eine H₂N-, HOOC-(CH₂)ₙ-CO-NH-, HOOC- , H₂N-(CH₂)ₙ-NH-CO- oder HS-Gruppe ist mit Z gleich -(CH₂)ₙ- mit n = 1 bis 10, insbesondere 1-5, oder Z gleich ein Oligo- oder Polyalkylenglycol der allgemeinen Formel - (CH₂)_{d}-[O-CH₂-CH₂]ᵥO-(CH₂)ₘ-(NH-CO-CH₂-O-CH₂)ₖ- oder -(CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 50, insbesondere 2 bis 10, m = 0, 1, 2, 3 oder 4 und k = 0 oder 1 oder U gleich eine CH₃-O-Gruppe ist mit Z gleich ein Oligo- oder Polyalkylenglycol der allgemeinen Formel -(CH₂)_{d}-[O-CH₂-CH₂]ᵥO-(CH₂)ₘ-(NH-CO-CH₂-O-CH₂)ₖ- oder - (CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 50, insbesondere 2 bis 10, m = 0, 1, 2, 3 oder 4 und k = 0 oder 1 ist; Y gleich eine -CO-NH-, eine -NH-CO-, eine -SO₂-NH-, eine - NH-SO₂-, eine -S-S- oder eine -S-Gruppe ist oder wenn U und Z nicht vorhanden sind gleich eine H₂N-, HOOC-, HS-, HO- oder Halogenalkyl-Gruppe ist; X gleich eine -(CH₂)ₙ-Gruppe mit n = 0, 1, 2, 3 oder 4, insbesondere n = 1 oder gleich eine -(CH₂)ₙ-O-Gruppe mit Bindung an den Benzylrest über den Sauerstoff und n = 1, 2, 3 oder 4 ist. Die Kopplung der Linkergruppe an den Benzylrest geht von X falls vorhanden oder von Y, wenn X nicht vorhanden ist, aus.

Wenn der Linker an P4 gekoppelt ist, dann ist P2 Glycin, Alanin, Prolin, Homoprolin oder Azetidincarbonsäure.

Gemäß einer weiteren bevorzugten Ausführungsform ist die Linkergruppe an P2 gekoppelt, wobei P2 die allgemeine Formel III 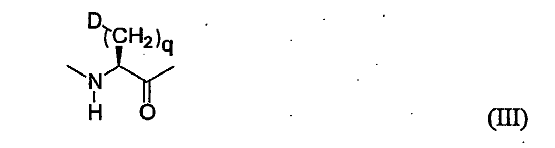 aufweist, wobei q = 0, 1, 2, 3, 4 oder 5 ist und D gleich die Formel 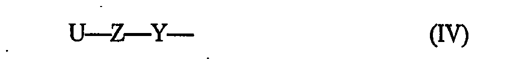 ist, wobei U, Z und Y die gleiche Bedeutung wie bei Formel II besitzen.

Gemäß einer besonders bevorzugten Ausführungsform weist das acylierte Amidino- oder Guanidinobenzylamin die allgemeine Formel V oder VI auf 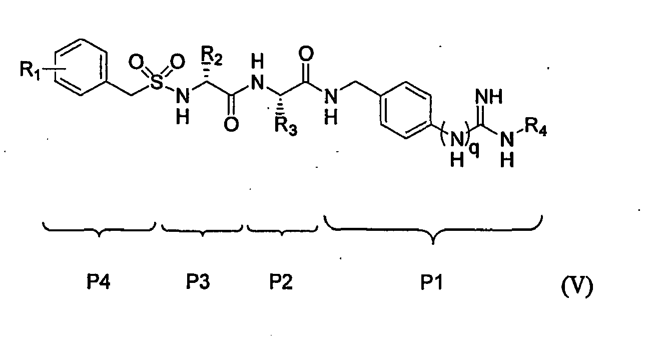 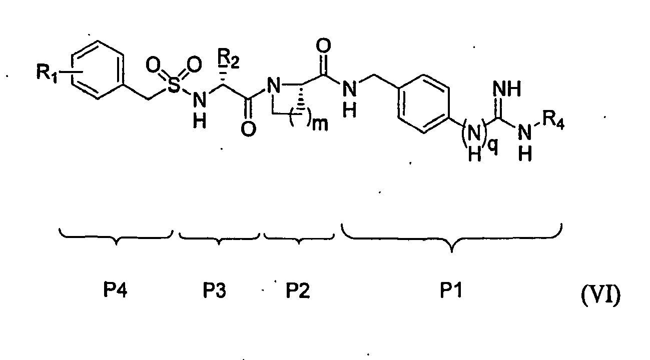 wobei m = 1 bis 3 und q gleich 0 oder 1, insbesondere 0 ist und wobei R₁, R₂, R₃ und/oder R₄ Wasserstoff und/oder ein Halogen, vorzugsweise Fluor, Chlor und/oder Brom und/oder ein substituierter oder nicht-substituierter, verzweigter oder linearer Alkylrest mit 1-6 C-Atomen, vorzugsweise 1-3 C-Atomen, insbesondere Methyl ist, wobei der Substituent des substituierten, verzweigten oder linearen Alkylrests vorzugsweise eine Halogen-, Hydroxy- , Amino-, Cyano-, Amidino-, Guanidino- und/oder eine Carboxylgruppe, gegebenenfalls verestert mit einem Niederalkylrest, insbesondere mit Methyl oder Ethyl ist und/oder eine Hydroxy-, Amino-, Cyano-, Amidino-, Guanidino-, Methyloxycarbonyl-, Benzyl-, Benzyloxycarbonyl-, Aminomethyl- oder Glutaryl- oder Succinyl-Amidomethylgruppe ist und/oder eine Oxyalkylcarbonyl, Carboxyl-, Carboxymethyl- oder Carboxyethylgruppe ist gegebenenfalls verestert mit einem Niederalkylrest, insbesondere mit Methyl oder Ethyl oder als unsubstituiertes oder mit einer Alkyl- oder Arylgruppe substituiertes Amid vorliegend.

Besonders bevorzugt sind im Rahmen der vorliegenden Erfindung als Reste R₄ der Hydroxy-, ein Amino- und ein Alkoxycarbonylrest, insbesondere ein Alkoxycarbonylrest mit 2 bis 10 C-Atomen.

R₁ und/oder R₃ kann zusätzlich eine Linkergruppe sein, wobei die Linkergruppe über einen oben beschriebenen Substituenten an P4 oder direkt an eine funktionelle Gruppe von P2, insbesondere über eine -NH- oder eine -CO-Gruppe gekoppelt ist, wobei die Linkergruppe vorzugsweise eine Dicarbonsäure, eine Aminocarbonsäure, ein Diamin, eine Disulfonsäure, oder eine Aminosulfonsäure mit einem Alkyl-, Aryl- oder Aralkylgrundgerüst ist, wobei das Alkylgrundgerüst 1 bis 12 C-Atome, insbesondere 2-6 C-Atome aufweist, das Arylgrundgerüst 6-10 C-Atome, insbesondere Phenyl aufweist, das Aralkylgrundgerüst 6-12 C-Atome, insbesondere Benzyl aufweist, eine Aminoalkyl- oder Carboxyalkylgruppe mit 2-12 C-Atomen, insbesondere 2-6 C-Atomen; oder wobei die Linkergruppe an P4 oder P2 eine Oligo- oder Polyalkylenglycolkette ist, insbesondere eine Poly- oder Oligoethylen- oder Poly- oder Oligopropylenglycolkette ist, wobei das Oligo- oder Polyalkylenglycol zumindest an beiden Enden eine funktionelle Gruppe, insbesondere eine substituierte oder unsubstituierte Amino-, Carboxyl- und/oder Mercaptogruppe aufweist oder wobei das Oligo- oder Polyalkylenglycol zumindest an einem Ende eine funktionelle Gruppe, insbesondere eine substituierte oder unsubstituierte Amino-, Carboxyl- und/oder Mercaptogruppe aufweist und am anderen Ende mit einer Alkylgruppe mit 1-4 C-Atomen, insbesondere CH₃-Gruppe modifiziert ist und/oder R₁ zusätzlich die Formel (II) wie oben definiert und P2 mit R₃ zusätzlich die Formeln (III) und (IV), wie oben definiert, aufweist.

Bevorzugte Ausführungsbeispiele für acylierte Amidino- und/oder Guanidinobenzylamine gemäß der allgemeinen Formel I mit einer Linkergruppe an P4 gemäß der allgemeinen Formel II weisen vorzugsweise eine der folgenden Strukturen auf: 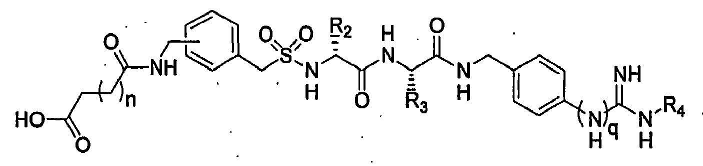 oder 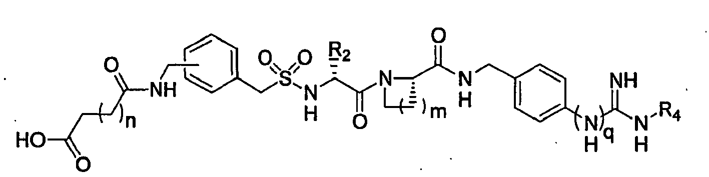 oder 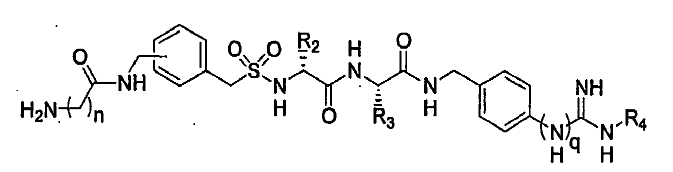 oder 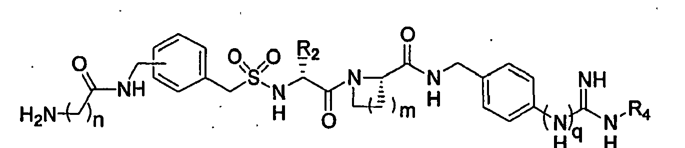 mit n = 1 bis 10, m = 1 bis 3 und q = 0 oder 1, insbesondere 0, wobei R₂ und R₃ die oben genannten Bedeutungen besitzen. Durch das Vorhandensein einer zweiten funktionellen Gruppe wie z.B. H₂N- oder HOOC- können die aufgeführten Substanzen gleichzeitig mit der Kopplung an P4 kovalent an künstliche Oberflächen gekoppelt werden.

Weitere bevorzugte Ausführungsbeispiele für acylierte Amidino- und/oder Guanidinobenzylamine gemäß der allgemeinen Formel I mit einer Linkergruppe an P4 gemäß der allgemeinen Formel II weisen vorzugsweise die folgenden Strukturen auf: 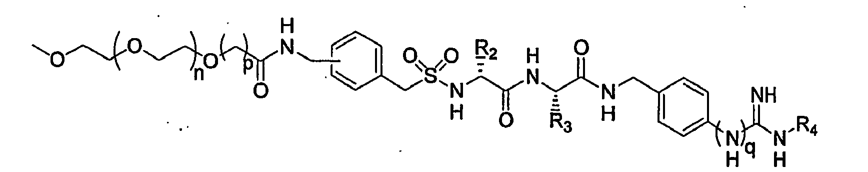 oder 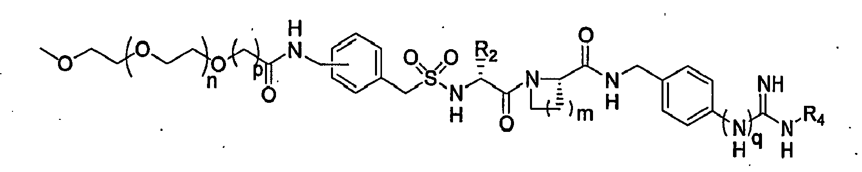 oder 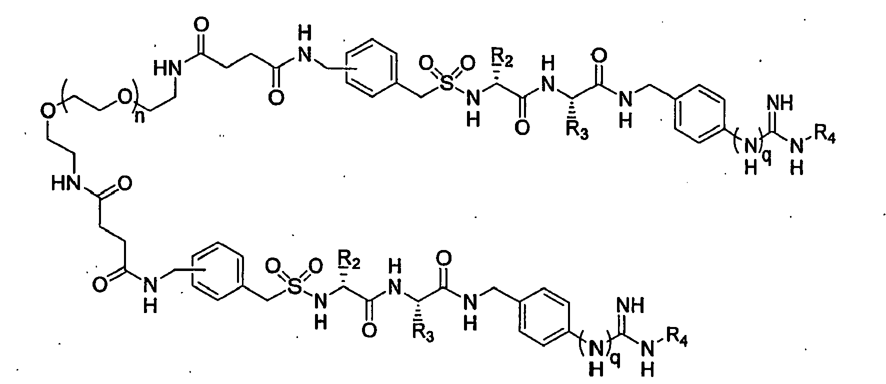 oder 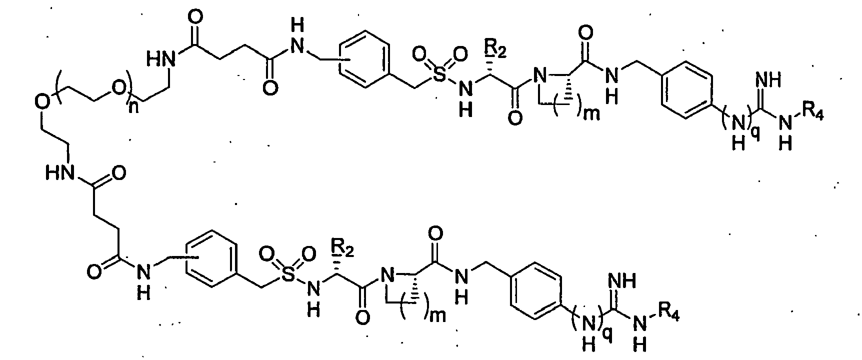 oder 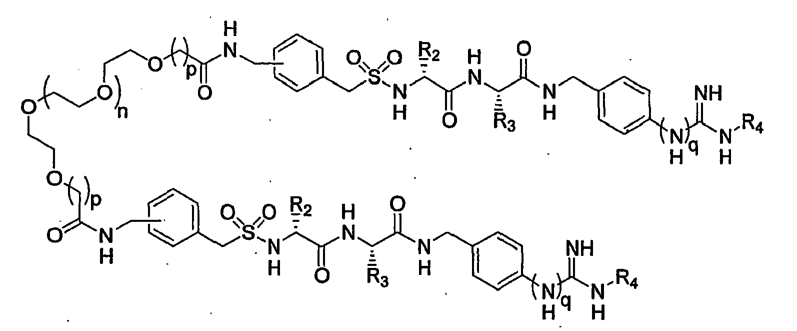 oder 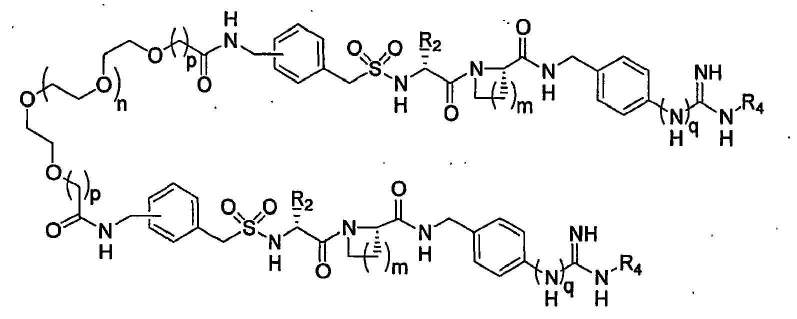 mit p = 0, 1, 2 oder 3, q = 0 oder 1, insbesondere 0, n = 1 bis 1000 und m = 1 bis 3, wobei R₂ und R₃ jeweils die oben genannten Bedeutungen besitzen. Durch das Fehlen einer zweiten funktionellen Gruppe können die aufgeführten Substanzen neben der kovalenten Kopplung an P4 nur nicht-kovalent an künstliche Oberflächen gekoppelt werden. Dies geschieht durch Interaktionen der Oligo- oder Polyalkylengruppe der Linkergruppe mit der künstlichen Oberfläche.

Unter Interaktion der Linkergruppe, insbesondere einer Linkergruppe, die eine Oligo- oder Polyalkylengruppe enthält, mit einer künstlichen Oberfläche im Sinne der vorliegenden Erfindung ist eine nicht-kovalente Wechselwirkung dieser Linkergruppe mit der künstlichen Oberfläche, beispielsweise über Wasser-vermittelte Wasserstoffbrückenbindungen, hydrophobe Wechselwirkungen oder van der Waalssche Wechselwirkungen zu verstehen.

Die Substanzen, bei denen an eine Oligo- oder Polyalkylengruppe zwei Moleküle der Formel I gekoppelt sind, werden im Sinne der vorliegenden Erfindung als zweifach Inhibitor-funktionalisierte Oligo- oder Polyalkylenglycole bezeichnet.

Ein weiterer Vorteil von Oligo- und/oder Polyalkylenderivaten, die an einem Ende als reine Monomethylether vorliegen und so nicht zum kovalenten Koppeln geeignet sind, besteht in ihrer verlängerten Halbwertszeit in der zirkulation nach systemischer Gabe.

Bevorzugte Ausführungsbeispiele für acylierte Amidinobenzylamine gemäß der allgemeinen Formel I mit einer Linkergruppe an P2 gemäß den allgemeinen Formeln III und IV weisen vorzugsweise eine der folgenden Strukturen auf: 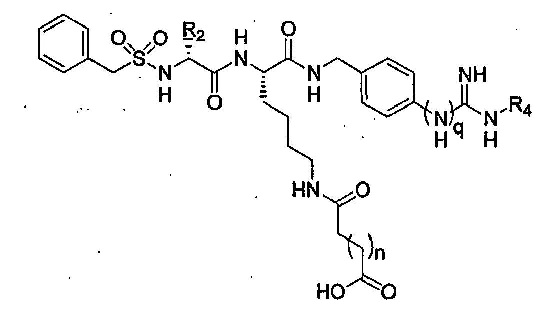 mit n= 0 bis 5, bevorzugt 1 oder 2 oder 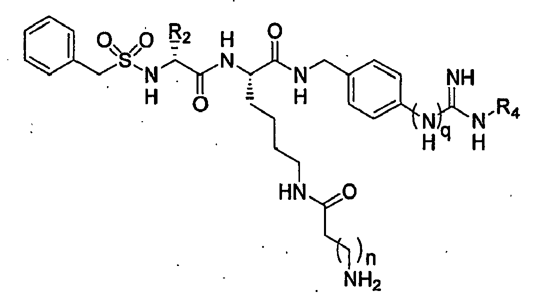 mit n = 0 bis 11 oder 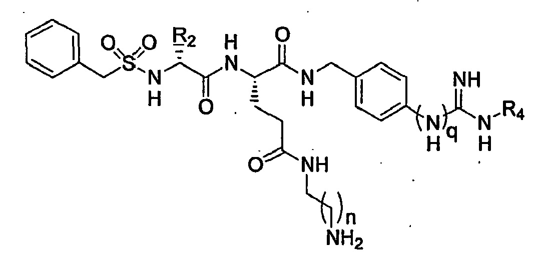 mit n = 1 bis 6 oder 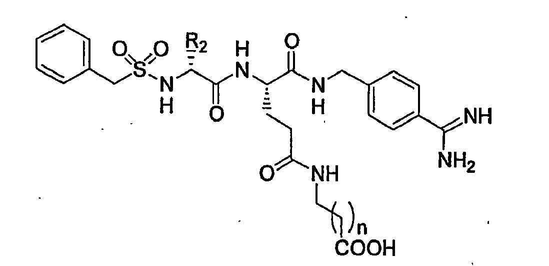 oder 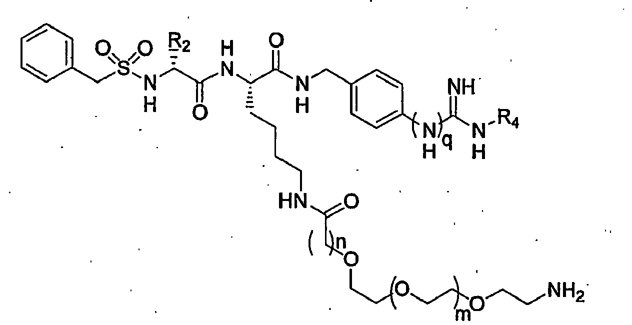 mit n= 0 bis 3 und m = 0 bis 1000
oder 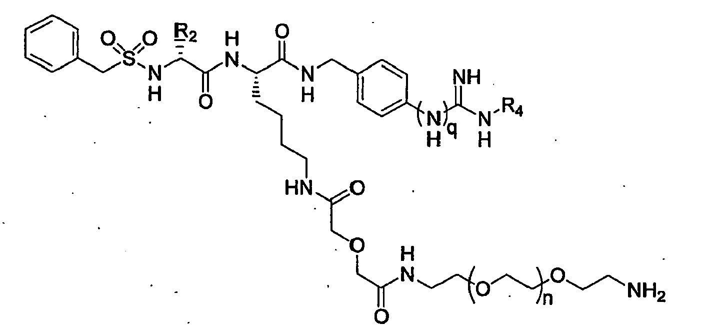 mit n = 1 bis 1000 oder 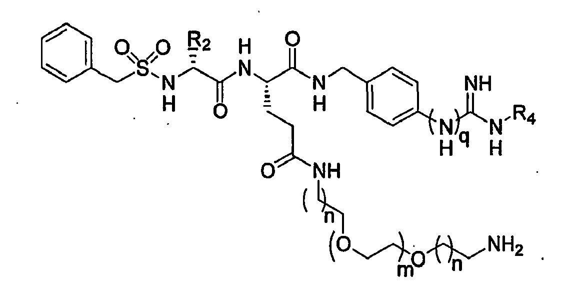 mit n = 1 bis 3 und m=1 bis 1000, wobei q jeweils gleich 0 oder 1, insbesondere 0 ist und R₂ jeweils die oben genannten Bedeutungen besitzt. Durch das Vorhandensein einer zweiten funktionellen Gruppe können die aufgeführten Substanzen gleichzeitig mit der Kopplung an P2 kovalent an künstliche Oberflächen oder an ein zweites Molekül der allgemeinen Formel I gekoppelt werden.

Ein weiteres bevorzugtes Ausführungsbeispiel für ein acyliertes Amidino- und/oder Guanidinobenzylamin gemäß der allgemeinen Formel I mit einer Linkergruppe an P2 gemäß den allgemeinen Formeln III und IV weist vorzugsweise eine der folgenden Strukturen auf: 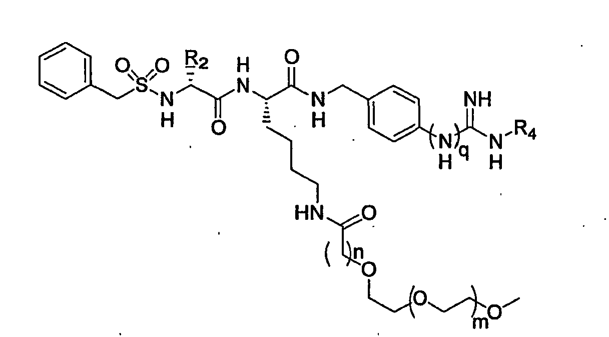 mit n = 0 bis 4 und m = 10 bis 1000 oder 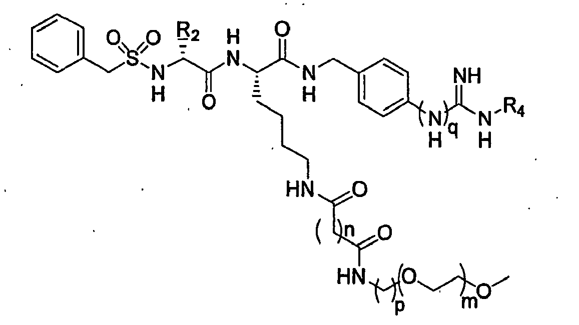 mit n = 1 bis 4, p = 2 bis 4 und m = 1 bis 1000 oder 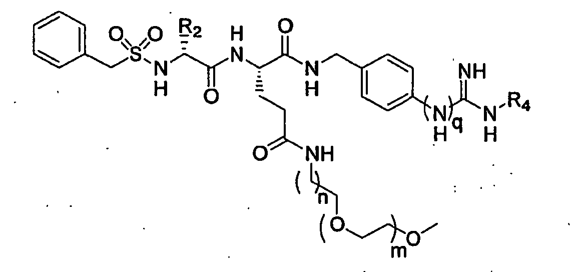 oder 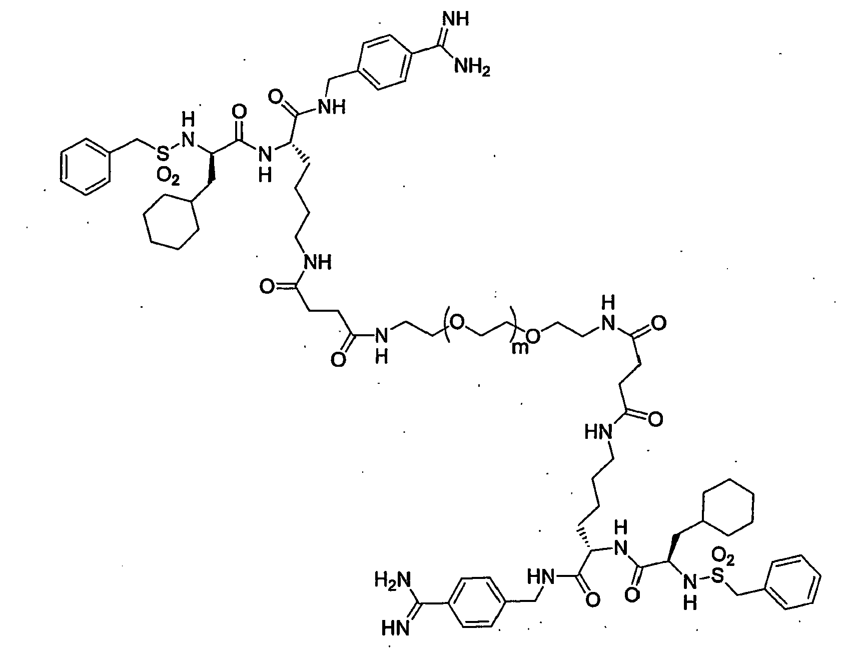 oder 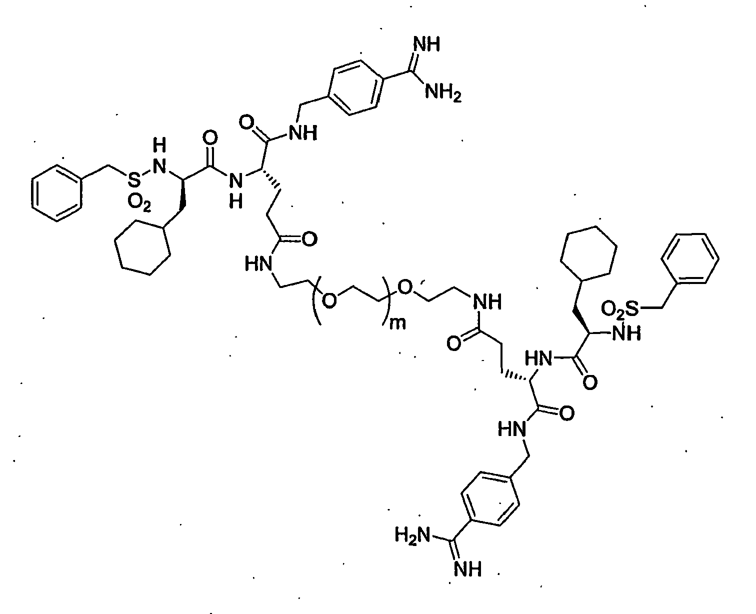 mit n =1 bis 3 und m=10 bis 1000, wobei q gleich 0 oder 1, insbesondere 0 ist und R₂ jeweils die oben genannten Bedeutungen besitzt. Durch das Fehlen einer zweiten funktionellen Gruppe können die aufgeführten Substanzen neben der kovalenten Kopplung an P2 nur nicht-kovalent an künstliche Oberflächen gekoppelt werden. Dies geschieht durch Interaktionen der Oligo- oder Polyalkylengruppe der Linkergruppe mit der künstlichen Oberfläche, beispielsweise aufgrund von Wasserstoffbrückenbindungen, hydrophoben Wechselwirkungen oder van der Waalsschen Wechselwirkungen. Die Substanzen, bei denen an eine Oligo- oder Polyalkylengruppe zwei Moleküle der Formel I gekoppelt sind, werden im Sinne der vorliegenden Erfindung als zweifach Inhibitor-funktionalisierte Oligo- oder Polyalkylenglycole bezeichnet.

Ein weiterer Vorteil dieser Oligo- und/oder Polyalkylenderivaten, die an einem Ende als reine Monomethylether vorliegen und so nicht zum kovalenten Koppeln geeignet sind, besteht wie bei den Derivaten, bei denen die Linkergruppe an P4 gekoppelt ist, in ihrer verlängerten Halbwertszeit in der Zirkulation nach systemischer Gabe.

Wenn die Kopplung an die künstliche Oberfläche über P2 erfolgt, ist der Substituent an P4 insbesondere H, ein Halogen, eine Aminogruppe, eine Hydroxygruppe oder eine lineare oder verzweigte Alkylgruppe mit 1 bis 6 Kohlenstoffatomen.

Eine besonders bevorzugte Ausführungsform eines acylierten Amidinobenzylamins gemäß der allgemeinen Formel I mit einer Linkergruppe an P4 gemäß der allgemeinen Formel II weist vorzugsweise die folgende Struktur auf: 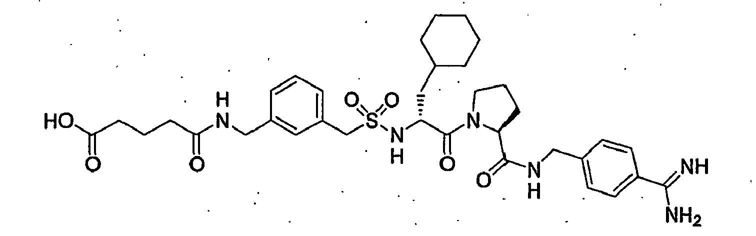 wobei D-Cha in Position P3 insbesondere auch D-Phe oder D-Ser(tBu) und Glutaryl an P4 auch Succinyl sein kann. Diese Verbindung eignet sich zur gleichzeitigen kovalenten Kopplung an eine künstliche Oberfläche.

Eine weitere besonders bevorzugte Ausführungsform eines acylierten Amidinobenzylamins gemäß der allgemeinen Formel I mit einer Linkergruppe an P2 gemäß den allgemeinen Formeln III und IV weist vorzugsweise die folgende Struktur auf: 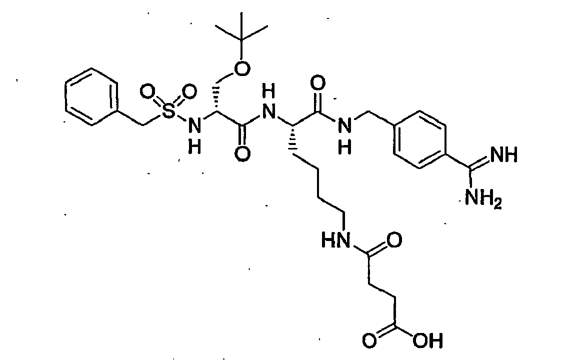 wobei D-Ser(tBu) in Position P3 insbesondere auch D-Cha oder D-Phe und Succinyl an P2 auch Glutaryl sein kann. Diese Verbindung eignet sich zur gleichzeitigen kovalenten Kopplung an eine künstliche Oberfläche.

Eine weitere besonders bevorzugte Ausführungsform eines acylierten Amidinobenzylamins gemäß der allgemeinen Formel I mit einer Linkergruppe an P2 gemäß den allgemeinen Formeln III und IV weist vorzugsweise eine der folgenden Strukturen auf: 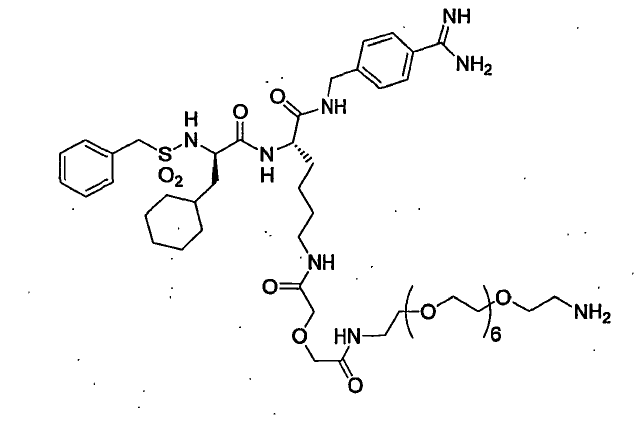 oder 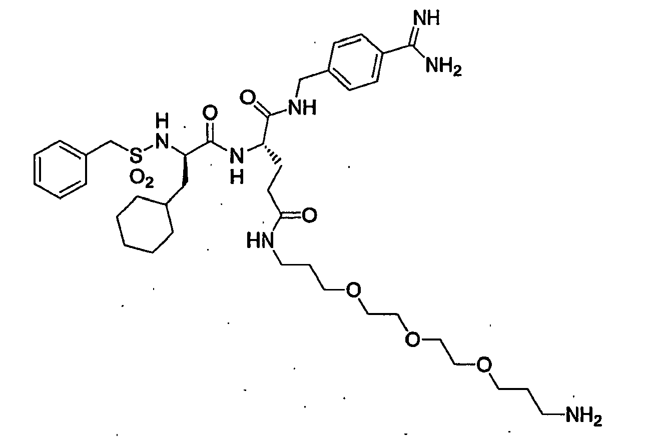 wobei D-Cha in Position P3 insbesondere auch D-Phe oder D-Ser(tBu) sein kann. Diese Verbindungen eignen sich zur gleichzeitigen kovalenten Kopplung an eine künstliche Oberfläche oder zur kovalenten Kopplung an ein zweites Molekül der allgemeinen Formel I.

Weitere mögliche Ausführungsbeispiele für acylierte Aminobenzylamine, die PK mit hoher Aktivität und Spezifität hemmen, sind Verbindungen gemäß Formel I, wobei P4 einen Rest R trägt, P3 D-Ser, D-Ser(tBu), D-Phe oder D-Cha und P2 eine natürliche oder unnatürliche Aminosäure Aaa bedeutet, wobei R gleich H-, 4-, 3- oder 2-, vorzugsweise 4- oder 3-COOH, 4-, 3- oder 2-, vorzugsweise 4- oder 3-COOMe, 4-, 3- oder 2-, vorzugsweise 4- oder 3-AMe, 4-, 3- oder 2-, vorzugsweise 4- oder 3-Glutaryl-AMe oder 4-, 3- oder 2-, vorzugsweise 4- oder 3-CN ist und Aaa gleich Gly, Ala, Pro, Asp, Glu, Gln, hGlu, Dap, Dap(Z), Lys, Lys(Z), Arg, Thr, Thr(Bzl), Ser, Ser(Bzl), hSer, hSer(Bzl), Phe oder hPhe ist.

Besonders bevorzugt sind dabei die acylierten Aminobenzylamine wobei bei P3 gleich D-Ser Aaa vorzugsweise Gln, Dap, Dap(Z), Lys, Lys(Z), Ser(Bzl), hSer, Phe oder hPhe, insbesondere Lys(Z) ist und R gleich H oder bei Aaa gleich Ala oder Ser R gleich HOOC- ist; oder bei P3 gleich D-Ser(tBu) Aaa gleich Pro, Gln, Dap, Dap(Z), Lys, Lys(Z), Arg, Thr, Thr(Bzl), Ser(Bzl), hSer(Bzl), Phe oder hPhe, insbesondere Pro, Gln, Lys, Lys(Z), hSer(Bzl), Phe oder hPhe ist und R gleich H oder bei Aaa gleich Gly oder Ala R gleich HOOC- ist oder bei Aaa gleich Pro R gleich CN- ist;
oder bei P3 gleich D-Cha Aaa gleich Lys oder Glu ist und R gleich H oder bei Aaa gleich Pro R gleich Glutaryl-AMe ist, insbesondere bei Aaa gleich -NH-CH-[CH₂-CH₂-CO-NH-(CH₂)₃-[O-(CH₂)₂]₃-CH₂-NH₂]-CO- R gleich H ist.

Die erfindungsgemäßen Derivate des acylierten 4-Amidino- bzw. 4-Guanidinobenzylamins liegen in der Regel in Form eines Salzes, insbesondere einer Mineralsäure beispielsweise Schwefelsäure oder Salzsäure oder einer geeigneten organischen Säure beispielsweise Essigsäure, Ameisensäure, Methylsulfonsäure, Bernsteinsäure, Apfelsäure oder Trifluoressigsäure, insbesondere als Hydrochlorid, Sulfat oder Acetat vor.

Eine weitere bevorzugte Ausführungsform der vorliegenden Erfindung ist die Umsetzung einer H₂N-Gruppe einer an das acylierte 4-Amidino- oder 4-Guanidinobenzylamin gekoppelten Linkergruppe mit einem Dicarbonsäureanhydrid, vorzugsweise dem Anhydrid der Bernsteinsäure oder der Glutarsäure unter Bildung einer HOOC-Gruppe oder die Umsetzung einer HOOC-Gruppe einer an das acylierte 4-Amidino- oder 4-Guanidinobenzylamin gekoppelten Linkergruppe mit einem Diamin unter Bildung einer H₂N-Gruppe. Diese Reaktionen erfolgen nach dem Fachmann bekannten Standardverfahren.

Die durch diese Reaktionen ermöglichte Umwandlung einer H₂N-Gruppe in eine HOOC-Gruppe und einer HOOC-Gruppe in eine H₂N-Gruppe erweitert die Kopplungsmöglichkeiten der Verbindungen der allgemeinen Formel I an künstliche Oberflächen oder ein zweites Molekül der allgemeinen Formel I.

In einer besonders bevorzugten Anwendungsform der vorliegenden Erfindung kann die kovalent an P4 oder P2 gekoppelte Linkergruppe bei Vorhandensein einer zweiten funktionellen Gruppe, insbesondere einer substituierten oder unsubstituierten Amino-, Carboxyl- und/oder Mercaptogruppe, gleichzeitig kovalent an künstliche Oberflächen oder sofern es sich bei der Linkergruppe um ein Oligo- oder Polyalkylenglycol handelt kovalent an ein zweites Molekül der allgemeinen Formel I gekoppelt sein, unter Bildung eines zweifach Inhibitor-funktionalisierten Oligo- oder Polyalkylenglycols bezeichnet.

Gemäß einer bevorzugten Ausführungsform der vorliegenden Erfindung besteht die künstliche Oberfläche, an die die Derivate des acylierten 4-Amidino- bzw. 4-Guanidinobenzylamins gekoppelt werden können, aus Cellulose-Diacetat, Cellulose-Triacetat, Poly(ethersulfon), Poly(arylethersulfon), regenerierter Cellulose, Cuprophan, Hemophan, Poly(sulfon), Poly(acrylnitril), Poly(vinylalkohol), Poly(carbonat), Poly(amid), Poly(methylmethacrylat), Poly(ethylen-co-vinylalkohol) oder einem anderen in Geräten wie Dialysatoren, Oxygenatoren, Kathetern, Membranen und/oder den zu den Geräten gehörenden Schlauchsystemen und/oder Luftfallen für die Oberflächen, die mit Blut in Kontakt kommen, verwendeten Material, wobei das Oberflächenmaterial für die kovalente Kopplung des Moleküls der allgemeinen Formel I über die an P4 oder P2 gekoppelte Linkergruppe gegebenenfalls mit funktionellen Gruppen, z.B. Aminogruppen, Aminoalkylgruppen, Carboxylgruppen, Carboxyalkylgruppen, Mercaptogruppen, Mercaptoalkylgruppen, Hydroxylgruppen, Hydroxyalkylgruppen, wobei der Alkylrest 1-10, insbesondere 1-6 C-Atome aufweist, modifiziert ist.

Gemäß einer weiteren bevorzugten Ausführungsform der vorliegenden Erfindung werden die Derivate des acylierten 4-Amidino- bzw. 4-Guanidinobenzylamins an künstliche Oberflächen von z.B. Geräten wie Dialysatoren, Oxygenatoren, Kathetern und/oder Membranen gekoppelt, um die Blutgerinnung an den Oberflächen dieser Geräte zu verhindern.

Die Kopplung der Derivate des acylierten 4-Amidino- bzw. 4-Guanidinobenzylamins erfolgt vorzugsweise durch kovalente oder nicht-kovalente Beschichtung der künstlichen Oberfläche(n) über eine der oben beschriebenen Linkergruppen, die an einen Substituenten an P4 und/oder gegebenenfalls direkt an die Seitenkette von P2 der allgemeinen Formel I gebunden ist.

Ein Gerät im Sinne der vorliegenden Erfindung ist jede Vorrichtung, die mit Blut und dessen Bestandteilen in Kontakt kommt.

Eine weitere bevorzugte Ausführungsform der vorliegenden Erfindung ist die Verwendung eines oder mehrerer der erfindungsgemäßen Derivate des acylierten 4-Amidino- bzw. 4-Guanidinobenzylamins zur Herstellung eines Arzneimittels zur Verwendung als Antikoagulanz und/oder Antithrombotikum zur Verhinderung und/oder Behandlung von Herzinfarkt, Hirnschlag, Embolien, tiefen Beinvenenthrombosen z.B. nach Hüftgelenksoperationen und/oder Kniegelenksersatz, instabiler Angina pectoris, Komplikationen infolge von Angioplastie, insbesondere perkutaner transluminaler Koronarangioplastie (PTCA).

Unter Antikoagulanz im Sinne der vorliegenden Erfindung ist jede Substanz zu verstehen, die die Blutgerinnung hemmt. Unter Antithrombotikum im Sinne der vorliegenden Erfmdung sind Substanzen zum Einsatz bei der Thromboseprophylaxe zu verstehen. Unter Angioplastie im Sinne der vorliegenden Erfindung ist eine Aufdehnung von Gefäßen zu verstehen, insbesondere mit Hilfe von Kathetern wie beispielsweise Ballonkathetern.

Eine weitere Ausführungsform ist die Verwendung eines oder mehrerer der oben beschriebenen acylierten 4-Amidino- bzw. 4-Guanidinobenzylamine zur Herstellung eines Arzneimittels zur Verwendung als Antikoagulanz und/oder Antithrombotikum zur Verhinderung und Therapie von disseminierter intravaskulärer Gerinnung, septischem Schock, Allergien, dem Postgastrektomiesyndrom, Arthritis und ARDS (adult respiratory distress syndrome).

Gemäß einer bevorzugten Ausführungsform der vorliegenden Erfindung werden die Derivate des acylierten 4-Amidino- bzw. 4-Guanidinobenzylamins zur Herstellung eines Arzneimittels zur Inhibition von Plasmakallikrein und/oder Faktor XIa und/oder Faktor XIIa in parenteraler Anwendungsform, insbesondere in intraarterieller, intravenöser, intramuskulär rer oder subkutaner Form, in enteraler Anwendungsform, insbesondere zur oralen oder rektalen Anwendung oder in topischer Anwendungsform, insbesondere als Dermatikum verwendet. Dabei sind intravenöse oder subkutane Anwendungsformen bevorzugt. Beispielsweise bevorzugt ist die Inhibition von Plasmakallikrein.

Die erfindungsgemäßen Derivate des acylierten 4-Amidino- bzw. 4-Guanidinobenzylamins können insbesondere zur Herstellung eines Arzneimittels zur Inhibition von Plasmakallikrein in Form einer Tablette, eines Dragees, einer Kapsel, eines Pellets, Suppositoriums, einer Lösung, insbesondere einer Injektions- oder Infusionslösung, von Augen-, Nasen und Ohrentropfen, eines Safts, einer Kapsel, einer Emulsion oder Suspension, eines Globuli, Styli, Aerosols, Puders, einer Paste, Creme oder Salbe verwendet werden.

Neben dem erfindungsgemäßen Inhibitor kann das Arzneimittel weitere pharmazeutisch geeignete Hilfs- und/oder Zusatzstoffe enthalten. Geeignete Hilfs- und/oder Zusatzstoffe, die z.B. der Stabilisierung und/oder Konservierung des Arzneimittels dienen, sind dem Fachmann allgemein geläufig (z.B. Sucker H. et al., (1991) Pharmazeutische Technologie, 2. Auflage, Georg Thieme Verlag, Stuttgart). Hierzu zählen beispielsweise physiologische Kochsalzlösungen, Ringer-Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Eine weitere Ausführungssform der vorliegenden Erfindung ist die Verwendung von acyliertem Amidinobenzylamin der allgemeinen Formel V oder VI mit R₄ insbesondere gleich HO- und R₁ und R₃ keine Oligo- oder Polyalkylengruppe zur Herstellung eines Arzneimittels zur Verwendung als Antikoagulanz und/oder Antithrombotikum bei oben aufgeführten Indikationen, wobei der Wirkstoff in Form eines Prodrugs zur oralen Gabe vorliegt.

Ein Prodrug im Sinne der vorliegenden Erfindung ist ein acyliertes Amidino- oder Guanidinobenzylamin gemäß der allgemeinen Formel I, das als pharmazeutisch inaktives Derivat der entsprechenden pharmazeutisch wirksamen Substanz vorliegt und nach oraler Gabe spontan oder enzymatisch biotransformiert wird unter Freisetzung der pharmazeutisch wirksamen Substanz.

Neben der bevorzugten Anwendung der beschriebenen acylierten Amidino- oder Guanidinobenzylamine zur Inhibierung von Plasmakallikrein können diese auch zur Inhibierung weiterer trypsinartiger Serinproteasen wie beispielsweise Thrombin, Faktor XIIa, Faktor Faktor XIa, Xa, Faktor IXa, Faktor VIIa, Urokinase, Tryptase und Plasmin sowie trypsinartiger Serinproteasen des Komplementsystems verwendet werden.

Ein weiterer Gegenstand der vorliegenden Erfindung ist acyliertes 4-Amidino- oder 4-Guanidinobenzylamin gemäß der allgemeinen Formel P4-P3-P2-P1- (I), wobei die Substanz über eine der oben beschriebenen Linkergruppen an P4 und/oder an P2 kovalent oder nicht-kovalent an eine künstliche Oberfläche gebunden ist. Dabei ist die Substanz vorzugsweise über eine Amid- oder Sulfonamidbindung, eine Disulfidbrücke oder die Alkylierung einer Mercaptogruppe, insbesondere über eine Amidbindung kovalent an eine künstliche Oberfläche gebunden. Eine nicht-kovalente Bindung der Substanz an eine künstliche Oberfläche erfolgt vorzugsweise über Interaktionen einer Oligo- oder Polyalkylenglykolgruppe, insbesondere einer Oligo- oder Polyethylenglykolgruppe, mit einer künstlichen Oberfläche.

Ein weiterer Gegenstand der vorliegenden Erfindung ist eine künstliche Oberfläche, wobei die Oberfläche mit einem erfindungsgemäßen acylierten 4-Amidino- oder 4-Guanidinobenzylamin kovalent oder nicht-kovalent beschichtet ist. Ein weiterer Gegenstand der vorliegenden Erfindung ist auch ein Gerät, beispielsweise ein Dialysator, Oxygenator, Katheter oder eine Membran mit den zugehörigen Schlauchsystemen und/oder Luftfallen, das eine mit einem erfindungsgemäßen acylierten 4-Amidino- oder 4-Guanidinobenzylamin kovalent oder nicht-kovalent beschichtete künstliche Oberfläche enthält.

Die Synthese der erfindungsgemäßen Derivate des acylierten 4-Amidino- oder 4-Guamdmobenzylamins erfolgt nach dem Fachmann bekannten Methoden. Beispielsweise wird aus dem kommerziell erhältlichen 4-Cyanobenzylamin (Showa Denko, Japan) das Boc-geschützte 4-(Acetyloxamidino)Benzylamin nach dem Fachmann bekannten Verfahren gewonnen. Eine weitere Möglichkeit besteht darin, dass man 4-Cyanobenzylamin direkt an die Boc- oder Z-geschützte P2-Aminosäure koppelt und auf dieser Stufe die Cyanogruppe in das Acetyloxamidin überführt. Nach Abspaltung der Boc-Schutzgruppe erfolgt die Ankopplung der weiteren Aminosäuren mittels Standardkopplungsmethoden mit Boc als N-terminaler Schutzgruppe. Die P3-Aminosäure kann auch direkt als N-aryl- bzw. N-aralkyl-sulfonyl-geschützte Aminosäure gekoppelt werden. Die meisten Zwischenprodukte kristallisieren gut und lassen sich damit einfach reinigen. Die Endreinigung der Inhibitoren erfolgt auf der letzten Stufe vorzugsweise über präparative, reversed-phase HPLC.

Die Erfindung soll nachstehend anhand der angefügten Ausführungsbeispiele und Tabellen näher erläutert werden, ohne sie zu beschränken.

### Verwendete Abkürzungen

- Aaa: Aminosäure
- Ac: Acetyl
- AcOH: Essigsäure
- ACN: Acetonitril
- Amba: Amidinobenzylamin
- AMe: Aminomethyl
- ARDS: adult respiratory distress syndrome
- Boc: tert.-Butyloxycarbonyl
- Bzl: Benzyl
- Bzls: Benzylsulfonyl
- Can: Canavanin
- Cha: Cyclohexylalanin
- CKIBE: Chlorkohlensäureisobutylester
- CNBzls: Cyanobenzylsulfonyl
- Dab: α,γ-Diaminobuttersäure
- Dap: α,β-Diaminopropionsäure
- Dap(Z): Benzyloxycarbonyl-α,γ-Diaminobuttersäure
- DCM: Dichlormethan
- DIEA: Diisopropylethylamin
- DMF: N,N-Dimethylformamid
- D-Ser: D-Serin, andere Aminosäuren entsprechend
- D-Ser(tBu): D-(tert.-Butylserin)
- F XIa: Faktor XIa
- F XIIa: Faktor XIIa
- Glut: Glutaryl
- GuMe: Guanidinomethylen
- hAla(4-Pyr): Homo-4-Pyridyl-Alanin 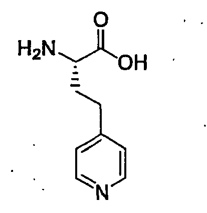
- hGlu: beta-Homoglutaminsäure 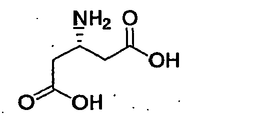
- hPhe: Homophenylalanin 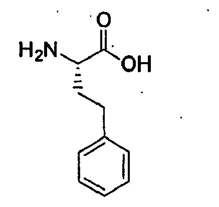
- hSer: beta-Homoserin 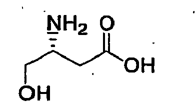
- hTyr: Homotyrosin 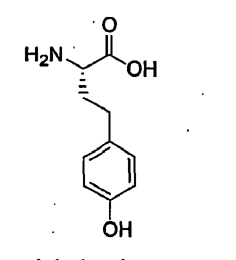
- n.b.: nicht bestimmt
- PEG: Polyethylenglykol
- Phe: Phenylalanin
- PK: Plasmakallikrein
- Pro-MMP 3: Pro-Matrixmetalloprotease 3
- PyBop: Benzotriazol-1-yl-N-oxy-tris(pyrrolidino)phosphoniumhexafluorophosphat
- RT: Raumtemperatur
- Ser(Bzl): Serin(Benzyl)
- Suc: Succinyl
- TFA: Trifluoressigsäure
- Tfa: Trifluoracetyl
- Z: Benzyloxycarbonyl

### Analytische Methoden:

Analytische HPLC: Shimadzu LC-10A System, Säule: Phenomenex Luna C₁₈, 5 µm (250 x 4 mm), Lösungsmittel A: 0,1 % TFA in Wasser; B: 0,1 % B in ACN, Gradient: 10 % B bis 70 % B in 60 min, 1 ml/min Flussrate, Detektion bei 220 nm.

Präparative HPLC: Shimadzu LC-8A System, Säule: Phenomenex Luna C₁₈, 5 µm (250 x 30 mm), Lösungsmittel A: 0,1 % TFA in Wasser; B: 0,1 % B in ACN, Gradient: 10 % B bis 55 % B in 120 min, 10 ml/min Flussrate, Detektion bei 220 nm. Massenspektroskopie: Die Massenspektren wurden entweder auf einem Kompact Probe der Firma Kratos (Manchester, UK) mit einem Flugzeitmessungsdetektor und α-Cyano-Hydroxyzimtsäure als Matrix gemessen oder mit einem ESI-MS LCQ der Firma Finnigan (Bremen, Deutschland) bestimmt.

### Ausführungsbeispiel 1:

Synthese von 3(Glutaryl-Amidomethyl)Benzylsulfonyl-D-Cha-Pro-4-Amidinobenzylamid x TFA 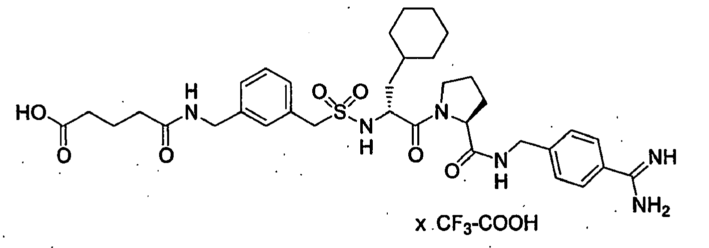
1a) 3-(Cyano)Benzylsulfonsäure Natrium-Salz
   30 g (153 mmol) 3-Cyanobenzylbromid (Aldrich) wurden in 150 ml Wasser suspendiert und nach Zugabe von 21,2 g (168,3 mmol) Na₂SO₃ 8 h unter Rückfluss gekocht. Der Ansatz wurde heiß filtriert und das Wasser im Vakuum etwas eingeengt. Der Ansatz wurde zur Kristallisation über Nacht im Kühlschrank aufbewahrt, danach wurden die Kristalle abgesaugt und nochmals aus Wasser umkristallisiert. Die Kristalle wurden abgesaugt und im Vakuum getrocknet. Ausbeute: 17,1 g (78 mmol), HPLC: 18,2 % B
1b) 3-(Cyano)Benzylsulfonylchlorid 5 g (22,83 mmol) 3-Cyano-Benzylsulfonsäure Natrium-Salz wurden mit ca. 20 ml Phosphorylchlorid angefeuchtet und mit 5,2 g (25,11 mmol) PCl₅ versetzt und 15 min unter Eiskühlung gerührt. Anschließend wurde der Ansatz 4 h auf 80 °C erwärmt. Danach wurde der Ansatz auf Eis gegossen und 30 min kräftig gerührt, das Produkt schied sich als weißer Feststoff auf dem Eis ab. Nachdem das Eis teilweise aufgetaut war, wurde der Ansatz durch eine Fritte filtriert und das verbleibende Produkt-Eis-Gemisch mehrmals mit Wasser gewaschen. Die verbleibenden Kristalle wurden im Vakuum getrocknet und direkt für den nächsten Syntheseschritt verwendet.
   Ausbeute: 3,4 g (15,8 mmol)
1c) 3-(Cyano)Benzylsulfonyl-D-Cha-OH
   3,775 g (22 mmol) H-D-Cha-OH wurden in 100 ml trockenem DCM suspendiert und mit 6,316 ml (50 mmol) Trimethylsilylchlorid und 8,7 ml (50 mmol) DIEA versetzt. Der Ansatz wurde 1 h unter Rückfluss gekocht und im Eisbad abgekühlt. Anschließend wurden 5 g (23,18 mmol) 3-Cyano-Benzylsulfonylchlorid und 5 ml (28,75 mmol) DIEA innerhalb von 30 min zugesetzt. Der Ansatz wurde weitere 30 min unter Eiskühlung und anschließend für weitere 3 h bei Raumtemperatur gerührt. Das Lösungsmittel wurde im Vakuum entfernt, der Rückstand in Wasser (mit 1 N NaOH auf pH 8,5-9 gebracht) gelöst und 2 x mit Essigester extrahiert. Die Essigesterphase wurde anschließend nochmals mit basischem Wasser (pH 9, NaOH) extrahiert. Die vereinten basischen Wasserphasen wurden anschließend mit konzentrierter HCl-Lösung angesäuert (pH ca. 3) und 3 x mit Essigester extrahiert. Die vereinte Essigesterphase wurde jeweils 3 x mit 5 % KHSO₄-Lösung und NaCl-gesättigter Lösung gewaschen und über Na₂SO₄ getrocknet. Das Lösungsmittel wurde im Vakuum entfernt.
   Ausbeute: 6,99 g Öl, das im Kühlschrank langsam kristallisiert, HPLC: 53,9 % B
1d) H-Pro-4(Acetyloxamidino)Benzylamid x HBr
   5 g Z-Pro-4(Acetyloxamidino)Benzylamid (synthetisiert wie beschrieben in WO 02/059065) wurden mit 75 ml HBr-Lösung (33 %ig in Essigsäure) bei Raumtemperatur versetzt. Der Ansatz wurde eine Stunde stehen gelassen und gelegentlich umgeschüttelt. Danach wurde der Ansatz mit Ether versetzt und das ausgefallene Produkt abgesaugt und auf der Fritte mehrmals mit Ether gewaschen. Das Produkt wurde im Vakuum getrocknet.
   Ausbeute: 4,3 g (11,16 mmol), HPLC 18,3 % B
1e) 3-(Cyano)Benzylsulfonyl-D-Cha-Pro-4(Acetyloxamidino)Benzylamid 2,5 g (7,13 mmol) 3-Cyano-Benzylsulfonyl-D-Cha-OH und 2,74 g (7,13 g) H-Pro-4-(Acetyl-oxamidino)Benzylamid x HBr wurden in 50 ml DMF gelöst. Unter Eiskühlung wurden 3,71 g (7,13 mmol) PyBop und 3,7 ml DIEA hinzugefügt. Der Ansatz wurde 30 min unter Eiskühlung und anschließend 3 h bei RT gerührt. Das Lösungsmittel wurde im Vakuum entfernt, der Ansatz in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen. Die Essigesterphase wurde mit Na₂SO₄ getrocknet und anschließend das Lösungsmittel im Vakuum entfernt. Das Rohprodukt wurde ohne weitere Reinigung für den nächsten Syntheseschritt verwendet.
   Ausbeute: 3,3 g Öl, HPLC bei 53,77 % B
   MS: berechnet 578,27 (monoisotopic), gefunden 579,4 [M+H]⁺
1f) 3-(Aminomethyl)Benzylsulfonyl-D-Cha-Pro-4(Amidino)Benzylamid x
   2 HCl
   1 g Rohprodukt an 3-Cyano-Benzylsulfonyl-D-Cha-Pro-4-(Acetyloxamidino) Benzylamid wurde in 500 ml Essigsäure gelöst und mit 150 ml 1 N HCl versetzt. Danach wurden 200 mg Katalysator (10 % Palladium auf Aktivkohle) hinzugefügt und der Ansatz bei 50 °C 15 h mit Wasserstoff hydriert. Der Katalysator wurde abfiltriert und das Lösungsmittel im Vakuum eingeengt. Der Rückstand wurde mit Toluol versetzt und das Lösungsmittel im Vakuum entfernt, der Vorgang wurde noch 2 x wiederholt. Der Rückstand wurde in wenig Methanol gelöst und das Produkt durch Zugabe von Ether gefällt und abgesaugt. Das Produkt wurde mit Ether gewaschen und im Vakuum getrocknet. Das Rohprodukt wurde ohne weitere Reinigung für den nächsten Syntheseschritt eingesetzt.
   Ausbeute: 0,8 g, HPLC bei 34,28 % B
   MS: berechnet 582,30 (monoisotopic), gefunden 583,5 [M+H]⁺
1 g) 3-(Glutarylamidomethyl)Benzylsulfonyl-D-Cha-Pro-4(Amidino)Benzylamid x TFA 200 mg (ca. 0,3 mmol) Rohprodukt an 3-(Aminomethyl)Benzylsulfonyl-D-Cha-Pro-4(Arnidino)Benzylamid x 2 HCl wurden mit 38 mg (0,33 mmol) Glutarsäureanhydrid und 115 µl (0,66 mmol) DIEA in 5 ml DMF unter Eiskühlung versetzt. Der Ansatz wurde 30 min unter Eiskühlung und weitere 3 h bei RT gerührt. Das Lösungsmittel wurde i. V. entfernt und das Rohprodukt mittels präparativer reversed-phase HPLC gereinigt.
   Ausbeute: 125 mg, HPLC bei 40,1 % B
   MS: berechnet 696,33 (monoisotopic), gefunden 697,8 [M+H]⁺

### Ausführungsbeispiel 2:

Synthese von Benzylsulfonyl-D-Ser(tBu)-Lys(Succinyl)-4-Amba x TFA 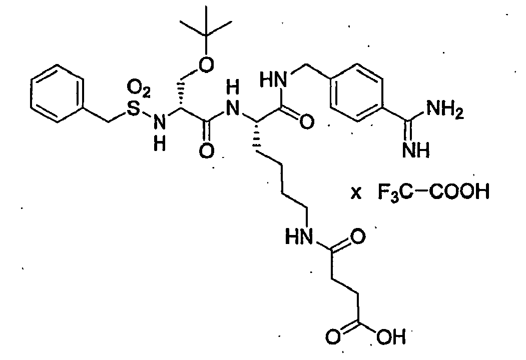
2a) Boc-Lys(Tfa)-4-(Acetyloxamidino)Benzylamid 5 g (14,61 mmol) Boc-Lys(Tfa)-OH wurden in 100 ml THF gelöst und bei -15 °C mit 1,767 ml (16,10 mmol) NMM und 1,899 ml (14,61 mmol) CKIBE versetzt. Der Ansatz wurde 10 min bei -15°C gerührt, dann wurden 3,74 g (15,33 mmol) 4-(Acetyloxamidino)Benzylamin x HCl (hergestellt wie in WO 01/96286 A2 beschrieben) und nochmals 1,767 ml (16,10 mmol) NMM hinzugefügt. Der Ansatz wurde eine weitere Stunde bei -15 °C und über Nacht bei Raumtemperatur gerührt. Das Lösungsmittel wurde im Vakuum entfernt, der Ansatz in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen und mit Na₂SO₄ getrocknet. Das Lösungsmittel wurde im Vakuum entfernt und das Produkt aus Essigester kristallisiert.
   Ausbeute: 6,82 g (12,83 mmol) weiße Kristalle, HPLC: 43,87 % B
2b) H-Lys(Tfa)-4-(Acetyloxamidino)Benzylamid x HCl 5 g (9,41 mmol) Boc-Lys(Tfa)-4-(Acetyloxamidino)Benzylamid wurden in wenig Eisessig angelöst und anschließend mit 100 ml 1 N HCl in Eisessig versetzt. Nach 45 min Stehen bei Raumtemperatur wurde das Lösungsmittel teilweise eingeengt und das Produkt durch Zugabe von Diethylether gefällt, abgesaugt und nochmals mit Diethylether gewaschen.
   Das Produkt wurde im Vakuum getrocknet.
   Ausbeute: 4,65 g (10,78 mmol) weißer Feststoff, HPLC: 25,52 % B
2c) Bzls-D-Ser(tBu)-Lys(Tfa)-4-(Acetyloxamidino)Benzylamid
   1,93 g (6,107 mmol) Bzls-D-Ser(tBu)-OH und 3 g (6,412 mmol) H-Lys(Tfa)-4-(Acetyloxamidino)Benzylamid x HCl wurden in 30 ml Acetonitril gelöst und bei 0 °C mit 3,337 g (6,412 mmol) PyBop und 3,187 ml (18,32 mmol) DIEA versetzt. Der Ansatz wurde 30 min bei 0 °C und weitere 4 h bei Raumtemperatur gerührt. Das Lösungsmittel wurde im Vakuum entfernt, der Rückstand in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen und anschließend mit Na₂SO₄ getrocknet. Das Lösungsmittel wurde im Vakuum entfernt. Es verblieb ein leicht gelbes, amorphes Rohprodukt, das ohne weitere Reinigung direkt für den nächsten Syntheseschritt eingesetzt wurde. Ausbeute: 5,88 g (Rohprodukt), HPLC: 52,93 % B
2d) Bzls-D-Ser(tBu)-Lys(Tfa)-4-(Amidino)Benzylamid x Acetat
   5,88 g (Rohprodukt) Bzls-D-Ser(tBu)-Lys(Tfa)-4-(Acetyloxamidino) Benzylamid wurden in 150 ml 90 % Essigsäure gelöst, dazu wurden 500 mg Katalysator (10 % Pd/C) gegeben. Der Ansatz wurde 6 h bei Raumtemperatur und Normaldruck mit Wasserstoff hydriert. Anschließend wurde der Katalysator abfiltriert, das Lösungsmittel teilweise eingeengt und das Produkt durch Zugabe von Diethylether gefällt, abgesaugt und nochmals mit Diethylether gewaschen. Der weiße, kristalline Niederschlag wurde im Vakuum getrocknet.
   Ausbeute: 4,36 g (5,962 mmol), HPLC: 43,50 % B.
2e) Bzls-D-Ser(tBu)-Lys-4-(Amidino)Benzylamid x 2 TFA 0,2 g Rohprodukt an Bzls-D-Ser(tBu)-Lys(Tfa)-4-(Amidino)Benzylamid x Acetat wurden unter Eiskühlung mit 5 ml 1M wässriger Piperidinlösung versetzt und 3 h gerührt. Das Lösungsmittel wurde danach im Vakuum eingeengt und der verbleibende Rückstand mit präparativer reversed-phase HPLC gereinigt.
   Ausbeute: 72 mg, HPLC: 30,9 % B
   MS: berechnet 574,29 (monoisotopic), gefunden 575,7 [M+H]⁺
2f) Bzls-D-Ser(tBu)-Lys(Succinyl)-4-(Amidino)Benzylamid x TFA
   60 mg (0,075 mmol) Bzls-D-Ser(tBu)-Lys-4-(Amidino)Benzylamid x 2 TFA wurden unter Eiskühlung mit 2 ml DMF, 7,8 mg (0,078 mmol) Bernsteinsäureanhydrid und 27,1 µl (0,156 mmol) DIEA versetzt. Der Ansatz wurde noch 30 min unter Eiskühlung und anschließend 3 h bei Raumtemperatur gerührt. Das Lösungsmittel wurde im Vakuum entfernt und das Produkt mit präparativer reversed-phase HPLC gereinigt.
   Ausbeute: 41 mg, HPLC: 35,8 % B
   MS: berechnet 674,31 (monoisotopic), gefunden 675,9 [M+H]⁺

### Ausführungsbeispiel 3:

Synthese von Benzylsulfonyl-D-Cha-Lys(CO-CH₂-O-CH₂-CO-NH-CH₂-CH₂-Hexaethylenglycol-CH₂-CH₂-NH₂)-4-Amba x 2 TFA 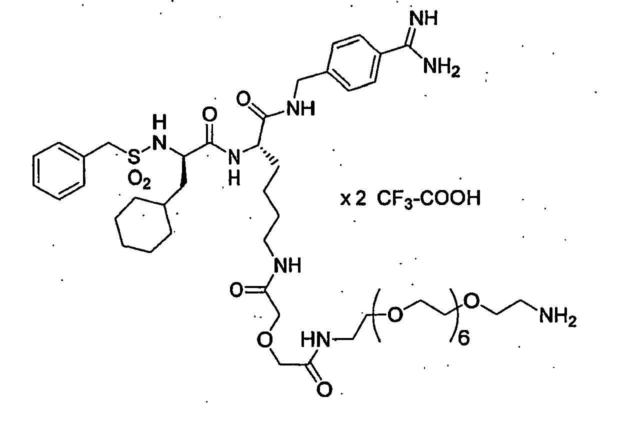
3a) Benzylsulfonyl-D-Cha-OH 6 g (35,1 mmol) H-D-Cha-OH wurden in 120 ml trockenem DCM suspendiert, mit 9,75 ml (77,2 mmol) Trimethylsilylchlorid und 13,4 ml (77,2 mmol) DIEA versetzt. Der Ansatz wurde 1 h unter Rückfluss gekocht und im Eisbad abgekühlt. Anschließend wurden 7,02 g (36,85 mmol) Benzylsulfonylchlorid und 7,83 ml (45 mmol) DIEA innerhalb von 30 min zugesetzt. Der Ansatz wurde weitere 30 min unter Eiskühlung und danach für weitere 3 h bei Raumtemperatur gerührt. Das Lösungsmittel wurde im Vakuum entfernt, der Rückstand in Wasser (mit 1 N NaOH auf pH 8,5-9 gebracht) gelöst und 2 x mit Essigester extrahiert. Die basische Wasserphase wurde anschließend mit konzentrierter HCl-Lösung angesäuert (pH ca. 3) und 3 x mit Essigester extrahiert. Die vereinte Essigesterphase wurde jeweils 3 x mit 5 % KHSO₄-Lösung und NaCl-gesättigter Lösung gewaschen und mit Na₂SO₄ getrocknet. Das Lösungsmittel wurde im Vakuum entfernt.
   Ausbeute: 9,2 g Öl (kristallisiert langsam im Kühlschrank), HPLC: 55,8 % B
3b) Boc-Lys(Z)-4-(Acetyloxamidino)Benzylamid
   4,41 g (11,59 mmol) Boc-Lys(Z)-OH wurden in 125 ml DMF gelöst und bei -15 °C mit 1,275 ml (11,59 mmol) NMM und 1,506 ml (11,59 mmol) CKIBE versetzt. Der Ansatz wurde 10 min bei -15°C gerührt, dann wurden 2,97 g (12,17 mmol) 4-(Acetyloxamidino)Benzylamin x HCl (hergestellt wie in WO 01/96286 A2 beschrieben) und nochmals 1,34 ml (12,17 mmol) NMM hinzugefügt. Der Ansatz wurde eine weitere Stunde bei -15 °C und über Nacht bei Raumtemperatur gerührt. Das Lösungsmittel wurde im Vakuum entfernt, der Ansatz in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen und mit Na₂SO₄ getrocknet. Das Lösungsmittel wurde im Vakuum entfernt, die verbleibende amorphe Substanz wurde im Vakuum getrocknet.
   Ausbeute: 5,2 g, HPLC: 51,12 % B
3c) H-Lys(Z)-4-(Acetyloxamidino)Benzylamid x HCl
   5 g Boc-Lys(Z)-4-(Acetyloxamidino)Benzylamid wurden mit 100 ml 1 N HCl in Eisessig versetzt. Nach 45 min Stehen bei Raumtemperatur wurde das Lösungsmittel teilweise eingeengt und das Produkt durch Zugabe von Diethylether gefällt, abgesaugt und nochmals mit Diethylether gewaschen. Das Produkt wurde im Vakuum getrocknet.
   Ausbeute: 4,2 g (8,3 mmol) weißer Feststoff, HPLC: 33,81 % B 3d) Bzls-D-Cha-Lys(Z)-4-(Acetyloxamidino)Benzylamid
   2 g (6,146 mmol) Bzls-D-Cha-OH und 3,13 g (6,146 mmol) H-Lys(Z)-4-(Acetyloxamidino)Benzylamid x HCl wurden in 50 ml DMF gelöst und bei 0 °C mit 3,198 g (6,146 mmol) PyBop und 3,2 ml (18,43 mmol) DIEA versetzt. Der Ansatz wurde 30 min bei 0 °C und weitere 5 h bei Raumtemperatur gerührt. Das Lösungsmittel wurde im Vakuum entfernt, der Rückstand in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen und anschließend mit Na₂SO₄ getrocknet. Das Lösungsmittel wurde im Vakuum entfernt. Das Rohprodukt wurde ohne weitere Reinigung direkt für den nächsten Syntheseschritt eingesetzt.
   Ausbeute: 3,7 g (Rohprodukt), HPLC: 61,84 % B
3e) Bzls-D-Cha-Lys-4-(Amidino)Benzylamid x 2 HBr
   3,5 g (Rohprodukt) Bzls-D-Cha-Lys(Z)-4-(Acetyloxamidino)Benzylamid wurden in 175 ml 90 % Essigsäure gelöst, dazu wurden 400 mg Katalysator (10 % Pd/C) gegeben. Der Ansatz wurde 6 h bei Raumtemperatur und Normaldruck mit Wasserstoff hydriert. Anschließend wurde der Katalysator abfiltriert, das Lösungsmittel eingeengt, der Rückstand mit Toluol versetzt und das Lösungsmittel wieder im Vakuum eingeengt. Zu dem Rückstand wurden 50 ml Bromwasserstoff-Lösung (33 %ig in Essigsäure) gegeben, der Ansatz wurde gelegentlich umgeschüttelt. Nach einer Stunde wurde das Produkt durch Zugabe von Diethylether gefällt, abgesaugt und mehrmals mit Diethylether gewaschen. Der erhaltene Feststoff (schwach gelblich) wurde im Vakuum getrocknet. Das Rohprodukt wurde für den nächsten Syntheseschritt eingesetzt.
   Ausbeute: 2,3 g Rohprodukt, HPLC: 34,77 % B.
   Ein Teil des Rohproduktes wurde mit präparativer reversed-phase HPLC gereinigt.
   MS: berechnet 584,31 (monoisotopic), gefunden 585,4 [M+H]⁺
3f) Bzls-D-Cha-Lys(CO-CH₂-O-CH₂-CO-NH-CH₂-CH₂-Hexaethyleneglycol-CH₂-CH₂-NH-Boc)-4-(Amidino)Benzylamid x HBr
   0,318 g (ca. 0,427 mmol) Rohprodukt an Bzls-D-Cha-Lys-4-(Amidino)Benzylamid x 2 HBr und 250 mg (0,4275 mmol) O-(N-Boc-2-Aminoethyl)-O'-(N-Diglycolyl)-2-Aminoethyl)-Hexaethylenglycol (Novabiochem, Bestell-Nr: 01-63-0102) wurden in 10 ml DMF gelöst. Unter Eiskühlung wurden 0,222 g (0,4275 mmol) PyBop und 149 µl (0,855 mmol) DIEA hinzugegeben. Der Ansatz wurde 15 min unter Eiskühlung und weitere 4 h bei Raumtemperatur gerührt. Danach wurde das Lösungsmittel im Vakuum eingeengt und der Rückstand in ca. 350 ml Essigester und 75 ml gesättigter NaHCO₃-Lösung aufgenommen. Die Essigesterphase wurde noch einmal mit gesättigter NaHCO₃-Lösung und 2 x mit gesättigter NaCl-Lösung gewaschen und mit Na₂SO₄ getrocknet. Das Lösungsmittel wurde im Vakuum entfernt, es wurde ein gelbes Öl erhalten, das ohne weitere Reinigung für den nächsten Syntheseschritt verwendet wurde.
   Ausbeute: 446 mg, HPLC: 47,03 % B
   Ein Teil der Verbindung wurde mit präparativer HPLC gereinigt.
3g) Bzls-D-Cha-Lys(CO-CH₂-O-CH₂-CO-NH-CH₂-CH₂-Hexaethyleneglycol-CH₂-CH₂-NH₂)-4-(Amidino)Benzylamid x 2 TFA
   400 mg der Verbindung 3f (Rohprodukt an Bzls-D-Cha-Lys(CO-CH₂O-CH₂-CO-NH-CH₂-CH₂-Hexaethyleneglycol-CH₂-CH₂-NH-Boc)-4-(Amidino)Benzylamid x HBr) wurden mit 10 ml 1 N HCl in Essigsäure versetzt. Nach einer Stunde bei Raumtemperatur wurde das Produkt durch Zugabe von Diethylether gefällt, abgesaugt und mittels präparativer HPLC gereinigt.
   Ausbeute: 210 mg, HPLC: 37,2 % B
   MS: berechnet 1050,57 (monoisotopic), gefunden 1051,6 [M+H]⁺

### Ausführungsbeispiel 4:

Synthese von Benzylsulfonyl-D-Cha-Glu(NH-[CH₂]₃-[O-CH₂-CH₂]₂-O-[CH₂]₃-NH₂)-4-Amba x 2 TFA 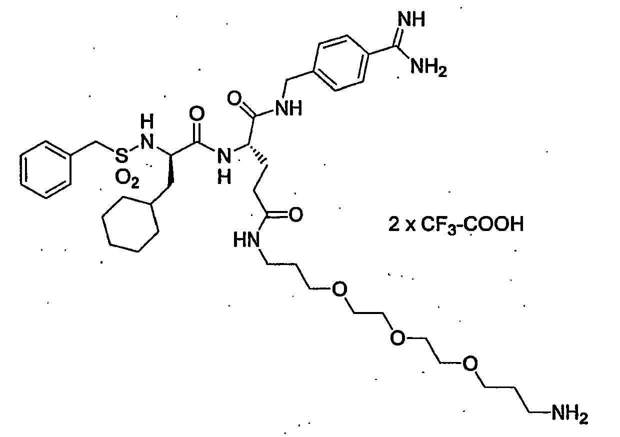
4a) Boc-Glu(OBzl)-4-(Acetyloxamidino)Benzylamid
   3,37 g (10 mmol) Boc-Glu(OBzl)-OH wurden in 100 ml DMF gelöst und bei -15 °C mit 1,1 ml (10 mmol) NMM und 1,3 ml (10 mmol) CKIBE versetzt. Der Ansatz wurde 8 min bei -15°C gerührt, dann wurden 2,44 g (10 mmol) 4-(Acetyloxamidino)Benzylamin x HCl (hergestellt wie in WO 01/96286 A2 beschrieben) und nochmals 1,1 ml (10 mmol) NMM hinzugefügt. Der Ansatz wurde eine weitere Stunde bei -15 °C und über Nacht bei Raumtemperatur gerührt. Das Lösungsmittel wurde im Vakuum entfernt, der Ansatz in Essigester aufgenommen und jeweils 3 x mit 5 % KHSO₄, NaCl-gesättigtem Wasser, gesättigter NaHCO₃-Lösung und nochmals mit NaCl-gesättigtem Wasser gewaschen und mit Na₂SO₄ getrocknet. Das Lösungsmittel wurde im Vakuum entfernt und die Verbindung aus Essigester kristallisiert.
   Ausbeute: 3,8 g (7,2 mmol), HPLC: 52,34 % B
4b) H-Glu(OBzl)-4-(Acetyloxamidino)Benzylamid x HCl
   3 g (6 mmol) Boc-Glu(OBzl)-4-(Acetyloxamidino)Benzylamid wurden mit 80 ml 1 N HCl in Eisessig versetzt. Nach 45 min Stehen bei Raumtemperatur wurde das Lösungsmittel teilweise eingeengt und das Produkt durch Zugabe von Diethylether gefällt, abgesaugt und nochmals mit Diethylether gewaschen. Das Produkt wurde im Vakuum getrocknet.
   Ausbeute: 2,5 g (5,4 mmol) weißer Feststoff, HPLC: 31,07 % B
4c) Bzls-D-Cha-Glu(OBzl)-4-(Acetyloxamidino)Benzylamid
   0,84 g (2,59 mmol) Bzls-D-Cha-OH und 1,2 g (2,59 mmol) H-Glu(OBzl)-4-(Acetyloxamidino)Benzylamid x HCl wurden in 40 ml DMF gelöst und bei 0 °C mit 1,35 g (2,59 mmol) PyBop und 1,35 ml (7,77 mmol) DIEA versetzt. Der Ansatz wurde 30 min bei 0 °C und weitere 4 h bei Raumtemperatur gerührt. Das Lösungsmittel wurde im Vakuum entfernt, der Rückstand in Essigester aufgenommen und jeweils 3 x mit 5% KHSO₄, NaCl-gesättigtem Wasser, gesättigter NaHCO₃-Lösung und nochmals mit NaCl-gesättigtem Wasser gewaschen und anschließend mit Na₂SO₄ getrocknet. Das Lösungsmittel wurde im Vakuum entfernt.
   Ausbeute: 1,35 g (Öl), HPLC: 63,16 % B
4d) Bzls-D-Cha-Glu-4-(Amidino)Benzylamid x HCl
   1,2 g Bzls-D-Cha-Glu(OBzl)-4-(Acetyloxamidino)Benzylamid wurden in 200 ml 90 % Essigsäure gelöst, dazu wurden 200 mg Katalysator (10 % Pd/C) gegeben. Der Ansatz wurde 24 h bei 45 °C und Normaldruck mit Wasserstoff hydriert. Anschließend wurde der Katalysator abfiltriert, das Lösungsmittel eingeengt, der Rückstand mit Toluol versetzt und das Lösungsmittel wieder im Vakuum eingeengt. Der Rückstand wurde in 25 ml 1 N HCl/Eisessig-Lösung gelöst und das Produkt durch Zugabe von Diethylether gefällt, abgesaugt und mehrmals mit Diethylether gewaschen. Der erhaltene Feststoff wurde im Vakuum getrocknet.
   Ausbeute: 0,82 g, HPLC: 40,55 % B.
   Ein Teil des Rohproduktes wurde mit präparativer reversed-phase HPLC gereinigt.
   MS: berechnet 585,26 (monoisotopic), gefunden 586,5 [M+H]⁺
4e) Bzls-D-Cha-Glu(NH-[CH₂]₃-[O-CH₂-CH₂]₂-O-[CH₂]₃-NH-Boc)-4-(Amidino) Benzylamid x HCl
   0,4 g (0,643 mmol) Bzls-D-Cha-Glu-4-(Amidino)Benzylamid x HCl und 0,216 g (0,675 mmol) Boc-NH-(CH₂)₃-(O-CH₂-CH₂)₂-O-(CH₂)₃-NH₂ (bezogen von Quanta Biodesign, Powell, Ohio) wurden in 10 ml DMF gelöst und bei 0 °C mit 0,335 g (0,643 mmol) PyBop und 224 µl (1,29 mmol) DIEA versetzt. Der Ansatz wurde 30 min bei 0 °C und weitere 6 h bei Raumtemperatur gerührt. Das Lösungsmittel wurde im Vakuum entfernt und der Rückstand wurde in einer Mischung aus Essigester und gesättigter NaHCO₃-Lösung aufgenommen. Die Mischung wurde im Scheidetrichter geschüttelt und die basische Phase abgetrennt. Die Essigesterphase wurde nochmals mit gesättigter NaHCO₃-Lösung gewaschen. Der Essigester wurde im Vakuum entfernt und der verbleibende Rückstand ohne Reinigung für den nächsten Syntheseschritt eingesetzt.
   Ausbeute: 0,35 g (Öl), HPLC: 49,17 % B
4f) Bzls-D-Cha-Glu(NH-[CH₂]₃-[O-CH₂-CH₂]₂-O-[CH₂]₃-NH₂)-4-(Amidino)Benzylamid x 2 TFA
   Das Rohprodukt der Verbindung 4e (Bzls-D-Cha-Glu(NH-[CH₂]₃-[O-CH₂-CH₂]₂-O-[CH₂]₃-NH-Boc)-4-(Amidino)Benzylamid x HCl) wurde mit 20 ml 1 N HCl in Eisessig versetzt. Der Ansatz wurde 45 min stehen gelassen und danach das Produkt durch Zugabe von Diethylether gefällt und abgesaugt. Der erhaltene Feststoff wurde mittel präparativer reversed-phase HPLC gereinigt und das Produkt lyophilisiert.
   Ausbeute: 0,21 g Lyophilisat, HPLC: 36,33 % B
   MS: berechnet 787,43 (monoisotopic), gefunden 788,5 [M+H]⁺

### Ausführungsbeispiel 5:

Bestimmung der Hemmkonstanten (Kᵢ-Werte in µM)

Die Bestimmung der Hemmwirkung für die einzelnen Enzyme wurde analog einer bereits früher beschriebenen Methode durchgeführt (Stürzebecher et al., J. Med. Chem. 40, 3091-3099, 1997).

Speziell für die Bestimmung der Hemmung von PK wurden 200 µl Tris-Puffer (0,05 M, 0,154 M NaCI, 5 % Ethanol, pH 8,0; enthält den Inhibitor), 50 µl Substrat (Bzl-Pro-Phe-Arg-pNA in H₂O) und 25 µl PK bei 25 °C inkubiert. Nach 3 min wurde die Reaktion durch Zugabe von 25 µl Essigsäure (50%) unterbrochen und die Absorption bei 405 nm mittels Microplate Reader (Labsystems iEMS Reader MF) bestimmt. Die Kᵢ-Werte wurden nach Dixon (Biochem. J. 55, 170-171, 1953) durch lineare Regression mittels eines Computerprogramms ermittelt. Die Kᵢ-Werte sind das Mittel aus mindestens drei Bestimmungen.

Die Bestimmung der Hemmung von Faktor XIa und Faktor XIIa erfolgte nach analoger Art und Weise. Im Falle der Bestimmung der Hemmkonstanten für humanen Faktor XIa (Haemochrom Diagnostica GmbH, Essen, Deutschland) wurde H-D-Lys(Z)-Pro-Arg-pNA (Chromozym PCa, Roche Diagnostics GmbH, Mannheim, Deutschland) als Substrat verwendet.

Für die Messung der Inhibierungskonstanten des humanen Faktors XIIa (Haemochrom Diagnostica GmbH, Essen, Deutschland) wurde H-D-HHT-Gly-Arg-pNA (Chromozym XII, Roche Diagnostics GmbH, Mannheim, Deutschland) als Substrat eingesetzt.

**Tabelle 1: Hemmung von PK, Faktor XIIa, Faktor XIa und Thrombin durch Verbindungen vom Typ (R)-Benzylsulfonyl-D-Ser-Aaa-4-Amba**

| | | | Kᵢ, µM | | | |
|---|---|---|---|---|---|---|
| Nr. | Aaa | R | PK | F XIIa | F XIa | Thrombin |
| 1 | Gly | H | 1,7 | 16 | 2,2 | 13 |
| 2 | Ala | H | 0,070 | 9,2 | 0,11 | 0,11 |
| 3 | Pro | H | 0,054 | 5,1 | 0,10 | 0,012 |
| 4 | Asp | H | 3,7 | > 1000 | n. b. | > 1000 |
| 5 | Glu | H | 1,1 | > 1000 | n. b. | 38 |
| 6 | Gln | H | 0,047 | 25 | 0,13 | 0,49 |
| 7 | hGlu | H | 20 | > 1000 | 11 | >1000 |
| 8 | Dap | H | 0,050 | 15 | 0,39 | 0,65 |
| 9 | Dap(Z) | H | 0,042 | 13 | 0,28 | 6,9 |
| 10 | Lys | H | 0,016 | 21 | 0,89 | 4,3 |
| 11 | Lys(Z) | H | 0,0035 | 15 | 0,3 | 0,18 |
| 12 | Arg | H | 0,079 | 16 | 0,77 | 4,7 |
| 13 | Thr | H | 0,24 | 51 | 0,25 | 4,0 |
| 14 | Thr(Bzl) | H | 0,091 | 23 | 0,33 | 0,30 |
| 15 | Ser | H | 0,16 | 80 | 0,30 | 14 |
| 16 | Ser(Bzl) | H | 0,025 | 9,8 | 0,30 | 0,48 |
| 17 | hSer | H | 0,020 | > 1000 | n. b. | 8,5 |
| 18 | Phe | H | 0,021 | 0,97 | 0,92 | 1,6 |
| 19 | hPhe | H | 0,048 | 2,8 | 0,084 | 1,2 |
| 20 | Gly | 4-COOH | 0,70 | > 1000 | 0,60 | 170 |
| 21 | Gly | 4-COOMe | 4,2 | 42 | 8,1 | 9,4 |
| 22 | Ala | 4-COOH | 0,016 | 17 | 0,015 | 2,3 |
| 23 | Ser | 4-COOH | 0,029 | > 1000 | 0,17 | 120 |
| 24 | Ser | 4-COOMe | 0,16 | 19 | 0,87 | 4,2 |
| 25 | Gly | 4-AMe | 6,3 | 17 | 6,0 | 8,0 |

**Tabelle 2: Hemmung von PK, Faktor XIIa, Faktor XIa und Thrombin durch Verbindungen vom Typ (R)-Benzylsulfonyl-D-Ser(tBu)-Aaa-4-Amba**

| | | | Kᵢ, µM | | | |
|---|---|---|---|---|---|---|
| Nr. | Aaa | R | PK | F XIIa | F XIa | Thrombin |
| 26 | Gly | H | 0,34 | 2,6 | 1,4 | 0,22 |
| 27 | Ala | H | 0,061 | 2,0 | 0,030 | 0,0021 |
| 28 | Pro | H | 0,0065 | 0,49 | 0,036 | 0,0020 |
| 29 | Asp | H | 0,91 | > 1000 | 0,39 | 6,0 |
| 30 | Glu | H | 0,36 | 19 | 0,079 | 2,6 |
| 31 | Gln | H | 0,0092 | 6,3 | 0,067 | 0,021 |
| 32 | hGlu | H | 8,0 | > 1000 | 8,2 | > 1000 |
| 33 | Dap | H | 0,022 | 4,0 | 0,19 | 0,0094 |
| 34 | Dap(Z) | H | 0,025 | 0,93 | 0,31 | 0,37 |
| 35 | Lys | H | 0,0036 | 4,4 | 0,51 | 0,055 |
| 36 | Lys(Z) | H | 0,0094 | 5,4 | 0,48 | 0,024 |
| 37 | Arg | H | 0,040 | 2,6 | 0,34 | 0,065 |
| 38 | Thr | H | 0,032 | 14 | n. b. | 0,044 |
| 39 | Thr(Bzl) | H | 0,044 | 17 | 0,40 | 0,019 |
| 40 | Ser | H | 0,052 | 6,0 | 0,20 | 0,047 |
| 41 | Ser(Bzl) | H | 0,012 | 1,4 | 0,20 | 0,012 |
| 42 | hSer | H | 0,21 | > 1000 | 0,74 | 13 |
| 43 | hSer(Bzl) | H | 0,0082 | 80 | 0,61 | 0,50 |
| 44 | Phe | H | 0,0055 | 4,6 | 0,26 | 0,16 |
| 45 | hPhe | H | 0,0045 | 1,3 | 0,083 | 0,048 |
| 46 | Gly | 4-COOH | 0,029 | 7,5 | n. b. | 2,2 |
| 47 | Gly | 4-COOMe | 1,1 | 4,8 | 1,6 | 0,36 |
| 48 | Ala | 4-COOH | 0,0062 | 9,5 | 0,0069 | 0,044 |
| 49 | Ala | 4-COOMe | 0,054 | 4,7 | 0,079 | 0,0043 |
| 50 | Gly | 4-AMe | 4,0 | 1,8 | 2,9 | 0,12 |
| 51 | Pro | 4-CN | 0,0094 | 1,6 | 0,0091 | 0,000064 |

**Tabelle 3: Hemmung von PK, Faktor XIIa, Faktor XIa und Thrombin durch Verbindungen vom Typ (R)-Benzylsulfonyl-D-Cha-Aaa-4-Amba**

| | | | Kᵢ, µM | | | |
|---|---|---|---|---|---|---|
| Nr. | R | Aaa | PK | F XIIa | F XIa | Thrombin |
| 52 | 3-CN | Pro | 0,086 | 13 | n. b. | < 0,0010 |
| 53 | H | Lys | 0,0023 | 0,83 | 0,15 | 0,010 |
| 54 | H | Lys(Z) | 0,020 | 4,0 | 0,34 | 0,015 |
| 55 | 3-AMe | Pro | 0,090 | 0,47 | 0,17 | 0,0032 |
| 56 | 3-(Glut-NHCH₂) | Pro | 0,044 | 5,6 | 0,052 | < 0,0010 |
| 57 | H | Glu | 0,030 | 4,0 | 0,020 | 0,081 |

Hemmkonstanten für PEG-gekoppelte Verbindungen in µM:

Inhibitor Nr. 58: 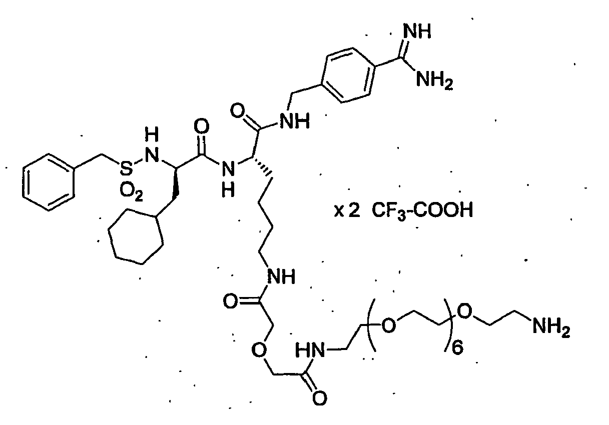 PK 0,059 ; F XIIa 2,0, F XIa 0,23, Thrombin 0,0080 Inhibitor Nr. 59: 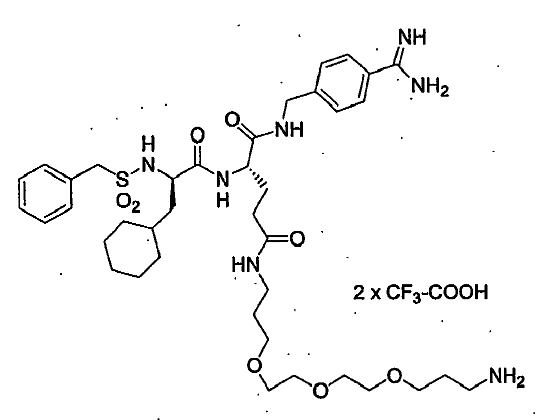 PK 0,015 ; F XIIa 0,98, F XIa 0,040, Thrombin 0,015

**Tabelle 4: Hemmung von PK, Faktor XIIa, Faktor XIa und Thrombin durch Verbindungen vom Typ (4-R)-Benzylsulfonyl-P3-Aaa-4-Amba ((4-R) bezeichnet die 4-Position des Restes R in Tabelle 4 am Phenylring des Benzylsulfonylrestes)**

| | | | | Kᵢ, µM | | | |
|---|---|---|---|---|---|---|---|
| Nr. | R | P3 | Aaa | PK | F XIIa | F XIa | Thrombin |
| 60 | H | D-hAla(4- Pyr) | Glu(OBzl) | 0,0055 | 0,094 | 0,031 | 0,17 |
| 61 | COOH | D-Ser | Pro | 0,0091 | 29 | 0,014 | 0,24 |
| 62 | H | D-Ser(tBu) | Lys(Tfa) | 0,011 | 6,1 | n. b. | 0,0029 |
| 63 | H | D-Cha | Gly | 0,011 | 0,70 | 25 | 0,0090 |
| 64 | H | D-Ser(tBu) | His | 0,014 | 61 | n. b. | 0,12 |
| 65 | COOH | D-Ser(tBu) | Ser | 0,015 | 17 | 0,030 | 2,0 |
| 66 | CH₂COOH | D-Ser(tBu) | Pro | 0,016 | 4 | n. b. | 0,0018 |
| 67 | H | D-hPhe | Ser | 0,019 | 0,63 | 3,0 | 0,55 |
| 68 | H | D-Ser(tBu) | Can | 0,019 | 6,8 | n. b. | 0,038 |
| 69 | H | D-hAla(4- Pyr) | Ser | 0,020 | 1,6 | n. b. | 0,91 |
| 70 | COOH | D-Ser(tBu) | Pro | 0,025 | 2,4 | n. b. | 0,0023 |
| 71 | H | D-Cha | Lys(Suc) | 0,029 | 11 | n. b. | 0,0021 |
| 72 | H | D-hTyr | Glu | 0,22 | 0,36 | 0,028 | 19 |
| 73 | H | D-hTyr | Ser | 0,13 | 0,28 | 0,078 | 1,4 |
| 74 | NO₂ | D-hPhe | Gly | 0,051 | 0,39 | 0,093 | 0,71 |
| 75 | H | D-hTyr | Gly | 0,12 | 0,78 | 0,61 | 1,5 |
| 76 | H | D-hPhe | Gly | 0,39 | 0,15 | 0,27 | 0,047 |
| 77 | H | D-Phe(3-Amidino) | Gly | 0,082 | 0,19 | 0,25 | 0,085 |
| 78 | NH₂ | D-hPhe | Gly | 0,045 | 0,26 | 0,12 | 0,26 |
| 79 | H | D-Phe(3-GuMe) | Gly | 0,075 | 0,31 | 0,22 | 0,059 |
| 80 | H | D-Norarginin | Gly | 0,068 | 0,34 | 0,49 | 2,1 |
| 81 | H | D-Arg | Gly | 0,074 | 0,35 | 0,70 | 1,4 |
| 82 | H | D-Cha | Gly | 0,10 | 1,4 | 0,33 | 0,023 |
| 83 | H | D-Indanylgly-cin | Gly | 0,075 | 0,37 | n. b. | 0,14 |
| 84 | COOCH₃ | D-Phe(3-Amidino) | Gly | 0,14 | 0,38 | 0,70 | 0,53 |
| 85 | H | D/L-hAla(4-Pyr) | Gly | 0,13 | 0,40 | 1,1 | 2,0 |
| 86 | H | D-Ser | Lys(Glut) | 0,39 | n. b. | n. b. | 2,8 |

Inhibitor 87:

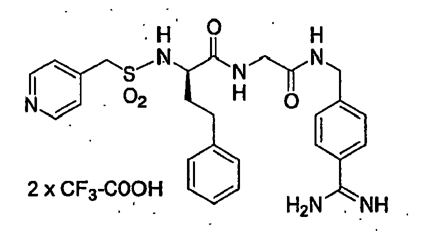

Kᵢ-Werte in µM: PK 0,42; F XIIa 0,16; F XIa 0,33, Thrombin 3,6 Inhibitor 88 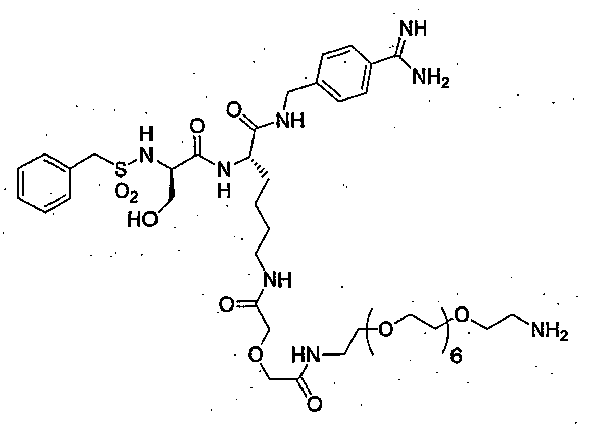 Kᵢ-Werte in µM: PK 0,22; F XIIa 21; F XIa 0,4, Thrombin 1,2
Inhibitor 89 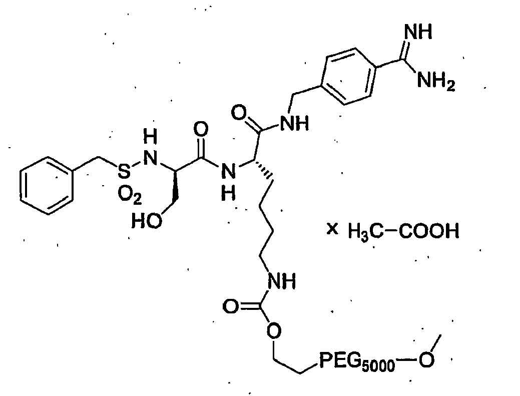 Kᵢ-Werte in µM: PK 0,19; F XIIa 79; Thrombin 1,72. PEG₅₀₀₀ bezeichnet ein Polyethylenglykol mit einem mittleren Molekulargewicht von 5000 Dalton.

### Ausführungsbeispiel 6:

### Verhinderung der Aktivierung von Prothrombin in Hirudin-antikoaguliertem Plasma

Venenblut von gesunden freiwilligen Spendern wurde unmittelbar nach der Entnahme mit Hirudinlösung (2000 ATE/ml 0,9 %iger NaCI-Lösung) im Verhältnis 10:1 gemischt und 10 min bei 250xg zentrifugiert. 950 µl Plasma wurden mit 20 µl Inhibitor-Lösung (5 bzw 0,5 mM) gemischt und für 5 h in Polypropylen-Röhrchen bei 37 °C inkubiert. Zur Verstärkung der Aktivierung an der künstlichen Oberfläche wurden 30 µl Kaolin (PTT-Reagenz, 1:1000 verdünnt; Roche Diagnostics, Penzberg, D) zugegeben.

Zur Bestimmung des Prothrombinfragments F 1+2 wurde ein Enzymimmunoassay (Enzygnost-F 1+2, DadeBehring GmbH, Marburg, Deutschland) nach dem Sandwich-Prinzip verwendet. Prothrombinfragment bindet an fixierte Antikörper gegen F 1+2. In einem zweiten Schritt binden Peroxidase-konjugierte Antikörper gegen Prothrombin und die gebundene Enzymaktivität wurde chromogen bestimmt. Die Konzentration an Prothrombinfragment F 1+2 wurde aus einer Eichkurve ermittelt.

**Tabelle 5: Einfluss verschiedener Verbindungen auf die Aktivierung von Prothrombin in Hirudin-antikoaguliertem Plasma in Gefäßen aus Polypropylen unter Zugabe von Kaolin. Die Menge des nachgewiesenen Prothrombinfragments F 1+2 (in nM) nach 5 h in Gegenwart von Kaolin wurde als 100 % gesetzt.**

| | Prothrombinfragment F 1+2 (%) | | | | |
|---|---|---|---|---|---|
| Inhibitor Nr. | + Kaolin - | Kaolin | Kaolin+Inhibitor 100 µM | Kaolin+Inhibitor 10 µM | Kaolin+Inhibitor 1 µM |
| 45 | 100 | 0,64 | 0,11 | 0,59 | n. b. |
| 11 | 100 | 0,49 | 0,15 | 110,9 | n. b. |
| 53 | 100 | 0,46 | 0,08 | 0.09 | 0,59 |
| 59 | 100 | 0.46 | 0,03 | 0,20 | 59,3 |
| 75 | 100 | 0,14 | n. b. | 0,01 | 0,07 |
| 73 | 100 | 0,14 | n. b. | 0,04 | 0,07 |

### Ausführungsbeispiel 7:

### Einsatz eines PK-Hemmstoffes zur Affinitätschromatographie als Modell für die Modifizierung einer künstlichen Oberfläche

Durch Kopplung des Inhibitors Benzylsulfonyl-D-Ser-Lys-4-Amba an CH-Sepharose 4B (Pharmacia) wurde das Material für eine Affinitätschromatographie hergestellt. Dazu wurden zunächst 16 g gequollene CH-Sepharose 4B in 65 ml MES-Puffer (0,1 M pH 4,75) suspendiert, anschließend wurde der Inhibitor (50 mg in 2 ml Puffer) zugegeben. Die Mischung wurde mit 2,837 g N-Cyclohexyl-N'-(2-Morpholinoethyl)-carboddiimid Metho-p-Toluolsulfonat (Acros Organics) versetzt (entspricht 0,1 M im Ansatz) und für 24 h bei Raumtemperatur inkubiert. Anschließend wurde mit MES-Puffer und Wasser gewaschen und mit Tris-Puffer (0,05 M, enthält 0,75 M NaCl, pH 7,5) äquilibriert. Nach dem Packen und Äquilibrieren der Säule (1,4 x 19 cm) wurden 100 µg PK (Haemochrom Diagnostics, Essen, Germany) in 1 ml Puffer aufgetragen. Dann wurde die Säule zuerst mit Tris-Puffer und anschließend mit 3 M NaCl-Lösung gewaschen, dabei wurde kein PK eluiert. Durch einen anschließenden Benzamidin-Gradienten (0,1 - 2,5 M) wurde 41 % aktives PK eluiert.

Ein vergleichbares Resultat kann bei Verwendung einer Affinitätschromatographie-Säule erhalten werden, bei der der nachfolgend abgebildete Inhibitor kovalent gekoppelt wird. 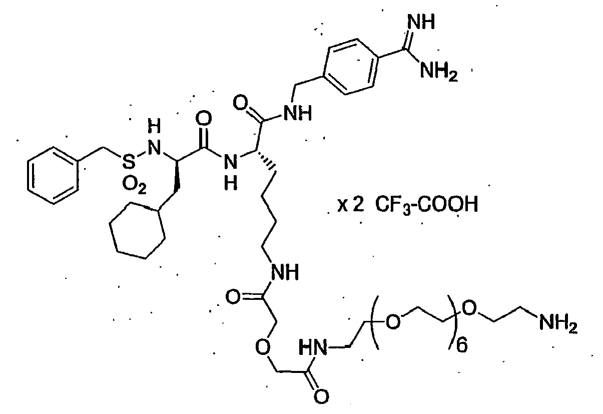 Die folgenden Seiten 49 bis 74 beinhalten spezielle Ausführungsbeispiele.
1. Verwendung von acyliertem 4-Amidino- oder 4-Guanidinobenzylamin gemäß der allgemeinen Formel I 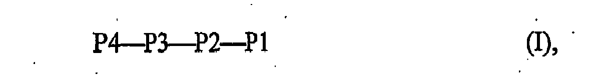 wobei P4 eine einfach oder mehrfach substituierte oder unsubstituierte Benzylsulfonylgruppe ist, P3 eine einfach oder mehrfach substituierte oder unsubstituierte, natürliche oder unnatürliche α-Amino- oder α-Iminosäure in der D-Konfiguration ist, P2 eine einfach oder mehrfach substituierte oder unsubstituierte, natürliche oder unnatürliche α-Amino- oder α-Iminosäure in der L-Konfiguration ist, und P1 eine einfach oder mehrfach substituierte oder unsubstituierte 4-Amidino- oder 4-Guanidinobenzylamingruppe ist, zur Inhibierung von Plasmakallikrein und / oder Faktor XIa und / oder Faktor XIIa.
2. Verwendung nach 1 zur Inhibierung von Plasmakallikrein.
3. Verwendung nach 1 oder 2, **dadurch gekennzeichnet, dass** der Substituent am substituierten P4, P3, P2 und/oder P1
   (a) Wasserstoff ist und/oder
   (b) ein Halogen, vorzugsweise Fluor, Chlor und/oder Brom ist, und/oder
   (c) ein substituierter oder nicht-substituierter, verzweigter oder linearer Alkylrest mit 1-6 C-Atomen, vorzugsweise 1-3 C-Atomen, insbesondere Methyl, oder ein substituierter oder nicht-substituierter, verzweigter oder linearer Aralkylrest mit 1-10 C-Atomen ist, wobei der Substituent des substituierten, verzweigten oder linearen Alkylrests oder Aralkylrestes vorzugsweise eine Halogen-, Hydroxy-, Amino-, Cyano-, Amidino-, Guanidino- und/oder eine Carboxylgruppe, gegebenenfalls verestert mit einem Niederalkylrest, insbesondere mit Methyl oder Ethyl ist, und/oder
   (d) eine Hydroxy-, Amino-, Cyano-, Amidino-, Guanidino-, Methyloxycarbonyl-, Benzyl-, Benzyloxycarbonyl-, Aminomethyl- oder Glutaryl- oder Succinyl-Amidomethylgruppe ist und/oder eine Oxyalkylcarbonyl-, Carboxyl-, Carboxymethyl- oder Carboxyethylgruppe ist gegebenenfalls verestert mit einem Niederalkylrest, insbesondere mit Methyl oder Ethyl oder eine Oxyalkylcarbonyl-, Carboxyl-, Carboxymethyl- oder Carboxyethylgruppe, die als unsubstituiertes oder mit einer Alkyl- oder Arylgruppe substituiertes Amid vorliegt.
4. Verwendung nach mindestens einem der 1 bis 3, **dadurch gekennzeichnet, dass** zusätzlich eine Linkergruppe an P4 oder P2 gekoppelt ist, wobei die Linkergruppe über einen wie in 3 definierten Substituenten an P4 oder direkt an eine funktionelle Gruppe von P2, insbesondere über eine -NH- oder eine -CO-Gruppe gekoppelt ist, wobei die Linkergruppe vorzugsweise eine Dicarbonsäure, eine Aminocarbonsäure, ein Diamin, eine Disulfonsäure, oder eine Aminosulfonsäure mit einem Alkyl-, Aryl- oder Aralkylgrundgerüst ist, wobei das Alkylgrundgerüst 1 bis12 C-Atome, insbesondere 2-6 C-Atome aufweist, das Arylgrundgerüst 6-10 C-Atome, insbesondere Phenyl aufweist, das Aralkylgrundgerüst 6-12 C-Atome, insbesondere Benzyl aufweist, oder eine Aminoalkyl- oder Carboxyalkylgruppe mit 2-12 C-Atomen, insbesondere 2-6 C-Atomen; oder wobei die Linkergruppe an P4 oder P2 eine Oligo- oder Polyalkylenglycolkette ist, insbesondere eine Poly- oder Oligoethylen- oder Poly- oder Oligopropylenglycolkette ist, wobei das Oligo- oder Polyalkylenglycol zumindest an beiden Enden eine funktionelle Gruppe, insbesondere eine substituierte oder unsubstituierte Amino-, Carboxyl- und/oder Mercaptogruppe aufweist oder wobei das Oligo- oder Polyalkylenglycol zumindest an einem Ende eine funktionelle Gruppe, insbesondere eine substituierte oder unsubstituierte Amino-, Carboxyl- und/oder Mercaptogruppe aufweist und am anderen Ende mit einer Alkylgruppe mit 1-4 C-Atomen, insbesondere Methyl modifiziert ist,
   wobei bei Kopplung der Linkergruppe an P4 über einen Substituenten der Substituent vorzugsweise eine -NH-Gruppe, -NH-Alkyl-Gruppe mit 1 bis 6 C-Atomen, insbesondere Methyl, eine -CO-Gruppe, eine -CO-Alkyl-Gruppe mit 2-6 C-Atomen, insbesondere -CO-Methyl, eine -CO-O-Alkyl-Gruppe mit 1-6 C-Atomen, insbesondere Methyl, eine -S-Gruppe, eine -S-Alkyl-Gruppe mit 1 bis 6 C-Atomen, insbesondere Methyl, eine -O-Alkyl-Gruppe mit 1-6 C-Atomen, insbesondere Methyl, eine -SO₂-Gruppe oder eine -SO₂-Alkyl-Gruppe mit 1-6 C-Atomen, insbesondere Methyl.ist oder wobei, bei Kopplung der Linkergruppe an P2, P2 vorzugsweise
   (a) Lysin oder dessen Homologe mit 1-5 C-Atomen in der Seitenkette, insbesondere Ornithin, Homolysin, α-γ-Diaminobuttersäure, α-β-Diaminopropionsäure, α-Diaminoglycin oder
   (b) Glutaminsäure oder dessen Homologe mit 1-5 C-Atomen in der Seitenkette, insbesondere Asparaginsäure, Glutaminsäure oder Homoglutaminsäure oder
   (c) Cystein oder Homocystein oder
   (d) Serin oder Threonin ist.
5. Verwendung nach 4, **dadurch gekennzeichnet, dass** die Linkergruppe mit dem Substituenten zur Kopplung an P4 die allgemeine Formel II aufweist 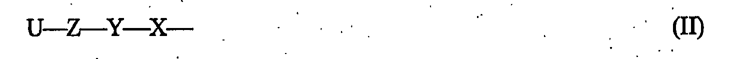 wobei
   U gleich eine H₂N-, HOOC-(CH₂)ₙ-CO-NH-, HOOC-, H₂N-(CH₂)ₙ-NH-CO- oder HS-Gruppe ist mit Z gleich -(CH₂)ₙ- mit n = 1 bis 10, insbesondere 1-5, oder Z gleich ein Oligo- oder Polyalkylenglycol der allgemeinen Formel -(CH₂)_{d}-[O-CH₂-CH₂]ᵥO-(CH₂)ₘ-(NH-CO-CH₂-O-CH₂)ₖ- oder -(CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 50, insbesondere 2 bis 10, m = 0, 1, 2, 3 oder 4 und k = 0 oder 1 oder
   U gleich eine CH₃-O-Gruppe ist mit Z gleich ein Oligo- oder Polyalkylenglycol der allgemeinen Formel -(CH₂)_{d}-[O-CH₂-CH₂]ᵥO-(CH₂)ₘ-(NH-CO-CH₂-O-CH₂)ₖ- oder -(CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 50, insbesondere 2 bis 10, m = 0, 1, 2, 3 oder 4 und k = 0 oder 1 ist;
   Y gleich eine -CO-NH-, eine -NH-CO-, eine -SO₂-NH-, eine -NH-SO₂-, eine -S-S- oder eine -S-Gruppe ist oder wenn U und Z nicht vorhanden sind gleich eine H₂N-, HOOC-, HS-, HO- oder Halogenalkyl-Gruppe ist;
   X gleich eine -(CH₂)ₙ-Gruppe mit n = 0, 1, 2, 3 oder 4, insbesondere n = 1 oder gleich eine -(CH₂)ₙ-O-Gruppe mit Bindung an den Benzylrest über den Sauerstoff und n = 1, 2, 3 oder 4 ist;
   und die Kopplung der Linkergruppe an den Phenylring des Benzylrestes von X falls vorhanden oder von Y, wenn X nicht vorhanden ist, ausgeht.
6. Verwendung nach einem der 4 oder 5, **dadurch gekennzeichnet, dass**, wenn die Linkergruppe an P4 gekoppelt ist, P2 Glycin, Alanin, Serin, Prolin, Homoprolin oder Azetidincarbonsäure ist.
7. Verwendung nach 4, **dadurch gekennzeichnet, dass** die Linkergruppe an P2 gekoppelt wird, wobei P2 die allgemeine Formel III aufweist 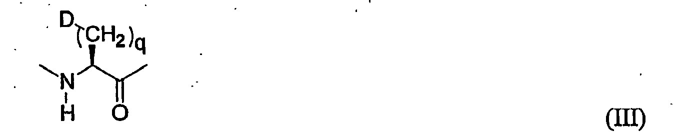 wobei q = 0, 1, 2, 3, 4 oder 5 ist und D gleich Formel IV ist 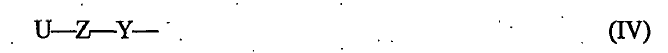 wobei U, Z und Y die gleiche Bedeutung wie bei Formel II gemäß 5 besitzen.
8. Verwendung nach mindestens einem der 1 bis 7, **dadurch gekennzeichnet, dass** das acylierte Amidino- oder Guanidinobenzylamin die allgemeine Formel V oder VI aufweist 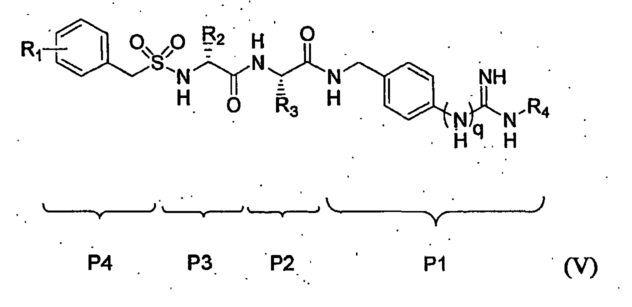 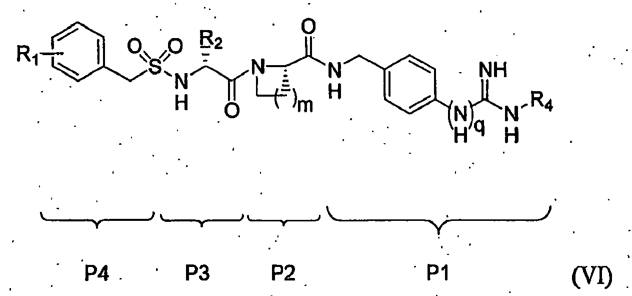 mit m = 1 bis 3 und q = 0 oder 1, insbesondere 0,
   wobei R₁, R₂, R₃ und/oder R₄
   (a) Wasserstoff ist und/oder
   (b) ein Halogen, vorzugsweise Fluor, Chlor und/oder Brom ist, und/oder
   (c) ein substituierter oder nicht-substituierter, verzweigter oder linearer Alkylrest mit 1-6 C-Atomen, vorzugsweise 1-3 C-Atomen, insbesondere Methyl ist, wobei der Substituent des substituierten, verzweigten oder linearen Alkylrests vorzugsweise eine Halogen-, Hydroxy-, Amino-, Cyano-, Amidino-, Guanidino- und/oder eine Carboxylgruppe, gegebenenfalls verestert mit einem Niederalkylrest, insbesondere mit Methyl oder Ethyl ist und/oder
   (d) eine Hydroxy-, Amino-, Cyano-, Amidino-, Guanidino-, Methyloxycarbonyl-, Benzyl-, Benzyloxycarbonyl-, Aminomethyl- oder Glutaryl- oder Succinyl-Amidomethylgruppe ist und/oder eine Oxyalkylcarbonyl-, Carboxyl-, Carboxymethyl- oder Carboxyethylgruppe ist gegebenenfalls verestert mit einem Niederalkylrest, insbesondere mit Methyl oder Ethyl oder eine Oxyalkylcarbonyl-, Carboxyl-, Carboxymethyl- oder Carboxyethylgruppe, die als unsubstituiertes oder mit einer Alkyl- oder Arylgruppe substituiertes Amid vorliegt und/oder
      R₁ und/oder R₃ eine Linkergruppe sein kann, wobei die Linkergruppe über einen Substituenten wie in 3 definiert an P4 oder direkt an eine funktionelle Gruppe von P2, insbesondere über eine -NH- oder eine -CO-Gruppe gekoppelt ist, wobei die Linkergruppe vorzugsweise eine Dicarbonsäure, eine Aminocarbonsäure, ein Diamin, eine Disulfonsäure, oder eine Aminosulfonsäure mit einem Alkyl-, Aryl- oder Aralkylgrundgerüst ist, wobei das Alkylgrundgerüst 1 bis 12 C-Atome, insbesondere 2-6 C-Atome aufweist, das Arylgrundgerüst 6-10 C-Atome, insbesondere Phenyl aufweist, das Aralkylgrundgerüst 6-12 C-Atome, insbesondere Benzyl aufweist, oder eine Aminoalkyl- oder Carboxyalkylgruppe mit 2-12 C-Atomen, insbesondere 2-6 C-Atomen; oder wobei die Linkergruppe an P4 oder P2 eine Oligo- oder Polyalkylenglycolkette ist, insbesondere eine Poly- oder Oligoethylen- oder Poly- oder Oligopropylenglycolkette ist, wobei das Oligo- oder Polyalkylenglycol zumindest an beiden Enden eine funktionelle Gruppe, insbesondere eine substituierte oder unsubstituierte Amino-, Carboxyl- und/oder Mercaptogruppe aufweist oder wobei das Oligo- oder Polyalkylenglycol zumindest an einem Ende eine funktionelle Gruppe, insbesondere eine substituierte oder unsubstituierte Amino-, Carboxyl- und/oder Mercaptogruppe aufweist und am anderen Ende mit einer Alkylgruppe mit 1-4 C-Atomen, insbesondere Methyl modifiziert ist und/oder
      R₁ die Formel (II) wie in 5 definiert und P2 mit R₃ die Formeln (III) und (IV), wie in 7 definiert, aufweist, und
      R₄ besonders bevorzugt Hydroxy, Amino und Alkoxycarbonyl ist.
9. Verwendung nach mindestens einem der 1 bis 6 oder 8, **dadurch gekennzeichnet, dass** eine Verbindung gemäß der allgemeinen Formel I mit einer Linkergruppe an P4 gemäß der allgemeinen Formel II, wie in 5 definiert, eine der folgenden Strukturen aufweist: 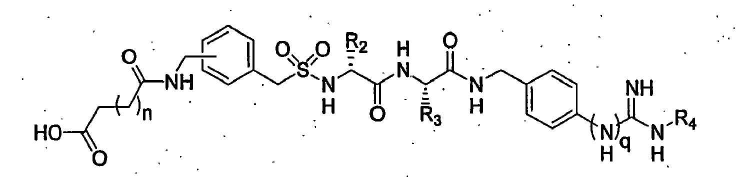 oder 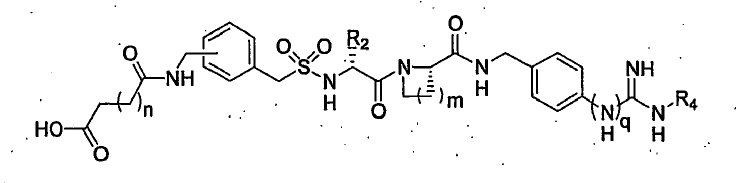 oder 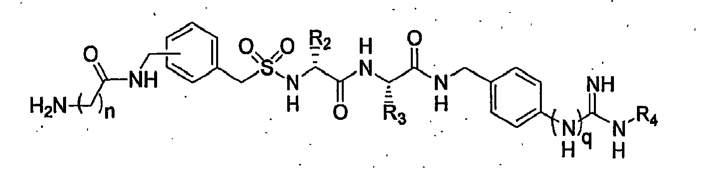 oder 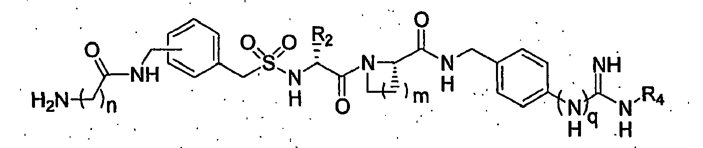 mit n = 1 bis 10, m = 1 bis 3 und q = 0 oder 1, insbesondere 0, wobei R₂ R₃ und R₄ die in 8 genannten Bedeutungen besitzen.
10. Verwendung nach mindestens einem der 1 bis 6 oder 8, **dadurch gekennzeichnet, dass** eine Verbindung gemäß der allgemeinen Formel I mit einer Linkergruppe an P4 gemäß der allgemeinen Formel II, wie in 5 definiert, eine der folgenden Strukturen aufweist: 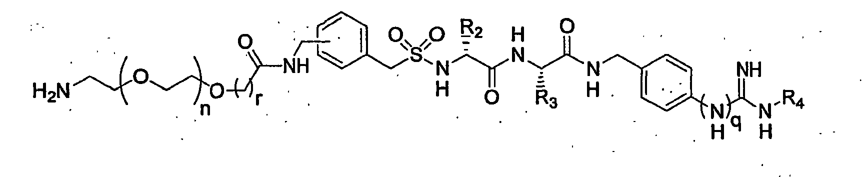 oder 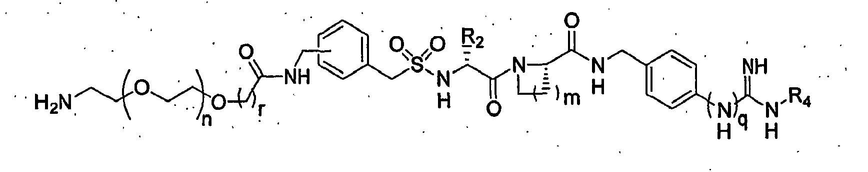 oder 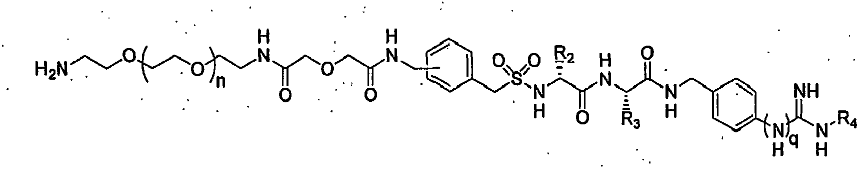 oder 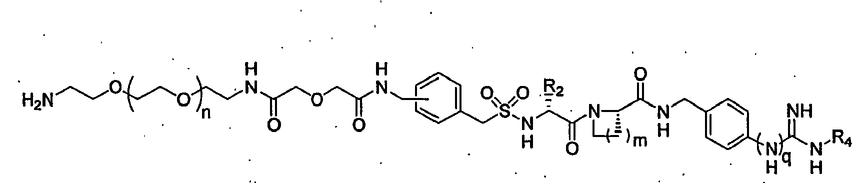 mit n = 1 bis 1000, m = 1 bis 3, r = 0 bis 3 und q = 0 oder 1, insbesondere 0, wobei R₂, R₃ und R₄ die in 8 genannten Bedeutungen besitzen.
11. Verwendung nach mindestens einem der 1 bis 6 oder 8, **dadurch gekennzeichnet, dass** eine Verbindung gemäß der allgemeinen Formel I mit einer Linkergruppe an P4 gemäß der allgemeinen Formel II, wie in 5 definiert, eine der folgenden Strukturen aufweist: 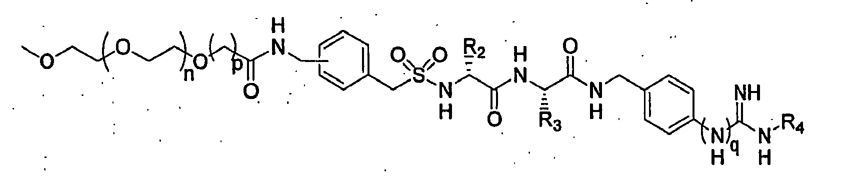 oder 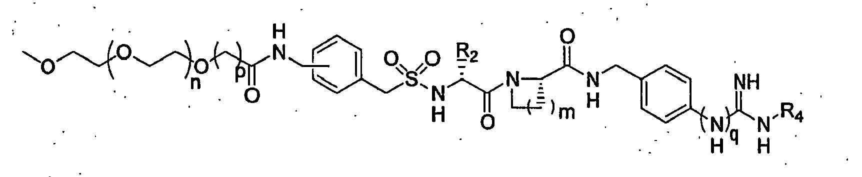 oder 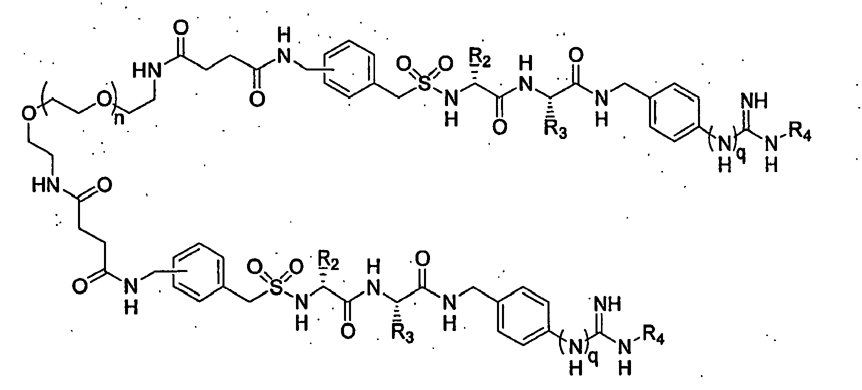 oder 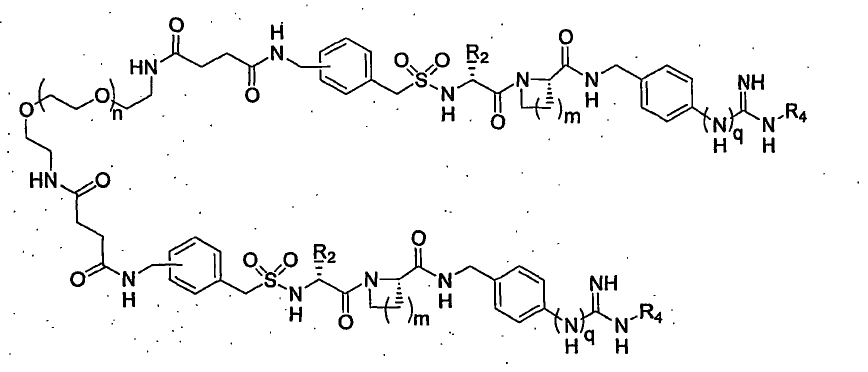 oder 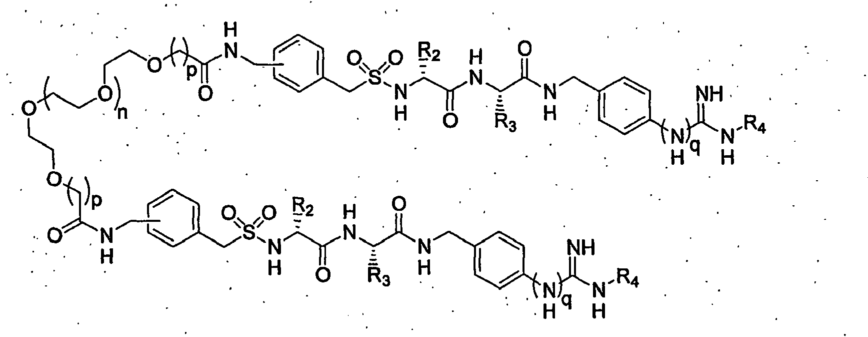 oder 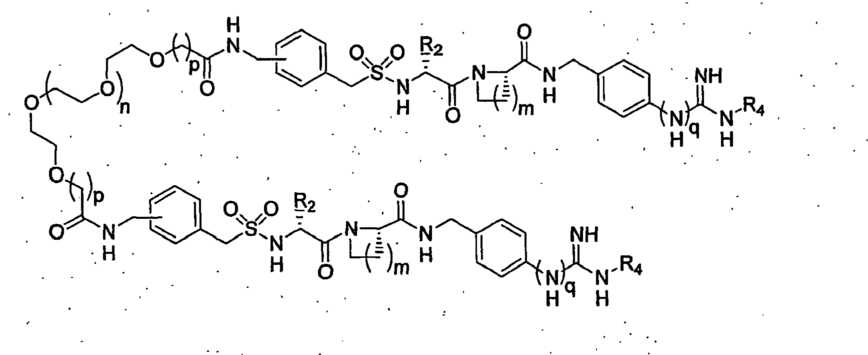 mit p = 0, 1, 2 oder 3, q = 0 oder 1, insbesondere 0, n = 1 bis 1000 und m = 1 bis 3, wobei R₂, R₃ und R₄ die in 8 genannten Bedeutungen besitzen.
12. Verwendung nach mindestens einem der 1, 2, 3, 4, 7 oder 8, **dadurch gekennzeichnet, dass** eine Verbindung gemäß der allgemeinen Formel I mit einer Linkergruppe an P2 gemäß der allgemeinen Formeln III und IV, wie in 7 definiert, eine der folgenden Strukturen aufweist: 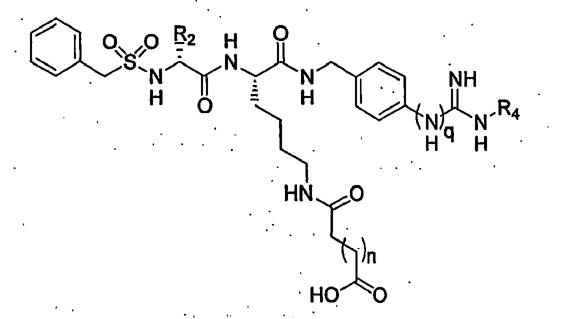 mit n= 0 bis 5, bevorzugt 1 oder 2
   oder 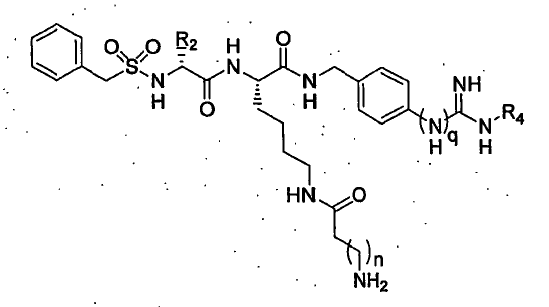 mit n = 0 bis 11,
   oder 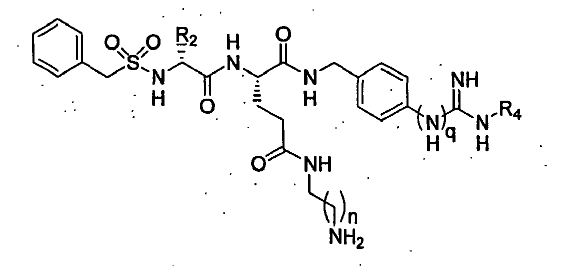 mit n = 1 bis 6
   oder 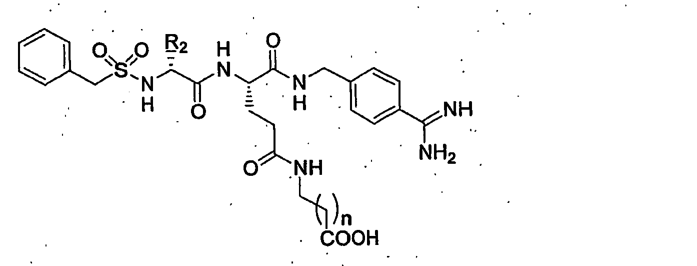 oder 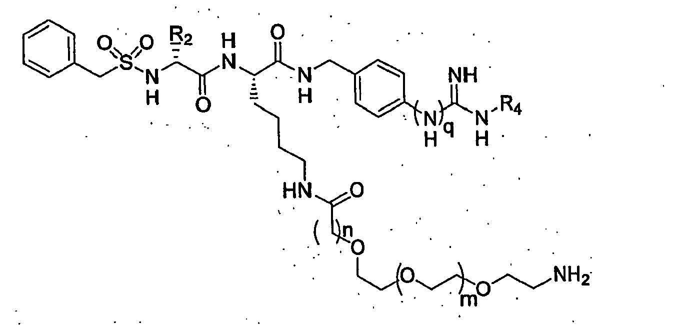 mit n= 0 bis 3 und m = 0 bis 1000
   oder 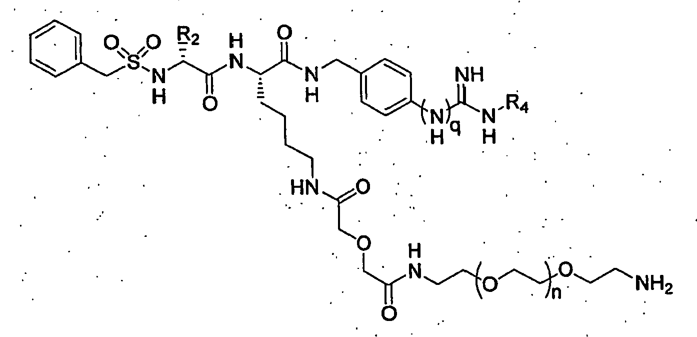 mit n = 1 bis 1000
   oder 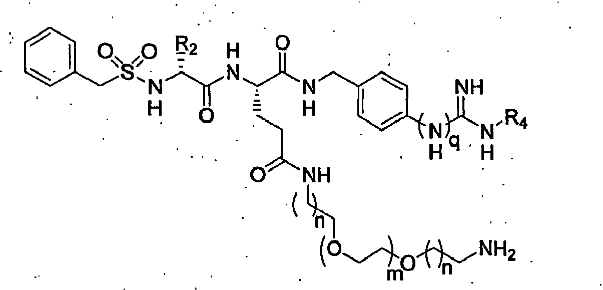 mit n = 1 bis 3 und m=1 bis 1000, wobei q jeweils gleich 0 oder 1, insbesondere 0
   ist und R₂ und R₄ jeweils die in 8 genannten Bedeutungen besitzen.
13. Verwendung nach mindestens einem der 1, 2, 3, 4, 7 oder 8, **dadurch gekennzeichnet, dass** eine Verbindung gemäß der allgemeinen Formel I mit einer Linkergruppe an P2 gemäß den allgemeinen Formeln III und IV, wie in 7 definiert, eine der folgenden Strukturen aufweist: 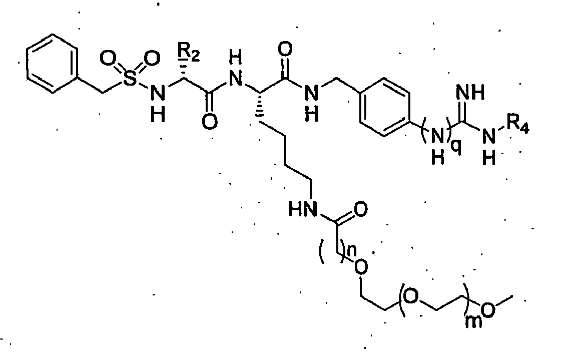 mit n = 0 bis 4 und m = 10 bis 1000
   oder 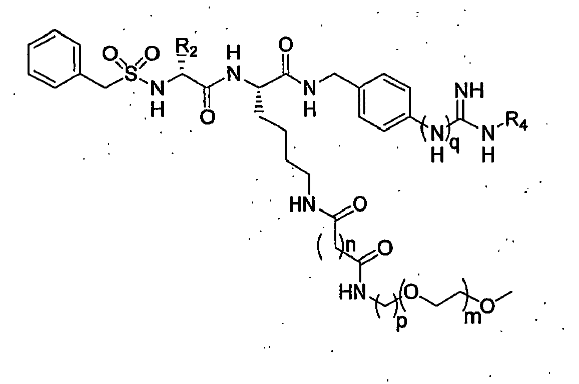 mit n = 1 bis 4, p = 2 bis 4 und m = 1 bis 1000
   oder 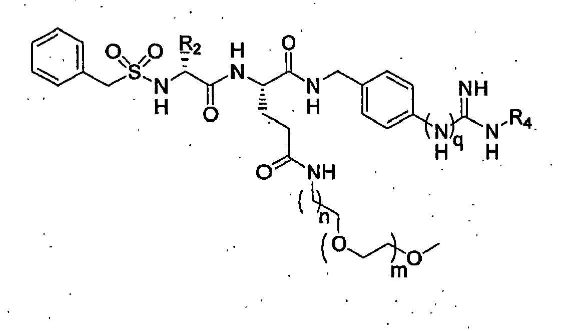 oder 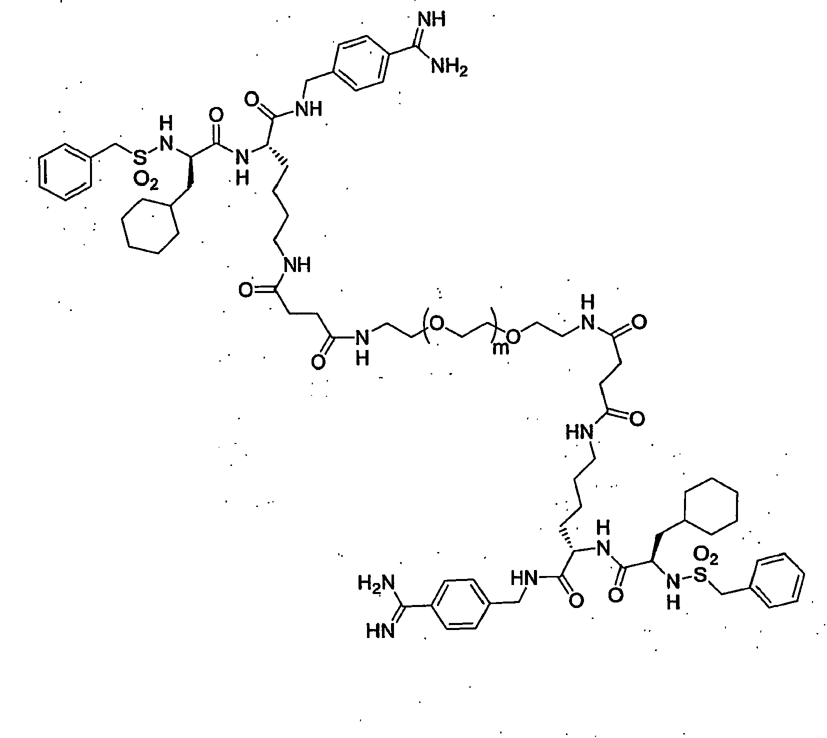 oder 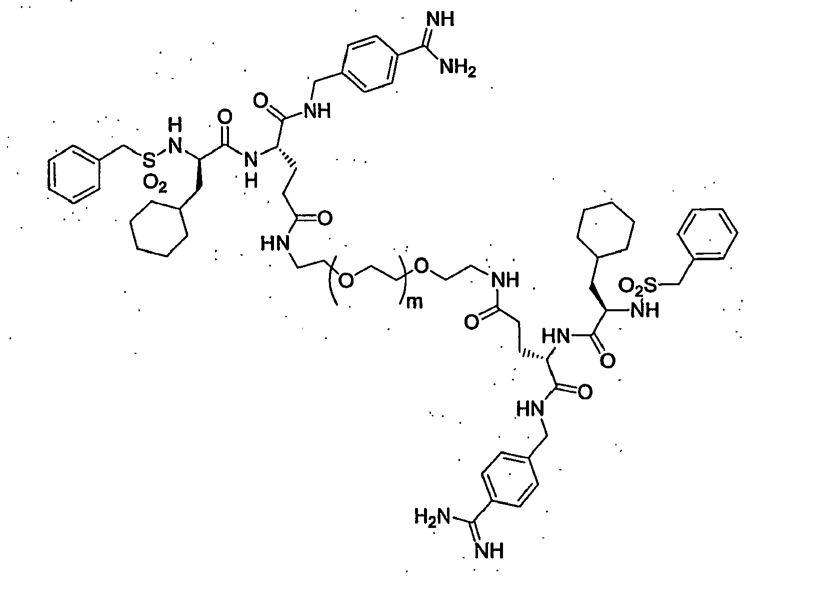 mit n =1 bis 3 und m=10 bis 1000,
   wobei q gleich 0 oder 1, insbesondere 0 ist und R₂ und R₄ jeweils die in 8 genannten Bedeutungen besitzen.
14. Verwendung nach mindestens einem der 1, 2, 3, 4, 7, 8, 12 oder 13, wobei eine Kopplung an eine künstliche Oberfläche über P2 erfolgt, **dadurch gekennzeichnet, dass** der Substituent an P4 insbesondere H, ein Halogen, eine Aminogruppe, eine Hydroxygruppe oder eine lineare oder verzweigte Alkylgruppe mit 1 bis 6 Kohlenstoffatomen ist.
15. Verwendung nach mindestens einem der 4 bis 6 und 8 bis 11, **dadurch gekennzeichnet, dass** eine Verbindung gemäß der allgemeinen Formel I mit einer Linkergruppe an P4 gemäß der allgemeinen Formel II, wie in 5 definiert, die folgende Struktur aufweist 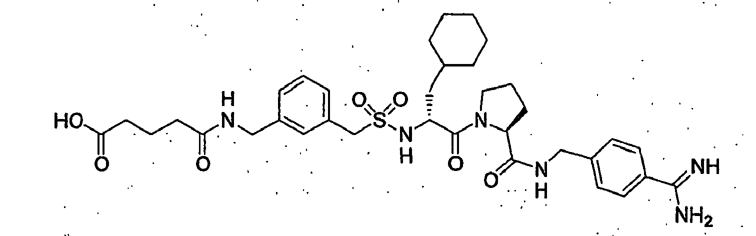 wobei D-Cha in Position P3 insbesondere auch D-Phe oder D-Ser(tBu) und Glutaryl an P4 auch Succinyl sein kann.
16. Verwendung nach mindestens einem der 1, 2, 3, 4, 8, 12 oder 14, **dadurch gekennzeichnet, dass** eine Verbindung gemäß der allgemeinen Formel I eine der folgenden Strukturen aufweist 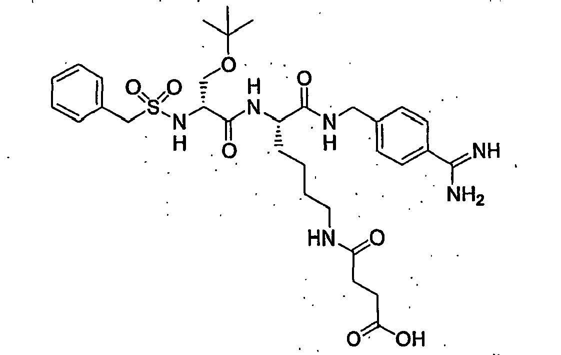 wobei D-Ser(tBu) in Position P3 insbesondere auch D-Cha oder D-Phe und Succinyl an P2 auch Glutaryl sein kann.
17. Verwendung nach mindestens einem der 1, 2, 3, 4, 7, 8, 12 oder 14, dadurch gekennzeichnet, dass eine Verbindung gemäß der allgemeinen Formel I eine der folgenden Strukturen aufweist: 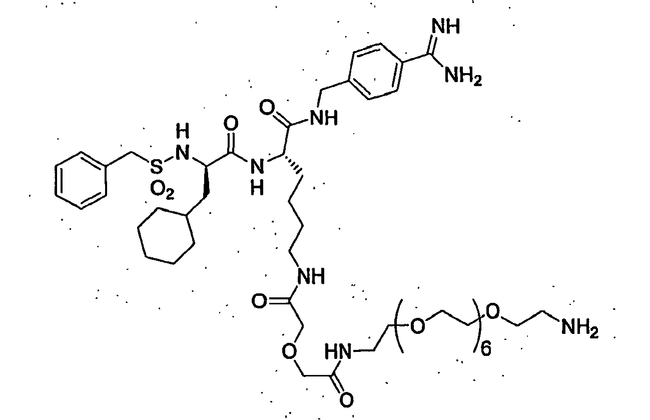 oder 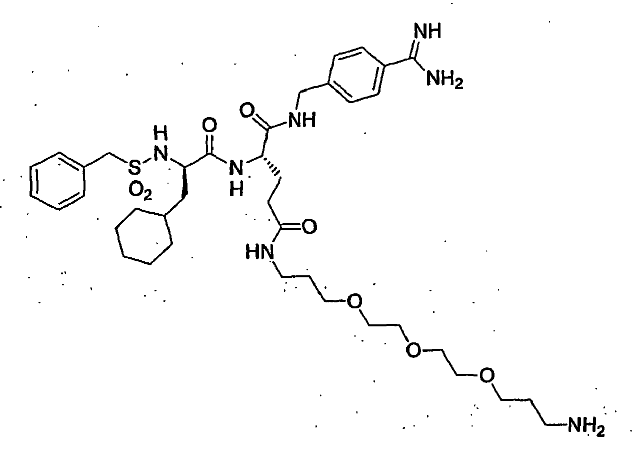 wobei D-Cha in Position P3 insbesondere auch D-Phe oder D-Ser(tBu) sein kann.
18. Verwendung nach mindestens einem der 1 bis 17, **dadurch gekennzeichnet, dass** in der allgemeinen Formel I P4 am Aromaten einen Rest R trägt, P3 . D-Ser, D-Ser(tBu), D-Phe oder D-Cha und P2 eine natürliche oder unnatürliche Aminosäure Aaa bedeutet, wobei R gleich H-, 4-, 3- oder 2-, vorzugsweise 4- oder 3-COOH, 4-, 3- oder 2-, vorzugsweise 4- oder 3-COOMe, 4-, 3- oder 2-, vorzugsweise 4- oder 3-AMe, 4-, 3- oder 2-, vorzugsweise 4- oder 3-Glutaryl-AMe oder 4-, 3- oder 2-, vorzugsweise 4- oder 3-CN ist und Aaa gleich Gly, Ala, Pro, Asp, Glu, Gln, hGlu, Dap, Dap(Z), Lys, Lys(Z), Arg, Thr, Thr(Bzl), Ser, Ser(Bzl), hSer, hSer(Bzl), Phe oder hPhe ist.
19. Verwendung nach 18, **dadurch gekennzeichnet, dass** bei P3 gleich D-Ser Aaa vorzugsweise Gln, Dap, Dap(Z), Lys, Lys(Z), Ser(Bzl), hSer, Phe oder hPhe, insbesondere Lys(Z) ist und R gleich H oder bei Aaa gleich Ala oder Ser R gleich HOOC- ist;
   oder bei P3 gleich D-Ser(tBu) Aaa gleich Pro, Gln, Dap, Dap(Z), Lys, Lys(Z), Arg, Thr, Thr(Bzl), Ser(Bzl), hSer(Bzl), Phe oder hPhe, insbesondere Pro, Gln, Lys, Lys(Z), hSer(Bzl), Phe oder hPhe ist und R gleich H oder bei Aaa gleich Gly oder Ala R gleich HOOC- ist oder bei Aaa gleich Pro R gleich CN- ist;
   oder bei P3 gleich D-Cha Aaa gleich Lys oder Glu ist und R gleich H oder bei Aaa gleich Pro R gleich Glutaryl-AMe ist, insbesondere bei Aaa gleich -NH-CH-[CH₂-CH₂-CO-NH-(CH₂)₃-[O-(CH₂)₂]₃-CH₂-NH₂]-CO- R gleich H ist.
20. Verwendung nach mindestens einem der 1-19, **dadurch gekennzeichnet, dass** das acylierte 4-Amidino- oder 4-Guanidinobenzylamin in Form eines Salzes, insbesondere als Salz einer Mineralsäure beispielsweise Schwefelsäure oder Salzsäure oder einer geeigneten organischen Säure beispielsweise Essigsäure, Ameisensäure, Methylsulfonsäure, Bernsteinsäure, Äpfelsäure oder Trifluoressigsäure, insbesondere als Hydrochlorid, Sulfat oder Acetat vorliegt.
21. Verwendung nach mindestens einem der 1-20, **dadurch gekennzeichnet, dass** eine H₂N-Gruppe einer an das acylierte 4-Amidino- oder 4-Guanidinobenzylamin gekoppelten Linkergruppe mit einem Dicarbonsäureanhydrid, vorzugsweise dem Anhydrid der Bernsteinsäure oder der Glutarsäure unter Bildung einer HOOC-Gruppe umgesetzt werden kann oder dass eine HOOC-Gruppe einer an das acylierte 4-Amidino- oder 4-Guanidinobenzylamin gekoppelten Linkergruppe mit einem Diamin unter Beibehaltung einer H₂N-Gruppe umgesetzt werden kann.
22. Verwendung nach mindestens einem der 1 bis 10, 12 oder 14 bis 17, **dadurch gekennzeichnet, dass** die kovalent an P4 oder P2 gekoppelte Linkergruppe bei Vorhandensein einer zweiten funktionellen Gruppe, insbesondere einer substituierten oder unsubstituierten Amino-, Carboxyl- und/oder Mercaptogruppe, kovalent an eine künstliche Oberfläche oder, sofern es sich bei der Linkergruppe um ein Oligo- oder Polyalkylenglycol handelt, kovalent an ein zweites Molekül der allgemeinen Formel I gekoppelt ist.
23. Verwendung nach mindestens einem der 1 bis 8, 11, 13 oder 14, dadurch gekennzeichnet, dass die kovalent an P4 oder P2 gekoppelte Linkergruppe ein Oligo- oder Polyalkylenglykol ist, das an dem nicht an P4 oder P2 gekoppelten Ende mit einer Alkylgruppe mit 1-4 C-Atomen, insbesondere Methyl oder mit einem zweiten Molekül der allgemeinen Formel I modifiziert sein kann, wobei die Linkergruppe durch Interaktion mit einer künstlichen Oberfläche nicht-kovalent an diese gekoppelt sein kann.
24. Verwendung nach mindestens einem der 22 oder 23, **dadurch gekennzeichnet, dass** die künstliche Oberfläche aus Cellulose-Diacetat, Cellulose-Triacetat, Poly(ethersulfon), Poly(arylethersulfon), regenerierter Cellulose, Cuprophan, Hemophan, Poly(sulfon), Poly(acrylnitril), Poly(vinylalkohol), Poly(carbonat), Poly(amid), Poly(methylmethacrylat), Poly(ethylen-co-vinylalkohol) oder einem anderen in Geräten wie Dialysatoren, Oxygenatoren, Kathetern, Membranen und/oder den zu den Geräten gehörenden Schlauchsystemen und/oder Luftfallen für die Oberflächen, die mit Blut in Kontakt kommen, verwendeten Material besteht, wobei das Oberflächenmaterial für die kovalente Kopplung des Moleküls der allgemeinen Formel I über die an P4 oder P2 gekoppelte Linkergruppe gegebenenfalls mit funktionellen Gruppen, z.B. Aminogruppen, Aminoalkylgruppen, Carboxylgruppen, Carboxyalkylgruppen, Mercaptogruppen, Mercaptoalkylgruppen, Hydroxylgruppen, Hydroxyalkylgruppen modifiziert sind.
25. Verwendung nach mindestens einem der 1 bis 24 zur Verhinderung der Blutgerinnung an künstlichen Oberflächen von z.B. Geräten wie Dialysatoren, Oxygenatoren, Kathetern, Membranen und/oder den zu den Geräten gehörenden Schlauchsystemen und/oder Luftfallen.
26. Verwendung nach 25 zur Verhinderung der Blutgerinnung an künstlichen Oberflächen durch kovalente oder nicht-kovalente Beschichtung der künstlichen Oberfläche(n) über eine Linkergruppe wie in einem der 4 bis 23 definiert.
27. Verwendung von acyliertem 4-Amidino- oder 4-Guanidinobenzylamin, wie in mindestens einem der 1 bis 23 definiert, zur Herstellung eines Arzneimittels zur Verwendung als Antikoagulanz und/oder Antithrombotikum zur Verhinderung und/oder Behandlung von Herzinfarkt, Hirnschlag, Embolien, tiefen Beinvenenthrombosen z.B. nach Hüftgelenksoperationen und/oder Kniegelenksersatz, instabiler Angina pectoris, Komplikationen infolge von Angioplastie, insbesondere perkutaner transluminaler Koronarangioplastie (PTCA).
28. Verwendung von acyliertem 4-Amidino- oder 4-Guanidinobenzylamin, wie in mindestens einem der 1 bis 23 definiert, zur Herstellung eines Arzneimittels zur Verwendung als Antikoagulanz und/oder Antithrombotikum zur Verhinderung und Therapie von disseminierter intravaskulärer Gerinnung, septischem Schock, Allergien, dem Postgastrektomiesyndrom, Arthritis und ARDS (adult respiratory distress syndrome).
29. Verwendung von acyliertem 4-Amidino- oder 4-Guanidinobenzylamin, wie in mindestens einem der 1 bis 22 definiert, zur Herstellung eines Arzneimittels zur Inhibition von Plasmakallikrein und/oder Faktor XIIa und/oder Faktor XIa in parenteraler Anwendungsform, insbesondere in intraarterieller, intravenöser, intramuskulärer oder subkutaner Form, in enteraler Anwendungsform, insbesondere zur oralen oder rektalen Anwendung oder in topischer Anwendungsform, insbesondere als Dermatikum.
30. Verwendung nach 29 zur Inhibition von Plasmakallikrein.
31. Verwendung von acyliertem 4-Amidino- oder 4-Guanidinobenzylamin, wie in mindestens einem der 1 bis 21 definiert, zur Herstellung eines Arzneimittels zur Inhibition von Plasmakallikrein und/oder Faktor XIIa und/oder Faktor XIa insbesondere in Form einer Tablette, eines Dragees, einer Kapsel, eines Pellets, Suppositoriums, einer Lösung, insbesondere einer Injektions- oder Infusionslösung, von Augentropfen, Nasentropfen, eines Safts, einer Kapsel, einer Emulsion oder Suspension, eines Globuli, Styli, Aerosols, Puders, einer Paste, Creme oder Salbe.
32. Verwendung nach 31 zur Inhibition von Plasmakallikrein.
33. Verwendung von acyliertem Amidinobenzylamin der allgemeinen Formel V oder VI, wie in 8 definiert, mit R₄ insbesondere gleich HO- und R₁ und R₃ keine Oligo- oder Polyalkylengruppe zur Herstellung eines Arzneimittels zur Verwendung als Antikoagulanz und/oder Antithrombotikum, wie in mindestens einem der 27 bis 32 definiert, wobei der Wirkstoff in Form eines Prodrugs zur oralen Gabe vorliegt.
34. Verwendung nach mindestens einem der 1 bis 33, **dadurch gekennzeichnet, dass** das acylierte Amidino- oder Guanidinobenzylamin zur Inhibierung weiterer trypsinartiger Serinproteasen wie beispielsweise Thrombin, Faktor XIIa, Faktor XIa, Faktor Xa, Faktor IXa, Faktor VIIa, Urokinase, Tryptase und Plasmin sowie trypsinartiger Serinproteasen des Komplementsystems verwendet wird, insbesondere acylierte Amidino- oder Guanidinobenzylamine der allgemeinen Formel I mit einer Linkergruppe an P4 oder P2 wie in den 4 bis 23 definiert.
35. Acyliertes 4-Amidino- oder 4-Guanidinobenzylamin gemäß der allgemeinen Formel I 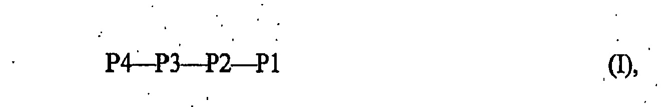 wobei P4 eine einfach oder mehrfach substituierte oder unsubstituierte Benzylsulfonylgruppe ist, P3 eine einfach oder mehrfach substituierte oder unsubstituierte, natürliche oder unnatürliche α-Amino- oder α-Iminosäure in der D-Konfiguration ist,
   P2 eine einfach oder mehrfach substituierte oder unsubstituierte, natürliche oder unnatürliche α-Amino- oder α-Iminosäure in der L-Konfiguration ist, und P1 eine einfach oder mehrfach substituierte oder unsubstituierte 4-Amidino- oder 4-Guanidinobenzylamingruppe ist,
   **dadurch gekennzeichnet, dass** zusätzlich eine Linkergruppe an P4 oder P2 gekoppelt ist, wobei die Linkergruppe über einen wie in 3 definierten Substituenten an P4 oder direkt an eine funktionelle Gruppe von P2, insbesondere über eine -NH- oder eine -CO-Gruppe gekoppelt ist, wobei die Linkergruppe vorzugsweise eine Dicarbonsäure, eine Aminocarbonsäure, ein Diamin, eine Disulfonsäure, oder eine Aminosulfonsäure mit einem Alkyl-, Aryl- oder Aralkylgrundgerüst ist, wobei das Alkylgrundgerüst 1 bis 12 C-Atome, insbesondere 2-6 C-Atome aufweist, das Arylgrundgerüst 6-10 C-Atome, insbesondere Phenyl aufweist, das Aralkylgrundgerüst 6-12 C-Atome, insbesondere Benzyl aufweist, oder eine Aminoalkyl- oder Carboxyalkylgruppe mit 2-12 C-Atomen, insbesondere 2-6 C-Atomen; oder wobei die Linkergruppe an P4 oder P2 eine Oligo- oder Polyalkylenglycolkette ist, insbesondere eine Poly- oder Oligoethylen- oder Poly- oder Oligopropylenglycolkette ist, wobei das Oligo- oder Polyalkylenglycol zumindest an beiden Enden eine funktionelle Gruppe, insbesondere eine substituierte oder unsubstituierte Amino-, Carboxyl- und/oder Mercaptogruppe aufweist oder wobei das Oligo- oder Polyalkylenglycol zumindest an einem Ende eine funktionelle Gruppe, insbesondere eine substituierte oder unsubstituierte Amino-, Carboxyl- und/oder Mercaptogruppe aufweist und am anderen Ende mit einer Alkylgruppe mit 1-4 C-Atomen, insbesondere Methyl modifiziert ist,
   wobei bei Kopplung der Linkergruppe an P4 über einen Substituenten der Substituent vorzugsweise eine -NH-Gruppe, -NH-Alkyl-Gruppe mit 1 bis 6 C-Atomen, insbesondere Methyl, eine -CO-Gruppe, eine -CO-Alkyl-Gruppe mit 2-6 C-Atomen, insbesondere -CO-Methyl, eine -CO-O-Alkyl-Gruppe mit 1-6 C-Atomen, insbesondere Methyl, eine -S-Gruppe, eine -S-Alkyl-Gruppe mit 1 bis 6 C-Atomen, insbesondere Methyl, eine -O-Alkyl-Gruppe mit 1-6 C-Atomen, insbesondere Methyl, eine -SO₂-Gruppe oder eine -SO₂-Alkyl-Gruppe mit 1-6 C-Atomen, insbesondere Methyl ist oderwobei, bei Kopplung der Linkergruppe an P2, P2 vorzugsweise
   (a) Lysin oder dessen Homologe mit 1-5 C-Atomen in der Seitenkette, insbesondere Ornithin, Homolysin, α-γ-Diaminobuttersäure, α-β-Diaminopropionsäure, α-Diaminoglycin oder
   (b) Glutaminsäure oder dessen Homologe mit 1-5 C-Atomen in der Seitenkette, insbesondere Asparaginsäure, Glutaminsäure oder Homoglutaminsäure oder
   (c) Cystein oder Homocystein oder
   (d) Serin oder Threonin ist.
36. Acyliertes 4-Amidino- oder 4-Guanidinobenzylamin nach 35, **dadurch gekennzeichnet, dass** die Linkergruppe an P4 oder P2 eine Oligo- oder Polyalkylenglycolkette ist, insbesondere eine Poly- oder Oligoethylen- oder Poly- oder Oligopropylenglycolkette ist, wobei das Oligo- oder Polyalkylenglycol zumindest an beiden Enden eine funktionelle Gruppe, insbesondere eine substituierte oder unsubstituierte Amino-, Carboxyl- und/oder Mercaptogruppe aufweist oder wobei das Oligo- oder Polyalkylenglycol zumindest an einem Ende eine funktionelle Gruppe, insbesondere eine substituierte oder unsubstituierte Amino-, Carboxyl- und/oder Mercaptogruppe aufweist und am anderen Ende mit einer Alkylgruppe mit 1-4 C-Atomen, insbesondere Methyl modifiziert ist.
37. Acyliertes 4-Amidino- oder 4-Guanidinobenzylamin nach 35 oder 36, **dadurch gekennzeichnet, dass** es die allgemeine Formel V oder VI, wie in 8 definiert, aufweist 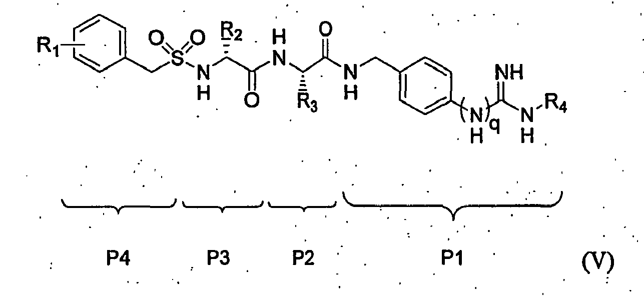 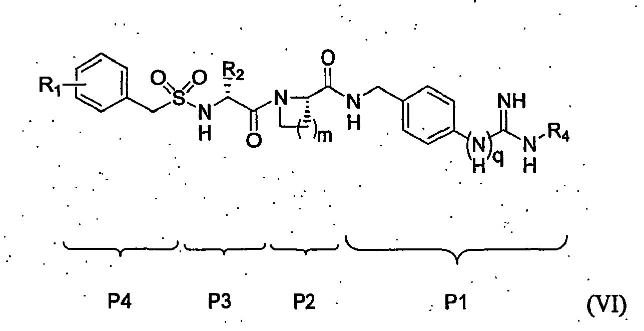 mit m = 1 bis 3 und q = 0 oder 1, insbesondere 0,
   wobei R₁, R₂, R₃ und/oder R₄
   (a) Wasserstoff ist und/oder
   (b) ein Halogen, vorzugsweise Fluor, Chlor und/oder Brom ist, und/oder
   (c) ein substituierter oder nicht-substituierter, verzweigter oder linearer Alkylrest mit 1-6 C-Atomen, vorzugsweise 1-3 C-Atomen, insbesondere Methyl, oder ein substituierter oder nicht-substituierter, verzweigter oder linearer Aralkylrest mit 1-10 C-Atomen ist, wobei der Substituent des substituierten, verzweigten oder linearen Alkylrests oder Aralkylrests vorzugsweise eine Halogen-, Hydroxy-, Amino-, Cyano-, Amidino-, Guanidino- und/oder eine Carboxylgruppe, gegebenenfalls verestert mit einem Niederalkylrest, insbesondere mit Methyl oder Ethyl ist und/oder
   (d) eine Hydroxy-, Amino-, Cyano-, Amidino-, Guanidino-, Methyloxycarbonyl-, Benzyl-, Benzyloxycarbonyl-, Aminomethyl- oder Glutaryl- oder Succinyl-Amidomethylgruppe ist und/oder eine Oxyalkylcarbonyl-, Carboxyl-, Carboxymethyl- oder Carboxyethylgruppe ist gegebenenfalls verestert mit einem Niederalkylrest, insbesondere mit Methyl oder Ethyl oder eine Oxyalkylcarbonyl-, Carboxyl-, Carboxymethyl- oder Carboxyethylgruppe, die als unsubstituiertes oder mit einer Alkyl- oder Arylgruppe substituiertes Amid vorliegt und/oder
      R₁ und/oder R₃ eine Linkergruppe sein kann, wobei die Linkergruppe über einen Substituenten wie in 2 definiert an P4 oder direkt an eine funktionelle Gruppe von P2, insbesondere über eine -NH- oder eine -CO-Gruppe gekoppelt ist, wobei die Linkergruppe vorzugsweise eine Dicarbonsäure, eine Aminocarbonsäure,
      ein Diamin, eine Disulfonsäure, oder eine Aminosulfonsäure mit einem Alkyl-, Aryl- oder Aralkylgrundgerüst ist, wobei das Alkylgrundgerüst 1 bis 12 C-Atome, insbesondere 2-6 C-Atome aufweist, das Arylgrundgerüst 6-10 C-Atome, insbesondere Phenyl aufweist, das Aralkylgrundgerüst 6-12 C-Atome, insbesondere Benzyl aufweist, oder eine Aminoalkyl- oder Carboxyalkylgruppe mit 2-12 C-Atomen, insbesondere 2-6 C-Atomen; oder wobei die Linkergruppe an P4 oder P2 eine Oligo- oder Polyalkylenglycolkette ist, insbesondere eine Poly- oder Oligoethylen- oder Poly- oder Oligopropylenglycolkette ist, wobei das Oligo- oder Polyalkylenglycol zumindest an beiden Enden eine funktionelle Gruppe, insbesondere eine substituierte oder unsubstituierte Amino-, Carboxyl- und/oder Mercaptogruppe aufweist oder wobei das Oligo- oder Polyalkylenglycol zumindest an einem Ende eine funktionelle Gruppe, insbesondere eine substituierte oder unsubstituierte Amino-, Carboxyl- und/oder Mercaptogruppe aufweist und am anderen Ende mit einer Alkylgruppe mit 1-4 C-Atomen, insbesondere Methyl modifiziert ist und/oder
      R₁ die Formel (II) wie in 5 definiert und P2 mit R₃ die Formeln (III) und (IV), wie in 7 definiert, aufweist, und
      R₄ besonders bevorzugt Hydroxy, Amino und Alkoxycarbonyl ist.
38. Acyliertes 4-Amidino- oder 4-Guanidinobenzylamin nach mindestens einem der 35 bis 37, **dadurch gekennzeichnet, dass** es eine Linkergruppe an P4 aufweist und eine wie in den 9, 10, 11, 15 definierte Struktur aufweist.
39. Acyliertes 4-Amidino- oder 4-Guanidinobenzylamin nach mindestens einem der 35 bis 37, **dadurch gekennzeichnet, dass** es eine Linkergruppe an P2 aufweist und eine wie in den 12, 13 oder 17 definierte Struktur aufweist.
40. Acyliertes 4-Amidino- oder 4-Guanidinobenzylamin gemäß der allgemeinen Formel I 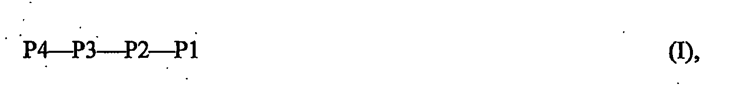 wobei P1, P2, P3 und P4 die in den 1 bis 23 genannten Bedeutungen haben, **dadurch gekennzeichnet, dass** das acylierte 4-Amidino- oder 4-Guanidinobenzylamin kovalent oder nicht-kovalent an eine künstliche Oberfläche gebunden ist.
41. Acyliertes 4-Amidino- oder 4-Guanidiriobenzylamin nach 40, **dadurch gekennzeichnet, dass** das acylierte 4-Amidino- oder 4-Guanidinobenzylamin über eine Amid- oder Sulfonamidbindung, eine Disulfidbrücke oder die Alkylierung einer Mercaptogruppe, insbesondere über eine Amidbindung kovalent an die künstliche Oberfläche gebunden ist.
42. Acyliertes 4-Amidino- oder 4-Guanidinobenzylamin nach 40, **dadurch gekenntzeichnet, dass** das acylierte 4-Amidino- oder 4-Guanidinobenzylamin über Interaktionen einer Oligo- oder Polyalkylenglykolgruppe, insbesondere einer Oligo- oder Polyethylenglykolgruppe mit der künstlichen Oberfläche nicht-kovalent an diese Oberfläche gebunden ist.
43. Künstliche Oberfläche, **dadurch gekennzeichnet, dass** die Oberfläche mit acyliertem 4-Amidino- oder 4-Guanidinobenzylamin gemäß mindestens einem der 35 bis 39 oder mit acyliertem 4-Amidino- oder 4-Guanidinobenzylamin, wie in mindestens einem der 1 bis 23 definiert, kovalent oder nicht-kovalent beschichtet ist.
44. Gerät, enthaltend eine künstliche Oberfläche gemäß 43.
45. Gerät nach 44, **dadurch gekennzeichnet, dass** das Gerät ein Dialysator, Oxygenator, Katheter oder eine Membran ist.

## Patentansprüche

1. Acyliertes Amidino- oder Guanidinobenzylamin zur Inhibierung von Plasmakallikrein und/oder Faktor XIa und/oder Faktor XIIa, **dadurch gekennzeichnet, dass** das acylierte Amidino- oder Guanidinobenzylamin die allgemeine Formel V oder VI aufweist 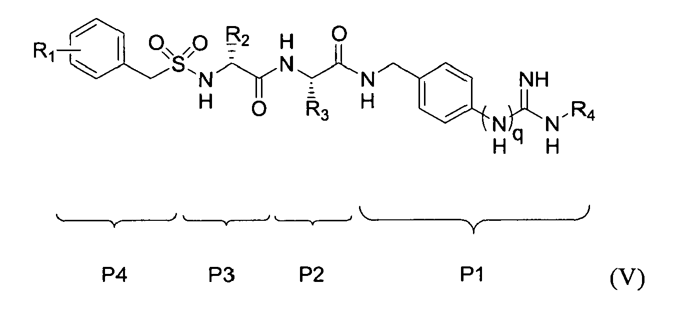 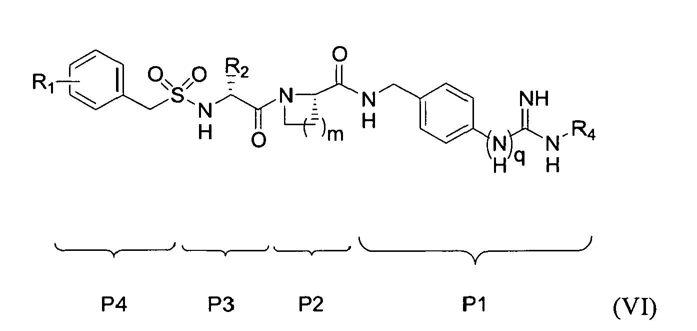 mit m = 1 bis 3 und q = 0 oder 1, insbesondere 0,
wobei R₁, R₂, R₃ und/oder R₄
(a) Wasserstoff ist und/oder
(b) ein Halogen, vorzugsweise Fluor, Chlor und/oder Brom ist, und/oder
(c) ein substituierter oder nicht-substituierter, verzweigter oder linearer Alkylrest mit 1-6 C-Atomen, vorzugsweise 1-3 C-Atomen, insbesondere Methyl ist, wobei der Substituent des substituierten, verzweigten oder linearen Alkylrests vorzugsweise eine Halogen-, Hydroxy-, Amino-, Cyano-, Amidino-, Guanidino- und/oder eine Carboxylgruppe, gegebenenfalls verestert mit einem Niederalkylrest, insbesondere mit Methyl oder Ethyl ist und/oder
(d) eine Hydroxy-, Amino-, Cyano-, Amidino-, Guanidino-, Methyloxycarbonyl-, Benzyl-, Benzyloxycarbonyl-, Aminomethyl- oder Glutaryl- oder Succinyl-Amidomethylgruppe ist und/oder eine Oxyalkylcarbonyl-, Carboxyl-, Carboxymethyl- oder Carboxyethylgruppe ist gegebenenfalls verestert mit einem Niederalkylrest, insbesondere mit Methyl oder Ethyl oder eine Oxyalkylcarbonyl-, Carboxyl-, Carboxymethyl- oder Carboxyethylgruppe, die als unsubstituiertes oder mit einer Alkyl- oder Arylgruppe substituiertes Amid vorliegt und/oder R₁ und/oder R₃ eine Linkergruppe sein kann, wobei die Linkergruppe über einen Substituenten ausgewählt aus
(a) Wasserstoff und/oder
(b) einem Halogen, vorzugsweise Fluor, Chlor und/oder Brom, und/oder
(c) einem substituierten oder nicht-substituierten, verzweigten oder linearen Alkylrest mit 1-6 C-Atomen, vorzugsweise 1-3 C-Atomen, insbesondere Methyl, oder einem substituierten oder nicht-substituierten, verzweigten oder linearen Aralkylrest mit 1-10 C-Atomen, wobei der Substituent des substituierten, verzweigten oder linearen Alkylrests oder Aralkylrestes vorzugsweise eine Halogen-, Hydroxy-, Amino-, Cyano-, Amidino-, Guanidino- und/oder eine Carboxylgruppe, gegebenenfalls verestert mit einem Niederalkylrest, insbesondere mit Methyl oder Ethyl ist, und/oder
(d) einer Hydroxy-, Amino-, Cyano-, Amidino-, Guanidino-, Methyloxycarbonyl-, Benzyl-, Benzyloxycarbonyl-, Aminomethyl- oder Glutaryl- oder Succinyl-Amidomethylgruppe und/oder einer Oxyalkylcarbonyl-, Carboxyl-, Carboxymethyl- oder Carboxyethylgruppe gegebenenfalls verestert mit einem Niederalkylrest, insbesondere mit Methyl oder Ethyl oder einer Oxyalkylcarbonyl-, Carboxyl-, Carboxymethyl- oder Carboxyethylgruppe, die als unsubstituiertes oder mit einer Alkyl- oder Arylgruppe substituiertes Amid vorliegt,
an P4 oder direkt an eine funktionelle Gruppe von P2, insbesondere über eine -NH- oder eine -CO-Gruppe gekoppelt ist, wobei die Linkergruppe vorzugsweise eine Dicarbonsäure, eine Aminocarbonsäure, ein Diamin, eine Disulfonsäure, oder eine Aminosulfonsäure mit einem Alkyl-, Aryl- oder Aralkylgrundgerüst ist, wobei das Alkylgrundgerüst 1 bis 12 C-Atome, insbesondere 2-6 C-Atome aufweist, das Arylgrundgerüst 6-10 C-Atome, insbesondere Phenyl aufweist, das Aralkylgrundgerüst 6-12 C-Atome, insbesondere Benzyl aufweist, oder eine Aminoalkyl- oder Carboxyalkylgruppe mit 2-12 C-Atomen, insbesondere 2-6 C-Atomen; oder wobei die Linkergruppe an P4 oder P2 eine Oligo- oder Polyalkylenglycolkette ist, insbesondere eine Poly- oder Oligoethylen- oder Poly- oder Oligopropylenglycolkette ist, wobei das Oligo- oder Polyalkylenglycol zumindest an beiden Enden eine funktionelle Gruppe, insbesondere eine substituierte oder unsubstituierte Amino-, Carboxyl- und/oder Mercaptogruppe aufweist oder wobei das Oligo- oder Polyalkylenglycol zumindest an einem Ende eine funktionelle Gruppe, insbesondere eine substituierte oder unsubstituierte Amino-, Carboxyl- und/oder Mercaptogruppe aufweist und am anderen Ende mit einer Alkylgruppe mit 1-4 C-Atomen, insbesondere Methyl modifiziert ist und/oder R₁ die Formel (II) 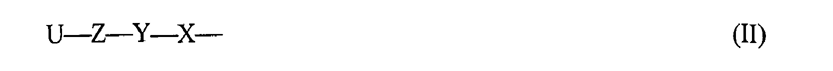 wobei
U gleich eine H₂N-, HOOC-(CH₂)ₙ-CO-NH-, HOOC-, H₂N-(CH₂)ₙ-NH-CO- oder HS-Gruppe ist mit Z gleich -(CH₂)ₙ- mit n = 1 bis 10, insbesondere 1-5, oder Z gleich ein Oligo- oder Polyalkylenglycol der allgemeinen Formel -(CH₂)_{d}-[O-CH₂-CH₂]ᵥO-(CH₂)ₘ-(NH-CO-CH₂-O-CH₂)ₖ- oder -(CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 50, insbesondere 2 bis 10, m = 0, 1, 2, 3 oder 4 und k = 0 oder 1 oder
U gleich eine CH₃-O-Gruppe ist mit Z gleich ein Oligo- oder Polyalkylenglycol der allgemeinen Formel -(CH₂)_{d}-[O-CH₂-CH₂]ᵥO-(CH₂)ₘ-(NH-CO-CH₂-O-CH₂)ₖ- oder -(CH₂)_{d}-[O-CH(CH₃)-CH₂]ᵥ-O-(CH₂)ₘ-(NH-CO-CH₂-O-CH₂)ₖ- mit d = 1, 2, 3 oder 4, v = eine ganze Zahl von 1 bis 1000, bevorzugt 1 bis 50, insbesondere 2 bis 10, m = 0, 1, 2, 3 oder 4 und k = 0 oder 1 ist;
Y gleich eine -CO-NH-, eine -NH-CO-, eine -SO₂-NH-, eine -NH-SO₂-, eine -S-S- oder eine -S-Gruppe ist oder wenn U und Z nicht vorhanden sind gleich eine H₂N-, HOOC-, HS-, HO- oder Halogenalkyl-Gruppe ist;
X gleich eine -(CH₂)ₙ-Gruppe mit n = 0, 1, 2, 3 oder 4, insbesondere n = 1 oder gleich eine -(CH₂)ₙ-O-Gruppe mit Bindung an den Benzylrest über den Sauerstoff und n = 1, 2, 3 oder 4 ist;
und die Kopplung der Linkergruppe an den Phenylring des Benzylrestes von X falls vorhanden oder von Y, wenn X nicht vorhanden ist, ausgeht und P2 mit R₃ die Formel (III) 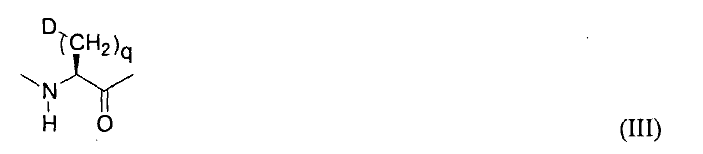 wobei q = 0, 1, 2, 3, 4 oder 5 ist und D gleich Formel IV ist 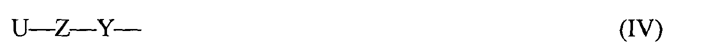 wobei U, Z und Y die gleiche Bedeutung wie bei Formel II besitzen,
aufweist, und R₄ besonders bevorzugt Hydroxy, Amino und Alkoxycarbonyl ist.

2. Acyliertes Amidino- oder Guanidinobenzylamin zur Inhibierung von Plasmakallikrein und/oder Faktor XIa und/oder Faktor XIIa nach Anspruch 1, **dadurch gekennzeichnet, dass**
i) eine Verbindung gemäß der allgemeinen Formel I mit einer Linkergruppe an P4 gemäß der allgemeinen Formel II, wie in Anspruch 1 definiert, eine der folgenden Strukturen aufweist: 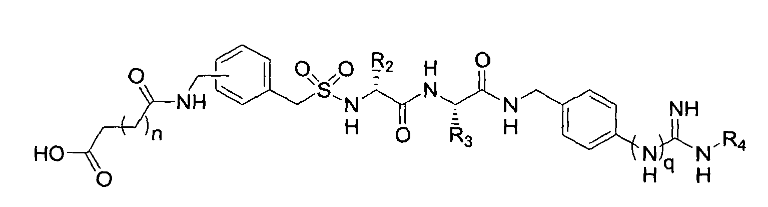 oder 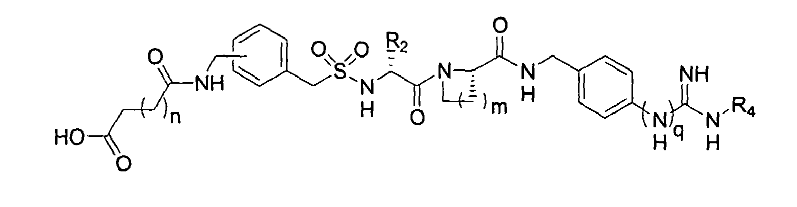 oder 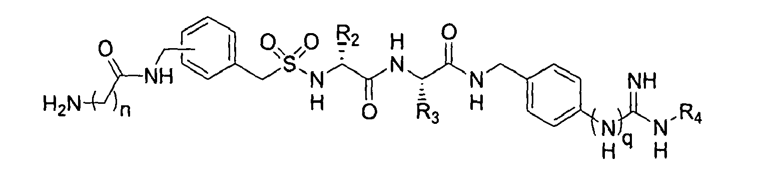 oder 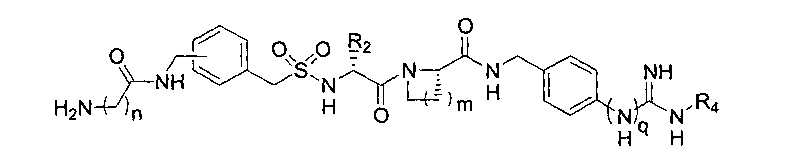 mit n = 1 bis 10, m = 1 bis 3 und q = 0 oder 1, insbesondere 0, wobei R₂ R₃ und R₄ die in Anspruch 1 genannten Bedeutungen besitzen,
oder
ii) eine Verbindung gemäß der allgemeinen Formel I mit einer Linkergruppe an P4 gemäß der allgemeinen Formel II, wie in Anspruch 1 definiert, eine der folgenden Strukturen aufweist: 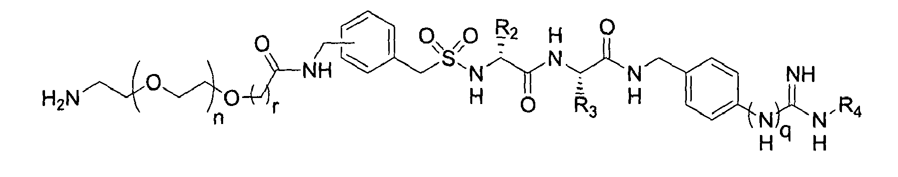 oder 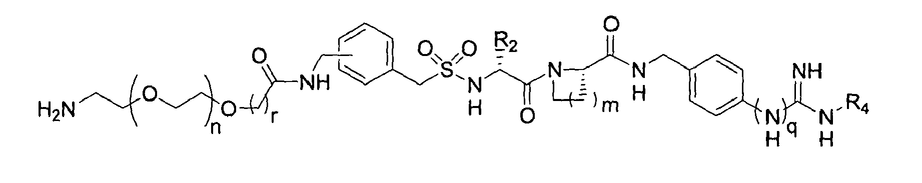 oder 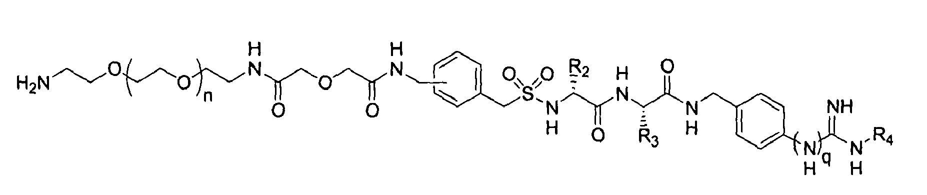 oder 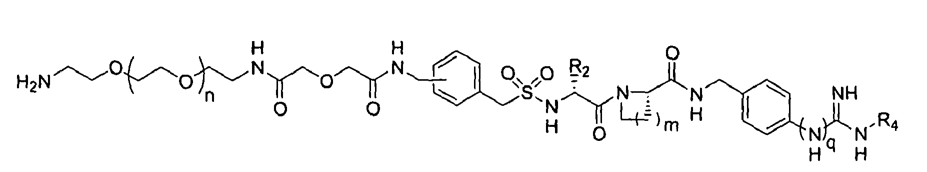 mit n = 1 bis 1000, m = 1 bis 3, r = 0 bis 3 und q = 0 oder 1, insbesondere 0, wobei R₂, R₃ und R₄ die in Anspruch 1 genannten Bedeutungen besitzen, oder
iii) eine Verbindung gemäß der allgemeinen Formel I mit einer Linkergruppe an P4 gemäß der allgemeinen Formel II, wie in Anspruch 1 definiert, eine der folgenden Strukturen aufweist: 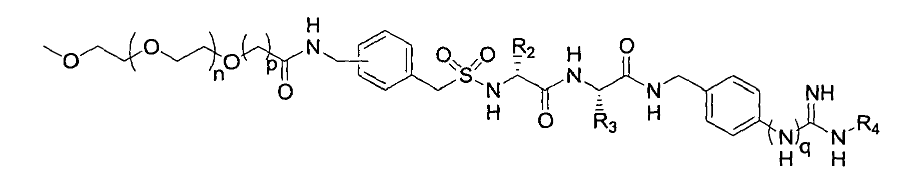 oder 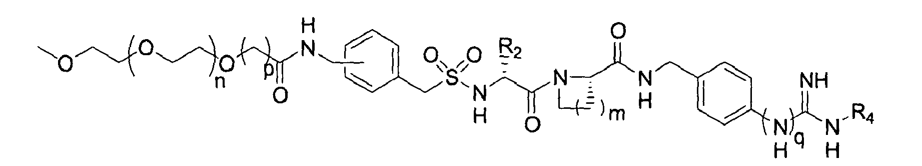 oder 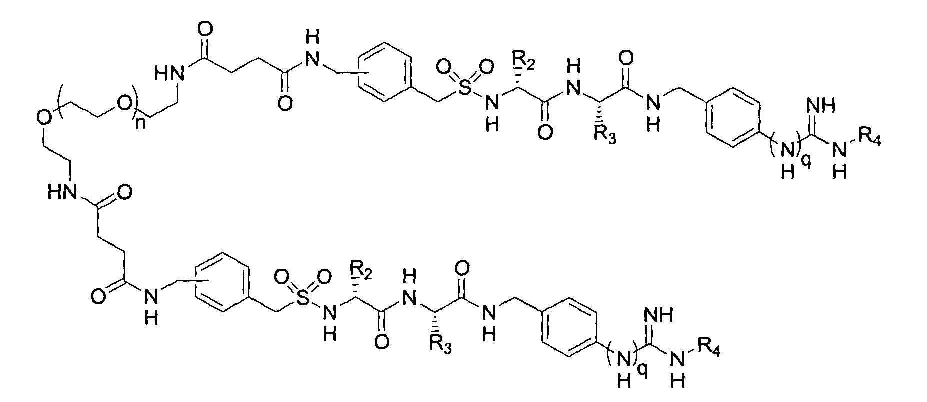 oder 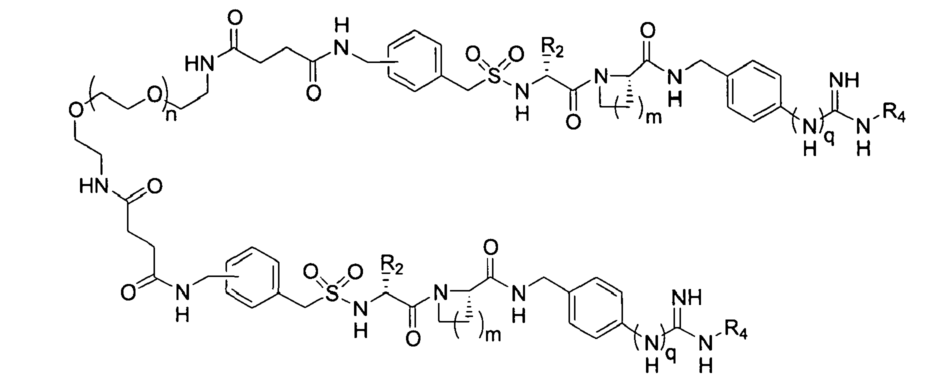 oder 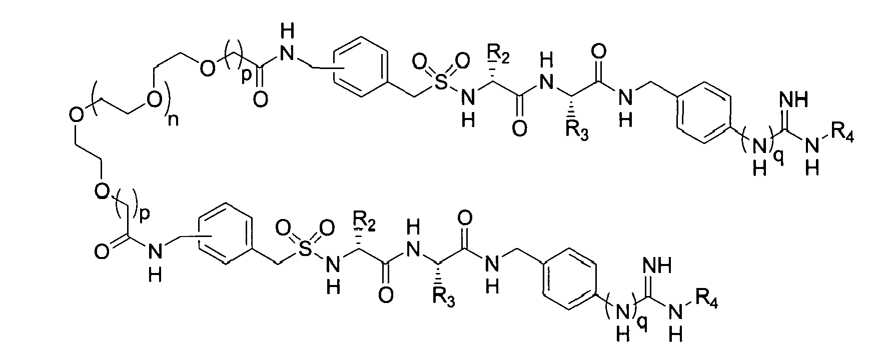 oder 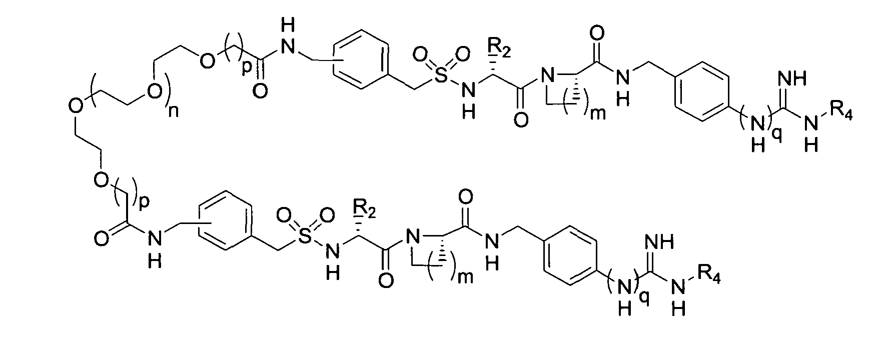 mit p = 0, 1, 2 oder 3, q = 0 oder 1, insbesondere 0, n = 1 bis 1000 und m = 1 bis 3, wobei R₂, R₃ und R₄ die in Anspruch 1 genannten Bedeutungen besitzen.

3. Acyliertes Amidino- oder Guanidinobenzylamin zur Inhibierung von Plasmakallikrein und/oder Faktor XIa und/oder Faktor XIIa nach Anspruch 1, **dadurch gekennzeichnet, dass**
i) eine Verbindung gemäß der allgemeinen Formel I mit einer Linkergruppe an P2 gemäß der allgemeinen Formeln III und IV, wie in Anspruch 1 definiert, eine der folgenden Strukturen aufweist: 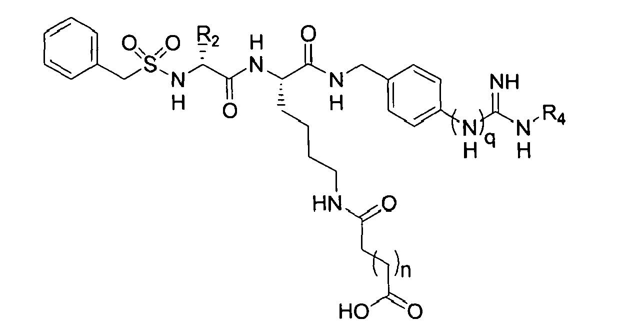 mit n= 0 bis 5, bevorzugt 1 oder 2
oder 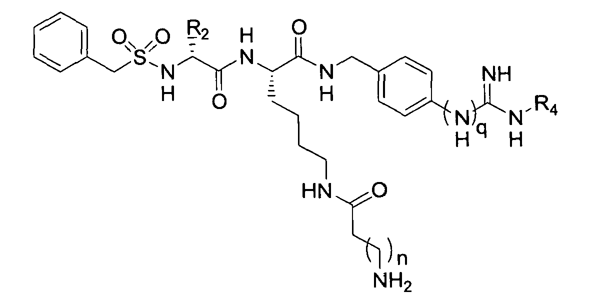 mit n = 0 bis 11,
oder 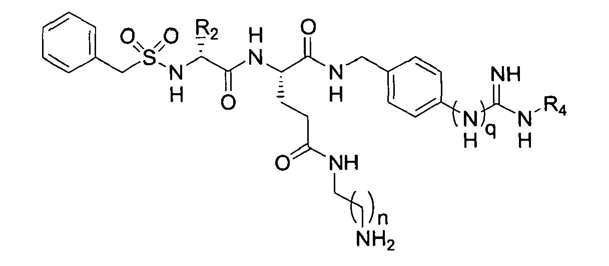 mit n = 1 bis 6
oder 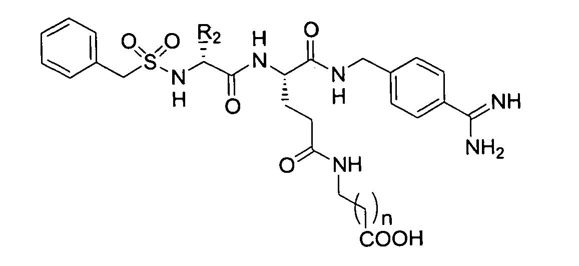 oder 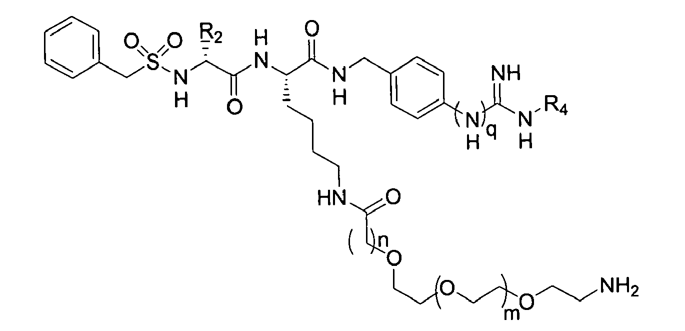 mit n= 0 bis 3 und m = 0 bis 1000
oder 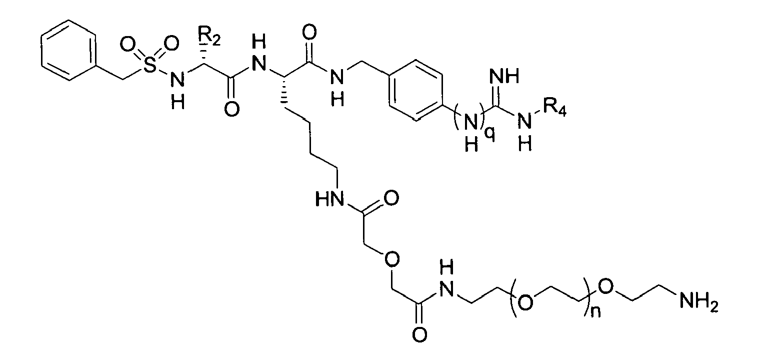 mit n = 1 bis 1000
oder 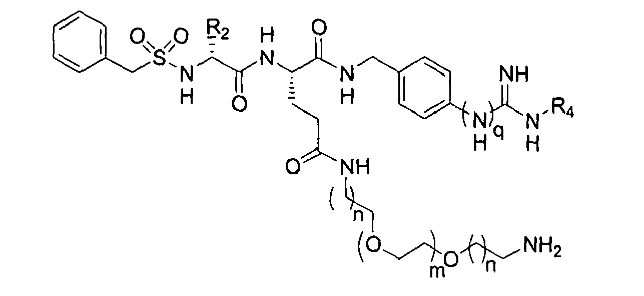 mit n = 1 bis 3 und m=1 bis 1000, wobei q jeweils gleich 0 oder 1, insbesondere 0 ist und R₂ und R₄ jeweils die in Anspruch 1 genannten Bedeutungen besitzen,
oder
ii) eine Verbindung gemäß der allgemeinen Formel I mit einer Linkergruppe an P2 gemäß den allgemeinen Formeln III und IV, wie in Anspruch 1 definiert, eine der folgenden Strukturen aufweist: 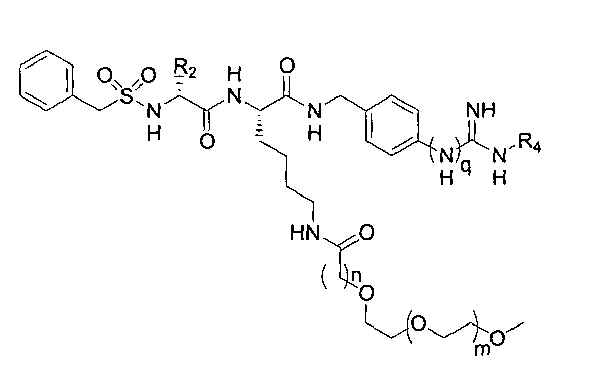 mit n = 0 bis 4 und m = 10 bis 1000 oder 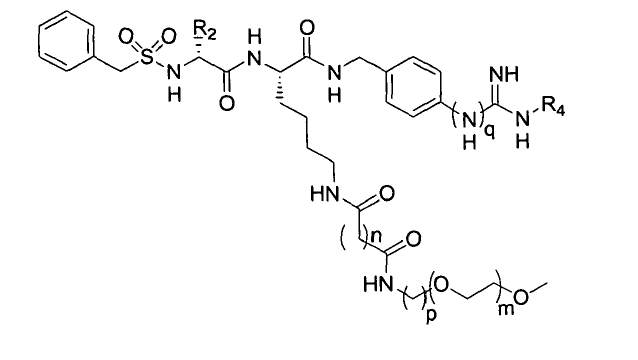 mit n = 1 bis 4, p = 2 bis 4 und m = 1 bis 1000
oder 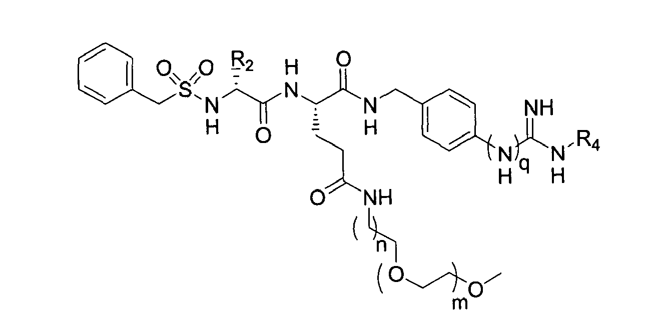 oder 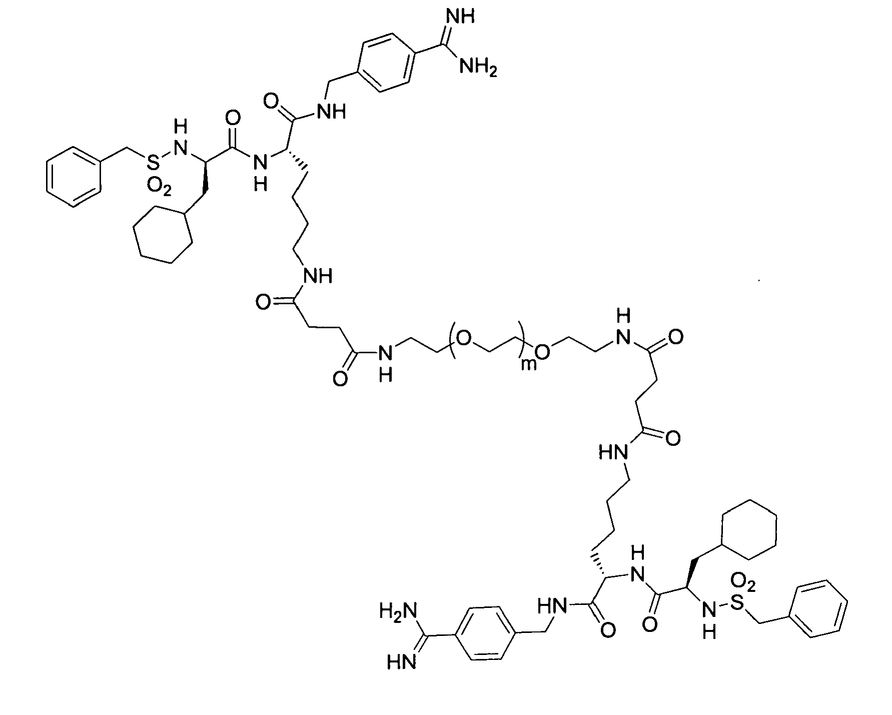 oder 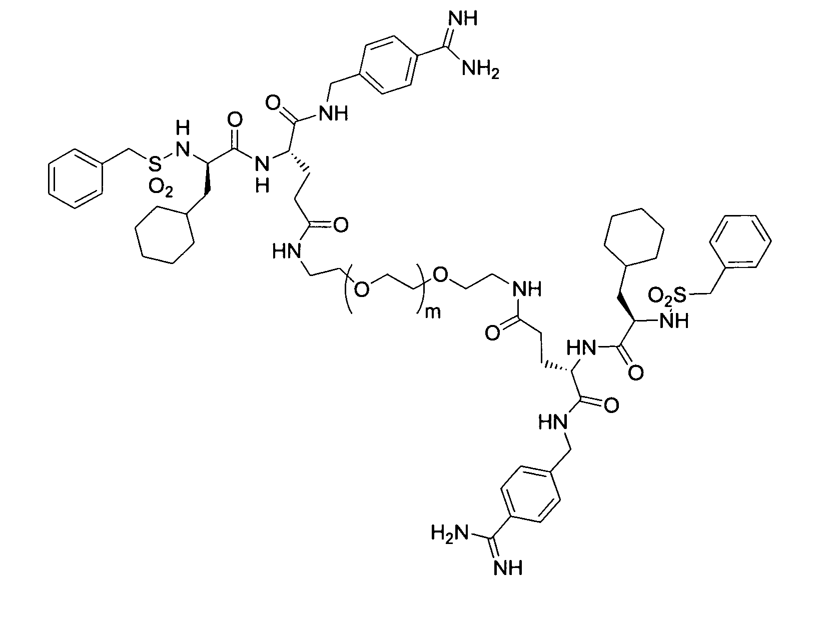 mit n =1 bis 3 und m=10 bis 1000,
wobei q gleich 0 oder 1, insbesondere 0 ist und R₂ und R₄ jeweils die in Anspruch 1 genannten Bedeutungen besitzen.

4. Acyliertes Amidino- oder Guanidinobenzylamin zur Inhibierung von Plasmakallikrein und/oder Faktor XIa und/oder Faktor XIIa nach Anspruch 1 oder 3, wobei eine Kopplung an eine künstliche Oberfläche über P2 erfolgt, **dadurch gekennzeichnet, dass** der Substituent an P4 insbesondere H, ein Halogen, eine Aminogruppe, eine Hydroxygruppe oder eine lineare oder verzweigte Alkylgruppe mit 1 bis 6 Kohlenstoffatomen ist, insbesondere **dadurch gekennzeichnet, dass** eine Verbindung gemäß der allgemeinen Formel I eine der folgenden Strukturen aufweist 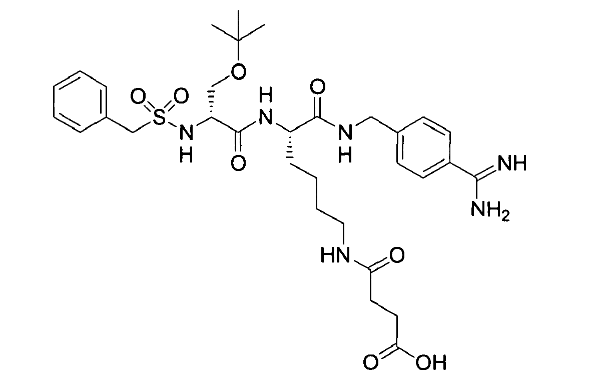 wobei D-Ser(tBu) in Position P3 insbesondere auch D-Cha oder D-Phe und Succinyl an P2 auch Glutaryl sein kann, oder dass eine Verbindung gemäß der allgemeinen Formel I eine der folgenden Strukturen aufweist: 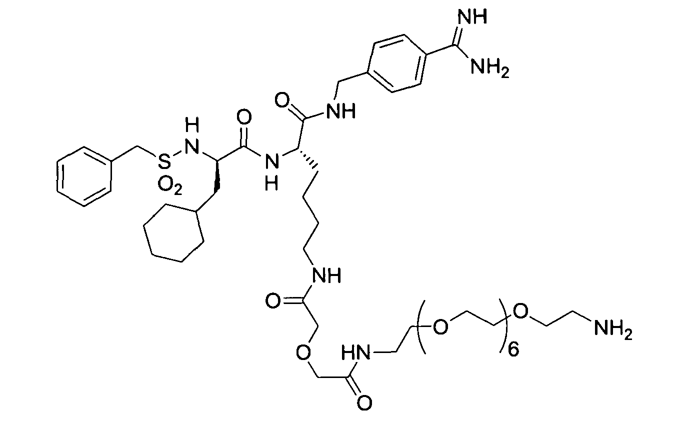 oder 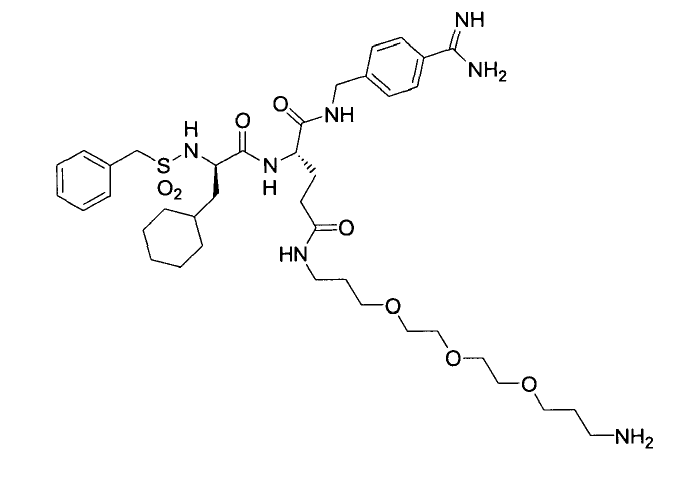 wobei D-Cha in Position P3 insbesondere auch D-Phe oder D-Ser(tBu) sein kann.

5. Acyliertes Amidino- oder Guanidinobenzylamin zur Inhibierung von Plasmakallikrein und/oder Faktor XIa und/oder Faktor XIIa nach 1 oder 2, **dadurch gekennzeichnet, dass** eine Verbindung gemäß der allgemeinen Formel I mit einer Linkergruppe an P4 gemäß der allgemeinen Formel II, wie in Anspruch 1 definiert, die folgende Struktur aufweist 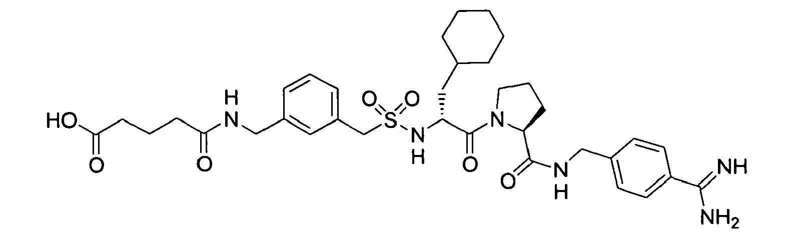 wobei D-Cha in Position P3 insbesondere auch D-Phe oder D-Ser(tBu) und Glutaryl an P4 auch Succinyl sein kann.

6. Acyliertes Amidino- oder Guanidinobenzylamin zur Inhibierung von Plasmakallikrein und/oder Faktor XIa und/oder Faktor XIIa nach mindestens einem der Ansprüche 1 bis 5, **dadurch gekennzeichnet, dass** in der allgemeinen Formel I P4 am Aromaten einen Rest R trägt, P3 D-Ser, D-Ser(tBu), D-Phe oder D-Cha und P2 eine natürliche oder unnatürliche Aminosäure Aaa bedeutet, wobei R gleich H-, 4-, 3- oder 2-, vorzugsweise 4- oder 3-COOH, 4-, 3- oder 2-, vorzugsweise 4- oder 3-COOMe, 4-, 3- oder 2-, vorzugsweise 4- oder 3-AMe, 4-, 3- oder 2-, vorzugsweise 4- oder 3-Glutaryl-AMe oder 4-, 3- oder 2-, vorzugsweise 4- oder 3-CN ist und Aaa gleich Gly, Ala, Pro, Asp, Glu, Gln, hGlu, Dap, Dap(Z), Lys, Lys(Z), Arg, Thr, Thr(Bzl), Ser, Ser(Bzl), hSer, hSer(Bzl), Phe oder hPhe ist, insbesondere **dadurch gekennzeichnet, dass** bei P3 gleich D-Ser Aaa vorzugsweise Gln, Dap, Dap(Z), Lys, Lys(Z), Ser(Bzl), hSer, Phe oder hPhe, insbesondere Lys(Z) ist und R gleich H oder bei Aaa gleich Ala oder Ser R gleich HOOC- ist;
oder bei P3 gleich D-Ser(tBu) Aaa gleich Pro, Gln, Dap, Dap(Z), Lys, Lys(Z), Arg, Thr, Thr(Bzl), Ser(Bzl), hSer(Bzl), Phe oder hPhe, insbesondere Pro, Gln, Lys, Lys(Z), hSer(Bzl), Phe oder hPhe ist und R gleich H oder bei Aaa gleich Gly oder Ala R gleich HOOC- ist oder bei Aaa gleich Pro R gleich CN- ist;
oder bei P3 gleich D-Cha Aaa gleich Lys oder Glu ist und R gleich H oder bei Aaa gleich Pro R gleich Glutaryl-AMe ist, insbesondere bei Aaa gleich -NH-CH-[CH₂-CH₂-CO-NH-(CH₂)₃-[O-(CH₂)₂]₃-CH₂-NH₂]-CO- R gleich H ist.

7. Acyliertes Amidino- oder Guanidinobenzylamin zur Inhibierung von Plasmakallikrein und/oder Faktor XIa und/oder Faktor XIIa nach mindestens einem der Ansprüche 1-6, **dadurch gekennzeichnet, dass** das acylierte 4-Amidino- oder 4-Guanidinobenzylamin in Form eines Salzes, insbesondere als Salz einer Mineralsäure beispielsweise Schwefelsäure oder Salzsäure oder einer geeigneten organischen Säure beispielsweise Essigsäure, Ameisensäure, Methylsulfonsäure, Bernsteinsäure, Äpfelsäure oder Trifluoressigsäure, insbesondere als Hydrochlorid, Sulfat oder Acetat vorliegt, insbesondere **dadurch gekennzeichnet, dass** eine H₂N-Gruppe einer an das acylierte 4-Amidino- oder 4-Guanidinobenzylamin gekoppelten Linkergruppe mit einem Dicarbonsäureanhydrid, vorzugsweise dem Anhydrid der Bernsteinsäure oder der Glutarsäure unter Bildung einer HOOC-Gruppe umgesetzt werden kann oder dass eine HOOC-Gruppe einer an das acylierte 4-Amidino- oder 4-Guanidinobenzylamin gekoppelten Linkergruppe mit einem Diamin unter Beibehaltung einer H₂N-Gruppe umgesetzt werden kann.

8. Acyliertes Amidino- oder Guanidinobenzylamin zur Inhibierung von Plasmakallikrein und/oder Faktor XIa und/oder Faktor XIIa nach mindestens einem der Ansprüche 1, 2I), 2II), 3I) oder 4 bis 5, **dadurch gekennzeichnet, dass** die kovalent an P4 oder P2 gekoppelte Linkergruppe bei Vorhandensein einer zweiten funktionellen Gruppe, insbesondere einer substituierten oder unsubstituierten Amino-, Carboxyl- und/oder Mercaptogruppe, kovalent an eine künstliche Oberfläche oder, sofern es sich bei der Linkergruppe um ein Oligo- oder Polyalkylenglycol handelt, kovalent an ein zweites Molekül der allgemeinen Formel I gekoppelt ist.

9. Acyliertes Amidino- oder Guanidinobenzylamin zur Inhibierung von Plasmakallikrein und/oder Faktor XIa und/oder Faktor XIIa nach mindestens einem der Ansprüche 1, 2III), 3II) oder 4, **dadurch gekennzeichnet, dass** die kovalent an P4 oder P2 gekoppelte Linkergruppe ein Oligo- oder Polyalkylenglykol ist, das an dem nicht an P4 oder P2 gekoppelten Ende mit einer Alkylgruppe mit 1-4 C-Atomen, insbesondere Methyl oder mit einem zweiten Molekül der allgemeinen Formel I modifiziert sein kann, wobei die Linkergruppe durch Interaktion mit einer künstlichen Oberfläche nicht-kovalent an diese gekoppelt sein kann.

10. Acyliertes Amidino- oder Guanidinobenzylamin zur Inhibierung von Plasmakallikrein und/oder Faktor XIa und/oder Faktor XIIa nach Aspruch 8 oder 9, **dadurch gekennzeichnet, dass** die künstliche Oberfläche aus Cellulose-Diacetat, Cellulose-Triacetat, Poly(ethersulfon), Poly(arylethersulfon), regenerierter Cellulose, Cuprophan, Hemophan, Poly(sulfon), Poly(acrylnitril), Poly(vinylalkohol), Poly(carbonat), Poly(amid), Poly(methylmethacrylat), Poly(ethylen-co-vinylalkohol) oder einem anderen in Geräten wie Dialysatoren, Oxygenatoren, Kathetern, Membranen und/oder den zu den Geräten gehörenden Schlauchsystemen und/oder Luftfallen für die Oberflächen, die mit Blut in Kontakt kommen, verwendeten Material besteht, wobei das Oberflächenmaterial für die kovalente Kopplung des Moleküls der allgemeinen Formel I über die an P4 oder P2 gekoppelte Linkergruppe gegebenenfalls mit funktionellen Gruppen, z.B. Aminogruppen, Aminoalkylgruppen, Carboxylgruppen, Carboxyalkylgruppen, Mercaptogruppen, Mercaptoalkylgruppen, Hydroxylgruppen, Hydroxyalkylgruppen modifiziert sind.

11. Acyliertes Amidino- oder Guanidinobenzylamin zur Inhibierung von Plasmakallikrein und/oder Faktor XIa und/oder Faktor XIIa nach mindestens einem der Ansprüche 1 bis 10 zur Verhinderung der Blutgerinnung an künstlichen Oberflächen von z.B. Geräten wie Dialysatoren, Oxygenatoren, Kathetern, Membranen und/oder den zu den Geräten gehörenden Schlauchsystemen und/oder Luftfallen, insbesondere zur Verhinderung der Blutgerinnung an künstlichen Oberflächen durch kovalente oder nicht-kovalente Beschichtung der künstlichen Oberfläche(n) über eine Linkergruppe wie in einem der Ansprüche 1 bis 9 definiert.

12. Acyliertes Amidino- oder Guanidinobenzylamin, wie in mindestens einem der Ansprüche 1 bis 9 definiert, zur Herstellung eines Arzneimittels zur Verwendung als Antikoagulanz und/oder Antithrombotikum zur Verhinderung und/oder Behandlung von Herzinfarkt, Hirnschlag, Embolien, tiefen Beinvenenthrombosen z.B. nach Hüftgelenksoperationen und/oder Kniegelenksersatz, instabiler Angina pectoris, Komplikationen infolge von Angioplastie, insbesondere perkutaner transluminaler Koronarangioplastie (PTCA), und/oder zur Verhinderung und Therapie von disseminierter intravaskulärer Gerinnung, septischem Schock, Allergien, dem Postgastrektomiesyndrom, Arthritis und ARDS (adult respiratory distress syndrome).

13. Acyliertes Amidino- oder Guanidinobenzylamin, wie in mindestens einem der Ansprüche 1 bis 8 definiert, zur Herstellung eines Arzneimittels zur Inhibition von Plasmakallikrein und/oder Faktor XIIa und/oder Faktor XIa in parenteraler Anwendungsform, insbesondere in intraarterieller, intravenöser, intramuskulärer oder subkutaner Form, in enteraler Anwendungsform, insbesondere zur oralen oder rektalen Anwendung oder in topischer Anwendungsform, insbesondere als Dermatikum, insbesondere zur Inhibition von Plasmakallikrein.

14. Acyliertes Amidino- oder Guanidinobenzylamin, wie in mindestens einem der Ansprüche 1 bis 7 definiert, zur Herstellung eines Arzneimittels zur Inhibition von Plasmakallikrein und/oder Faktor XIIa und/oder Faktor XIa insbesondere in Form einer Tablette, eines Dragees, einer Kapsel, eines Pellets, Suppositoriums, einer Lösung, insbesondere einer Injektions- oder Infusionslösung, von Augentropfen, Nasentropfen, eines Safts, einer Kapsel, einer Emulsion oder Suspension, eines Globuli, Styli, Aerosols, Puders, einer Paste, Creme oder Salbe, insbesondere zur Inhibition von Plasmakallikrein.

15. Acyliertes Amidinobenzylamin der allgemeinen Formel V oder VI, wie in Anspruch 1 definiert, mit R₄ insbesondere gleich HO- und R₁ und R₃ keine Oligo- oder Polyalkylengruppe zur Herstellung eines Arzneimittels zur Verwendung als Antikoagulanz und/oder Antithrombotikum, wie in mindestens einem der Ansprüche 12 bis 14 definiert, wobei der Wirkstoff in Form eines Prodrugs zur oralen Gabe vorliegt.
